# EUROPEAN PATENT APPLICATION

(11) **EP 4 786 496 A1**
(43) Date of publication of application: **05.08.2026**
(21) Application number: 24871031.1
(22) Date of filing: 27.09.2024
(51) Int. Cl.: C07K 16/28, A61K 39/39, A61P 29/00, A61P 35/00, A61K 39/395

(54) **FULLY-HUMAN ANTIBODY AND CAR-T CELL TARGETING BAFF-R AND USE THEREOF**

(30) Priority: 28.09.2023 CN 202311274988; 28.09.2023 CN 202311275013
(71) Applicant: Nanjing Iaso Biotechnology Co., Ltd., Nanjing, Jiangsu 211500 (CN); Nanjing Iaso Medical Technology Co., Ltd., Nanjing, Jiangsu 211500 (CN)
(72) Inventor: TAN, Taochao, Nanjing, Jiangsu 211500 (CN); LIU, Jianwei, Nanjing, Jiangsu 211500 (CN); LUO, Qian, Nanjing, Jiangsu 211500 (CN); WEI, Qiaoe, Nanjing, Jiangsu 211500 (CN); JIA, Xiangyin, Nanjing, Jiangsu 211500 (CN); YANG, Yongkun, Nanjing, Jiangsu 211500 (CN); XIE, Meng, Nanjing, Jiangsu 211500 (CN); XU, Le, Nanjing, Jiangsu 211500 (CN)
(74) Representative: Lavoix
(86) International application number: PCT/CN2024/121964
(87) International publication number: WO 2025/067496

(57) **Abstract**

A fully human single-domain antibody a monoclonal antibody, a bispecific antibody, a CAR-T cell targeting BAFF-R and a use thereof. Provided are a fully human antibody or antigen-binding fragment thereof targeting BAFF-R obtained by means of screening a fully human phage library, a fully human chimeric antigen receptor (CAR) targeting BAFF-R, and an immune cell expressing the CAR. Further provided is a bispecific antibody targeting BAFF-R and CD3 in different combination configurations. Further provided is a use of the foregoing antibodies and the CAR-immune cell in the treatment of B cell tumors.

## Description

### TECHNICAL FIELD

The present application relates to the field of biomedicine, and in particular to a fully human monoclonal antibody, a monoclonal antibody, a bispecific antibody, and a CAR-T cell targeting BAFF-R, and the use thereof in CAR-T therapy.

### BACKGROUND

Lymphoma is a malignant tumor originating from lymph nodes and extranodal lymphoid tissues, accounting for 3%-4% of all malignant tumors in China, and is the most common malignant tumor in the hematological system. Two of the most common subtypes of B-cell lymphoma are diffuse large B-cell lymphoma (DLBCL) and follicular lymphoma (FL). Diffuse large B-cell lymphoma (DLBCL) is the most common aggressive form of B-NHL and accounts for about 30% of newly diagnosed NHL cases. Although standard first-line therapy with R-CHOP is currently used, approximately 10% of newly diagnosed DLBCL cases are refractory. Of those who respond to initial treatment, 40% relapse within 2-5 years 1. Autologous hematopoietic stem cell transplantation (ASCT) following achievement of CR or PR after chemotherapy is currently the optimal therapeutic regimen for relapsed and refractory DLBCL. However, the 2-year disease-free survival rate after transplantation remains less than 15%. Follicular lymphoma (FL) is the most common indolent B-NHL and accounts for about 22% of newly diagnosed NHL cases. Although disease progression is slow, follicular lymphoma is generally considered incurable, with a median overall survival of approximately 10 years. Approximately 15-28% of FL cases transform to aggressive lymphoma within 10 years.

CAR-T therapy for B-cell lymphoma is currently based primarily on the CD19 target. Four CD19-CAR-T products have been approved and launched. The main indications for the four CD19-CAR-T products are B-cell lymphoma (DLBCL) and follicular lymphoma (FL), with ORR of approximately 70%-80% and CR of 50%-60%. Although CD19 CAR-T has achieved higher remission rates than standard treatment for the two major B-NHL, follow-up studies have shown that some patients relapse due to antigen escape, i.e. mutation or deletion of CD19 protein on the surface of tumor cells that would render CAR-T cell therapy ineffective, accounting for about 10% to 20% 2. Therefore, it is urgent to seek new targets of CAR-T cell immunotherapy for patients with relapse and refractory to treatment.

BAFF-R (B-cell activating factor receptor) belongs to the tumor necrosis factor (TNF) receptor superfamily. BAFF-R is expressed on the surface of peripheral B cells, with the exception of plasma cells and centroblasts located in the dark zone of the germinal center. The investigators examined 116 clinical samples of B-cell lymphoma and found that BAFF-R was overexpressed on a variety of B-cell lymphoma cells, including chronic lymphocytic leukemia (CLL) (21/21, 100%), mantle cell lymphoma (MCL) (7/7, 100%), FL (13/16, 81%), and DLBCL (14/18, 78%) 3. Therefore, BAFF-R can be used as a new target for B cell tumor therapy in addition to CD19.

Therefore, BAFF-R is an effective therapeutic target for a variety of B-lymphocyte tumors, autoimmune diseases, and other diseases characterized by BAFF-R expression. It is of great significance and application value to develop immunotherapy products targeting BAFF-R.

### SUMMARY OF THE INVENTION

An objective of the present invention is to provide a fully human single-domain antibody a monoclonal antibody, a bispecific antibody, a CAR-T cell targeting BAFF-R and a use thereof.

An objective of the invention is to provide an antibody and a CAR targeting BAFF-R, and a host cell expressing the CAR targeting BAFF-R, as well as a preparation method therefor and a use thereof.

Another objective of the present invention is to provide a method for the construction and preparation of a bispecific antibody targeting BAFF-R and CD3, a method for biological activity evaluation, and a method for safety evaluation.

A first aspect of the present invention provides a single-domain antibody targeting B-cell activating factor receptor (BAFF-R), wherein the VHH chain of the single-domain antibody comprises the following complementarity-determining regions (CDRs): CDR1 set forth in SEQ ID NO. 27, CDR2 set forth in SEQ ID NO. 28, and CDR3 set forth in SEQ ID NO. 29;
any one of the aforementioned amino acid sequences also includes derivative sequences optionally obtained by addition, deletion, modification and/or substitution of at least one amino acid while being capable of retaining the binding affinity to BAFF-R.

In another preferred embodiment, the VHH chain of the single-domain antibody further comprises a framework region (FR).

In another preferred embodiment, the framework regions (FR) are of human, murine, rabbit or camelid origin.

In another preferred embodiment, the framework regions (FR) comprise human FR regions, murine or camelid FR regions.

In another preferred embodiment, the VHH chain of the single-domain antibody targeting BAFF-R has an amino acid sequence set forth in SEQ ID NO. 20, or an amino acid sequence having at least 70%, 75%, 80%, 85%, 90%, 91%, 92%, 93%, 94%, 95%, 96%, 97%, 98%, 99% or 100% identity thereto.

In another preferred embodiment, the single-domain antibody targeting BAFF-R is a fully human antibody.

A second aspect of the present invention provides an antibody targeting B-cell activating factor receptor (BAFF-R), wherein the heavy chain variable region of the antibody comprises the following complementarity-determining regions (CDRs): CDR1 set forth in SEQ ID NO. 27, CDR2 set forth in SEQ ID NO. 28, and CDR3 set forth in SEQ ID NO. 29; or
the light chain variable region of the antibody comprises the following complementarity-determining regions (CDRs): CDR1 set forth in SEQ ID NO. 36, CDR2 set forth in AAS, and CDR3 set forth in SEQ ID NO. 37, and the heavy chain variable region of the antibody comprises the following complementarity-determining regions (CDRs): CDR1 set forth in SEQ ID NO. 38, CDR2 set forth in SEQ ID NO. 39, and CDR3 set forth in SEQ ID NO. 40;
any one of the aforementioned amino acid sequences also includes derivative sequences optionally obtained by addition, deletion, modification and/or substitution of at least one amino acid while being capable of retaining the binding affinity to BAFF-R.

In another preferred embodiment, the light chain variable region and/or the heavy chain variable region further comprise a framework region (FR).

In another preferred embodiment, the framework regions (FR) are of human, murine, rabbit or camelid origin.

In another preferred embodiment, the framework regions (FR) comprise human FR regions, murine or camelid FR regions.

In another preferred embodiment, the heavy chain variable region has an amino acid sequence set forth in SEQ ID NO. 25, or an amino acid sequence having at least 70%, 75%, 80%, 85%, 90%, 91%, 92%, 93%, 94%, 95%, 96%, 97%, 98%, 99% or 100% identity thereto.

In another preferred embodiment, the light chain variable region has an amino acid sequence set forth in SEQ ID NO. 30, or an amino acid sequence having at least 70%, 75%, 80%, 85%, 90%, 91%, 92%, 93%, 94%, 95%, 96%, 97%, 98%, 99%, 100% identity thereto, and the heavy chain variable region has an amino acid sequence set forth in SEQ ID NO. 32, or an amino acid sequence having at least 70%, 75%, 80%, 85%, 90%, 91%, 92%, 93%, 94%, 95%, 96%, 97%, 98%, 99%, 100% identity thereto.

In another preferred embodiment, the antibody is a single-domain antibody.

In another preferred embodiment, the antibody is a monomeric antibody, bivalent antibody, and/or multivalent antibody.

In another preferred embodiment, the antibody is a fully human antibody, animal-derived antibody, humanized antibody, fully human antibody chimeric antibody, or chimeric antigen receptor antibody (CAR).

In another preferred embodiment, the CDR region of the humanized antibody comprise 1, 2, or 3 amino acid changes.

In another preferred embodiment, the animal is a non-human mammal, preferably mouse, sheep, rabbit, or camelid.

In another preferred embodiment, the antibody is a diabody, or a single-chain antibody.

In another preferred embodiment, the antibody is a monoclonal antibody.

In another preferred embodiment, the antibody is a fully human monoclonal antibody.

In another preferred embodiment, the number of amino acids added, deleted, modified and/or substituted does not exceed 40% of the total number of amino acids in the initial amino acid sequence, preferably 20%, more preferably 10%.

In another preferred embodiment, the number of amino acids added, deleted, modified and/or substituted is 1-7, preferably 1-3, more preferably 1.

In another preferred embodiment, the at least one amino acid sequence subjected to addition, deletion, modification and/or substitution is an amino acid sequence with a homology of at least 80%.

A third aspect of the present invention provides a chimeric antigen receptor, wherein the antigen-binding domain of the chimeric antigen receptor comprises an antigen-binding fragment targeting BAFF-R, and the heavy chain variable region of the antigen-binding fragment comprises the following complementarity-determining regions (CDRs): CDR1 set forth in SEQ ID NO. 27, CDR2 set forth in SEQ ID NO. 28, and CDR3 set forth in SEQ ID NO. 29;
or the light chain variable region of the antigen-binding fragment comprises the following complementarity-determining regions (CDRs): CDR1 set forth in SEQ ID NO. 36, CDR2 set forth in AAS, and CDR3 set forth in SEQ ID NO. 37, and the heavy chain variable region of the antibody comprises the following complementarity-determining regions (CDRs): CDR1 set forth in SEQ ID NO. 38, CDR2 set forth in SEQ ID NO. 39, and CDR3 set forth in SEQ ID NO. 40.

In another preferred embodiment, the antigen-binding fragment is the heavy chain variable region of a single-domain antibody.

In another preferred embodiment, The antigen-binding fragment has an amino acid sequence set forth in SEQ ID NO. 25, or an amino acid sequence having at least 70%, 75%, 80%, 85%, 90%, 91%, 92%, 93%, 94%, 95%, 96%, 97%, 98%, 99% or 100% identity thereto.

In another prefer embodiment, the antigen-binding domain of the chimeric antigen receptor comprise one or more of the antigen-binding fragments.

In another preferred embodiment, the antigen-binding fragment is a single-chain antibody.

In another preferred embodiment, the antigen-binding fragment comprises, from N-terminus to C-terminus, a light chain variable region, a linker peptide, and a heavy chain variable region in sequence.

In another preferred embodiment, the antigen-binding fragment has an amino acid sequence set forth in SEQ ID NO. 34, or an amino acid sequence having at least 70%, 75%, 80%, 85%, 90%, 91%, 92%, 93%, 94%, 95%, 96%, 97%, 98%, 99% or 100% identity thereto.

In another preferred embodiment, the CAR further comprises a transmembrane region derived from CD8a and/or an intracellular domain derived from CD3ζ.

In another preferred embodiment, the CAR comprises an intracellular co-stimulatory region derived from CD28.

In another preferred embodiment, the CAR comprises a co-stimulatory molecule derived from 4-1BB.

In another preferred embodiment, the structure of the chimeric antigen receptor is shown in Formula I below:

L-V-H-TM-C-CD3ζ (I)

where
each "-" is independently a linker peptide or peptide bond;
L is a signal peptide sequence;
V is an antigen-binding domain;
H is a hinge region;
TM is a transmembrane domain;
C is a co-stimulatory signal molecule;
CD3ζ is a cytoplasmic signaling sequence derived from CD3ζ.

In another preferred embodiment, the L is a signal peptide of a protein selected from the group consisting of CD8, CD28, GM-CSF, CD4, CD137, or a combination thereof.

In another preferred embodiment, the L is a signal peptide derived from CD8.

In another preferred embodiment, the amino acid sequence of the CD8-derived signal peptide is set forth in SEQ ID NO. 2.

In another preferred embodiment, the anti-BAFF-R single-domain antibodies include fully human single-domain antibodies, humanized single-domain antibodies, and camelid single-domain antibodies, preferably fully human single-domain antibodies.

In another preferred embodiment, the V is a fully human single-domain antibody.

In another preferred embodiment, the H is a hinge region derived from a protein selected from the group consisting of Fc, CD8, CD28, CD137, or a combination thereof.

In another preferred embodiment, the H is a hinge region derived from CD8, the amino acid sequence of which is set forth in SEQ ID NO. 4.

In another preferred embodiment, the TM is a transmembrane region of a protein selected from the group consisting of ICOS, CD28, CD3 epsilon, CD45, CD4, CD5, CD8, CD9, CD16, CD22, CD33, CD37, CD64, CD80, CD86, CD134, CD137, CD154, or a combination thereof.

In another preferred embodiment, the TM is a transmembrane region derived from CD8.

In another preferred embodiment, the amino acid sequence of the CD8-derived transmembrane region is set forth in SEQ ID NO. 6.

In another preferred embodiment, the C is a co-stimulatory signal molecule of a protein selected from the group consisting of ICOS, OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2, or a combination thereof.

In another preferred embodiment, the C is a co-stimulatory signal molecule derived from ICOS and/or 4-1BB.

In another preferred embodiment, the amino acid sequence of the 4-1BB-derived co-stimulatory signal molecule is set forth in SEQ ID NO. 8.

In another preferred embodiment, the amino acid sequence of the CD3ζ is set forth in SEQ ID NO. 10.

In another preferred embodiment, the CAR (preferably at the C-terminus) further comprises a cell suicide element.

In another preferred embodiment, the CAR is linked to the cell suicide element via a self-cleaving element.

In another preferred embodiment, the cell suicide element is linked to CD3ζ of the CAR by T2A or P2A.

In another preferred embodiment, the cell suicide element is selected from the group consisting of HSV-TK, iCasp9, ΔCD20, mTMPK, ΔCD19, RQR8, EGFRt, or a combination thereof.

In another preferred embodiment, the cell suicide element is tEGFR.

In another preferred embodiment, the amino acid sequence of the tEGFR is set forth in SEQ ID NO: 16.

In another preferred embodiment, the amino acid sequence of the T2A is set forth in SEQ ID NO: 18.

In another preferred embodiment, the amino acid sequence of the CAR is set forth in SEQ ID NO. 20.

A fourth aspect of the present invention provides a multispecific antibody, wherein the multispecific antibody comprise a single-domain antibody targeting BAFF-R according to the first aspect of the present invention or the antibody targeting BAFF-R according to the second aspect of the present invention.

In another preferred embodiment, the multispecific antibody further comprises a second antigen-binding region targeting a target selected from the group consisting of: CD3, CD28, 4-1BB, BCMA, CD73, GPC3, HER2, PMSA, OX40, GLP-1, Trop2, FGL1, LFA-3, 2B4, 5T4, α-4 integrin, α-V integrin, α4β7 integrin, α4β7 integrin, α-SMA, AGR2, Apelin J receptor, APRIL, B7-H3, B7-H4, BAFF, BTLA, C5 complement, C-242, CA9, CA19-9, carbonic anhydrase 9, CD2, CD6, CD9, CDlla, CD19, CD20, CD22, CD24, CD25, CD27, CD30, CD33, CD38, CD40, CD40L, CD41, CD44, CD44v6, CD47, CD51, CD52, CD56, CD64, CD69, CD70, CD71, CD74, CD80, CD81, CD86, CD95, CD107a, CD117, CD123, CD125, CD132(IL-2Rg), CD133, CD137, CD138, CD160, CD166, CD172A, CD248, CEACAM5 (CEA), CEACAM6 (NCA-90), CLAUDIN-3, CLAUDIN-4, cMet, collagen, Cripto, CSFR, CSFR-1, CTLA-4, CTGF, CXCL10, CXCL13, CXCR1, CXCR2, CXCR4, CYR61, DL44, DLK1, DLL4, DPP-4, DSG1, EDA, EDB, EGFR, EGFRviii, endothelin B receptor (ETBR), ENPP3, EpCAM, EPHA2, EPHB2, ERBB3, RSV F protein, FAP, FGF-2, FGF8, FGFR1, FGFR2, FGFR3, FGFR4, FLT-3, folate receptor α (FRα), FSP -1, GAL3ST1, G-CSF, G-CSFR, GD2, GITR, GLUT1, GLUT4, GM-CSF , GM-CSFR, GP Ilb/IIIa receptor, Gpl30, GPIIB/IIIA, GPNMB, GRP78, HER2/neu, HER3, HER4, HGF, hGH, HLA-DR, HVEM, hyaluronidase, ICOS, IFNα, IFNβ, IFNγ, IgE , IgE receptor (FceRI), IGF, IGF1R, IL1B, IL1R, IL2, IL11, IL12, IL12p40, IL-12R, IL-12Rβl, IL13, IL13R, IL13Ra2, IL15, IL17, IL18, IL21, IL23, IL23R, IL27 /IL27R (wsxl), IL29, IL-31R, IL31/IL31R, IL2R, IL4, IL4R, IL6, IL6R, IL1 receptor accessory protein (IL1RAP), insulin receptor, Jagged ligand, Jagged 1, Jagged 2, KISS1-R, KLRG1, LAG-3, LIF-R, Lewis X, LIGHT, LRP4, LRRC26, Ly6G6D, LyPD1, MCSP, mesothelin, MRP4, MUC1, mucin-16 (MUC16, CA-125), Na/K ATPase, NGF, Nicastrin, Notch receptor, Notch 1, Notch 2, Notch 3, Notch 4, NOV, OSM-R, OX-40, PAR2, PDGF-AA, PDGF-BB, PDGFRα, PDGFRβ, PD-1, PD-L1, PD-L2, phosphatidylserine, P1GF, PSCA, PSMA, PSGR, RAAG12, RAGE, SLC44A4, Siglecl5, STEAP1, STEAP2, TAG-72, TAPA1, TEM-8, TGFβ, TIGIT, TIM-3, TLR2, TLR4, TLR6, TLR7, TLR8, TLR9, TMEM31, TNFα, TNFR, TNFRS12A, TRAIL-R1, TRAIL-R2, transferrin, transferrin receptor, TRK-A, TRK-B, uPAR, VAP1, VCAM-1, VEGF, VEGF-A, VEGF-B, VEGF-C, VEGF -D, VEGFR1, VEGFR2, VEGFR3, VISTA, WISP-1, WISP-2, WISP-3, or a combination thereof.

In another preferred embodiment, the second antigen-binding region is a fully human antibody, an animal-derived antibody, a humanized antibody, or a chimeric antibody.

In another preferred embodiment, the multispecific antibody comprises one or more second antigen-binding regions.

In another preferred embodiment, the multispecific antibody either comprises or does not comprise an Fc segment of an antibody.

In another prefer embodiment, the Fc segment of the antibody is of IgG 1 subtype or IgG4 subtype.

In another preferred embodiment, the Fc segment of the antibody comprises L234A/L235A mutation or S228P/F234A/L235A mutation.

In another preferred embodiment, the Fc segment of the antibody comprises a knobs-into-holes structure; preferably, the knobs side has S354C/T366W combined mutation, and the holes side has Y349C/T366S/L368A/Y407V combined mutation.

In another preferred embodiment, the antigen-binding region is an antibody or antibody fragment, and the antibody fragment includes: (i) Fab fragment; (ii) F(ab')2 fragment; (iii) Fd fragment; (iv) Fv fragment; (v) single-chain Fv (scFv) molecule; (vi) dAb fragment.

A fifth aspect of the present invention provides a bispecific antibody, the bispecific antibody comprising:
a first antigen-binding region targeting BAFF-R; and
a second antigen-binding region targeting CD3; wherein,
the first antigen-binding region targeting BAFF-R comprises the following complementarity-determining regions CDRs: CDR1 set forth in SEQ ID NO. 27, CDR2 set forth in SEQ ID NO. 28, and CDR3 set forth in SEQ ID NO. 29.

In another preferred embodiment, the first antigen-binding region targeting BAFF-R has an amino acid sequence set forth in SEQ ID NO. 25, or an amino acid sequence having at least 70%, 75%, 80%, 85%, 90%, 91%, 92%, 93%, 94%, 95%, 96%, 97%, 98%, 99% or 100% identity thereto.

In another preferred embodiment, the second antigen-binding region targeting CD3 comprises a heavy chain variable region and a light chain variable region targeting CD3, wherein,
the heavy chain variable region targeting CD3 comprises the following complementarity-determining regions CDRs: CDR1 set forth in SEQ ID NO. 79, CDR2 set forth in SEQ ID NO. 80, and CDR3 set forth in SEQ ID NO. 81;
the light chain variable region targeting CD3 comprises the following complementarity-determining regions CDRs: CDR1 set forth in SEQ ID NO. 82, CDR2 with the amino acid sequence GTN, and CDR3 set forth in SEQ ID NO. 83;
any one of the aforementioned amino acid sequences also includes derivative sequences optionally obtained by addition, deletion, modification and/or substitution of at least one amino acid while being capable of retaining the binding affinity to CD3.

In another preferred embodiment, the amino acid sequence of the heavy chain variable region targeting CD3 is set forth in SEQ ID NO. 45, and the amino acid sequence of the light chain variable region targeting CD3 is set forth in SEQ ID NO. 47; or
the amino acid sequence of the heavy chain variable region targeting CD3 is set forth in SEQ ID NO. 65, and the amino acid sequence of the light chain variable region targeting CD3 is set forth in SEQ ID NO. 67.

In another preferred embodiment, the nucleotide sequence encoding the heavy chain variable region targeting CD3 is set forth in SEQ ID NO. 46, and the nucleotide sequence encoding the light chain variable region targeting CD3 is set forth in SEQ ID NO. 48; or
the nucleotide sequence encoding the heavy chain variable region targeting CD3 is set forth in SEQ ID NO. 66, and the nucleotide sequence encoding the light chain variable region targeting CD3 is set forth in SEQ ID NO. 68.

In another preferred embodiment, the second antigen-binding region targeting CD3 is a fully human antibody, an animal-derived antibody, a humanized antibody, or a chimeric antibody.

In another preferred embodiment, the second antigen-binding region is an antibody or antibody fragment, and the antibody fragment includes: (i) Fab fragment; (ii) F(ab')2 fragment; (iii) Fd fragment; (iv) Fv fragment; (v) single-chain Fv (scFv) molecule; (vi) dAb fragment.

In another preferred embodiment, the amino acid sequence of the Fab fragment targeting CD3 is set forth in SEQ ID NO. 49.

In another preferred embodiment, the nucleotide sequence encoding the Fab fragment targeting CD3 is set forth in SEQ ID NO. 50.

In another preferred embodiment, the bispecific antibody is a symmetric or asymmetric dimer.

In another preferred embodiment, the first antigen-binding region is linked to the N-terminus or C-terminus of the second antigen-binding region.

In another preferred embodiment, the first antigen-binding region is linked to the N-terminus or C-terminus of the Fab fragment targeting CD3.

In another preferred embodiment, the first antigen-binding region is linked to the N-terminus or C-terminus of the heavy chain or light chain encoding the Fab fragment targeting CD3, preferably to the N-terminus of the heavy chain encoding the Fab fragment targeting CD3.

In another preferred embodiment, the bispecific antibody further comprise an Fc segment of an antibody.

In another preferred embodiment, the first antigen-binding region is linked to the N-terminus or C-terminus of the Fc segment, preferably to the C-terminus of the Fc segment.

In another preferred embodiment, the bispecific antibody has the following structure:
(a) two heavy chains, the first heavy chain comprising, from N-terminus to C-terminus: anti-BAFF-R VH and human IgG Fc region; the second heavy chain comprising, from N-terminus to C-terminus: anti-CD3 VH, antibody heavy chain constant region (CH1), and human IgG Fc region;
(b) one light chain, comprising, from N-terminus to C-terminus: anti-CD3 VL and antibody light chain constant region (CL),
wherein a disulfide bond is present between the antibody heavy chain constant region (CH1) and the antibody light chain constant region (CL).

In another preferred embodiment, the anti-CD3 VL pairs with the anti-CD3 VH, and the antibody heavy chain constant region (CH1) pairs with the antibody light chain constant region (CL).

In another preferred embodiment, the anti-BAFF-R VH has the same meaning as the first antigen-binding region targeting BAFF-R.

In another preferred embodiment, the antibody heavy chain constant region (CH1) is linked to the human IgG Fc region via a hinge region.

In another preferred embodiment, a disulfide bond exists between the two human IgG Fc regions comprised in the bispecific antibody.

In another preferred embodiment, the two human IgG Fc regions comprised in the bispecific antibody together constitute the Fc segment of the antibody. Preferably, the Fc segment of the antibody is of the IgG4 isotype, more preferably comprising S228P/F234A/L235A mutation.

In another preferred embodiment, the Fc segment of the antibody comprises a knobs-into-holes structure; preferably, the knobs side has S354C/T366W combined mutation, and the holes side has Y349C/T366S/L368A/Y407V combined mutation.

In another preferred embodiment, the bispecific antibody has the following structure:
(a) two heavy chains, the first heavy chain comprising, from N-terminus to C-terminus: anti-BAFF-R VH, anti-CD3 VH, antibody heavy chain constant region (CH1), and human IgG Fc region; the second heavy chain comprising, from N-terminus to C-terminus: anti-BAFF-R VH and human IgG Fc region;
(b) one light chain, comprising, from N-terminus to C-terminus: anti-CD3 VL and antibody light chain constant region (CL),
wherein a disulfide bond is present between the antibody heavy chain constant region (CH1) and the antibody light chain constant region (CL).

In another preferred embodiment, the anti-CD3 VL pairs with the anti-CD3 VH, and the antibody heavy chain constant region (CH1) pairs with the antibody light chain constant region (CL).

In another preferred embodiment, the bispecific antibody has the following structure:
(a) two identical heavy chains, each comprising, from N-terminus to C-terminus: anti-BAFF-R VH, anti-CD3 VH, antibody heavy chain constant region (CH1), and human IgG Fc region;
(b) two identical light chains, each comprising, from N-terminus to C-terminus: anti-CD3 VL and antibody light chain constant region (CL),
wherein a disulfide bond is present between the antibody heavy chain constant region (CH1) and the antibody light chain constant region (CL).

In another preferred embodiment, the anti-CD3 VL pairs with the anti-CD3 VH, and the antibody heavy chain constant region (CH1) pairs with the antibody light chain constant region (CL).

In another preferred embodiment, for the bispecific antibody,
the heavy chain comprises an amino acid sequence set forth in SEQ ID NO: 73 or 51, or an amino acid sequence having at least 70%, 75%, 80%, 85%, 90%, 91%, 92%, 93%, 94%, 95%, 96%, 97%, 98%, 99%, or 100% identity thereto,
the light chain comprises an amino acid sequence set forth in SEQ ID NO: 71, or an amino acid sequence having at least 70%, 75%, 80%, 85%, 90%, 91%, 92%, 93%, 94%, 95%, 96%, 97%, 98%, 99%, or 100% identity thereto.

In another preferred embodiment, for the bispecific antibody,
the heavy chain comprises an amino acid sequence set forth in SEQ ID NO: 75 or 51, or an amino acid sequence having at least 70%, 75%, 80%, 85%, 90%, 91%, 92%, 93%, 94%, 95%, 96%, 97%, 98%, 99%, or 100% identity thereto,
the light chain comprises an amino acid sequence set forth in SEQ ID NO: 71, or an amino acid sequence having at least 70%, 75%, 80%, 85%, 90%, 91%, 92%, 93%, 94%, 95%, 96%, 97%, 98%, 99%, or 100% identity thereto.

In another preferred embodiment, for the bispecific antibody,
the heavy chain comprises an amino acid sequence set forth in SEQ ID NO: 77, or an amino acid sequence having at least 70%, 75%, 80%, 85%, 90%, 91%, 92%, 93%, 94%, 95%, 96%, 97%, 98%, 99%, or 100% identity thereto,
the light chain comprises an amino acid sequence set forth in SEQ ID NO: 71, or an amino acid sequence having at least 70%, 75%, 80%, 85%, 90%, 91%, 92%, 93%, 94%, 95%, 96%, 97%, 98%, 99%, or 100% identity thereto.

A sixth aspect of the present invention provides a recombinant protein, wherein the recombinant protein comprises:
(i) the single-domain antibody targeting BAFF-R according to the first aspect of the present invention, the antibody targeting BAFF-R according to the second aspect of the present invention, the chimeric antigen receptor according to the third aspect of the present invention, the multispecific antibody according to the fourth aspect of the present invention, or the bispecific antibody according to the fifth aspect of the present invention; and
(ii) optionally, a polypeptide molecule or fragment having therapeutic function; and/or
(iii) optionally, a functional domain that improves the physicochemical properties or druggability of the protein.

A seventh aspect of the present invention provides a nucleic acid molecule, wherein the nucleic acid molecule encodes a polypeptide selected from the group consisting of:
(1) the single-domain antibody targeting BAFF-R according to the first aspect of the present invention, the antibody targeting BAFF-R according to the second aspect of the present invention, the chimeric antigen receptor according to the third aspect of the present invention, the multispecific antibody according to the fourth aspect of the present invention, or the bispecific antibody according to the fifth aspect of the present invention; or
(2) the recombinant protein according to the sixth aspect of the present invention.

In another preferred embodiment, the nucleotide sequence of the nucleic acid molecule encoding the chimeric antigen receptor according to the second aspect of the present invention is set forth in SEQ ID NO: 26.

In another preferred embodiment, the polynucleotide comprises RNA, DNA or cDNA.

An eighth aspect of the present invention provides is a vector, which comprises the nucleic acid molecule according to the seventh aspect of the present invention.

In another preferred embodiment, the vector is selected from the group consisting of: DNA, RNA, plasmid, lentiviral vector, adenoviral vector, retroviral vector, transposon, or a combination thereof.

In another preferred embodiment, the vector is a lentiviral vector.

A ninth aspect of the present invention provides is a host cell, wherein the host cell comprises the vector according to the eighth aspect of the present invention, or has integrated into its chromosome the exogenous nucleic acid molecule according to the seventh aspect of the present invention.

A tenth aspect of the present invention provides an engineered immune cell, wherein the immune cell comprises the vector according to the eighth aspect of the present invention, or has integrated into its chromosome the exogenous nucleic acid molecule according to the seventh aspect of the present invention, or expresses the chimeric antigen receptor according to the third aspect of the present invention.

In another preferred embodiment, the immune cell is a T cell or an NK cell.

In another preferred embodiment, the TCR gene expression of the T cell is silenced.

In another preferred embodiment, the immune cell expresses an exogenous cell suicide element.

In another preferred embodiment, the engineered immune cell is selected from the group consisting of:
(i) chimeric antigen receptor T cells (CAR-T cells); or
(ii) chimeric antigen receptor NK cells (CAR-NK cells).

In another preferred embodiment, CAR and the cell suicide element are co-expressed in the engineered immune cell.

An eleventh aspect of the present invention provides an immunoconjugate, wherein the immunoconjugate comprises:
(a) an antibody moiety, the antibody moiety being the single-domain antibody targeting BAFF-R according to the first aspect of the present invention, the antibody targeting BAFF-R according to the second aspect of the present invention, the multispecific antibody according to the fourth aspect of the present invention, or the bispecific antibody according to the fifth aspect of the present invention; and
(b) a conjugate moiety conjugated to the antibody moiety, the conjugate moiety being selected from the group consisting of: a detectable label, a drug, a toxin, a cytokine, an enzyme, or a combination thereof.

In another preferred embodiment, the immunoconjugate is a single-domain antibody-drug conjugate.

In another preferred embodiment, the antibody moiety is conjugated to the conjugate moiety via a chemical bond or a linker.

In another preferred embodiment, the conjugate moiety is a chemical label and a biological label.

In another preferred embodiment, the chemical label is an isotope, an immunotoxin, and/or a chemical drug.

In another preferred embodiment, the biological label is biotin, avidin, or an enzyme label.

In another preferred embodiment, the conjugate moiety is a drug or a toxin.

In another preferred embodiment, the drug is a cytotoxic drug.

In another preferred embodiment, the cytotoxic drug is selected from the group consisting of antitubulin drugs, DNA minor groove binding agents, DNA replication inhibitors, alkylating agents, antibiotics, folate antagonists, antimetabolites, chemosensitizers, topoisomerase inhibitors, vinca alkaloids, or a combination thereof.

Examples of particularly useful classes of cytotoxic drugs include, for example, DNA minor groove binding agents, DNA alkylating agents, and tubulin inhibitors. Typical cytotoxic drugs include, for example, auristatins, camptothecins, duocarmycins, etoposides, maytansines and maytansinoids (e.g., DM1 and DM4), taxanes, benzodiazepines or benzodiazepine-containing drugs (e.g., pyrrolo[1,4]benzodiazepines (PBDs), indolinobenzodiazepines, and oxazolidinobenzodiazepines), vinca alkaloids, or a combination thereof.

In another preferred embodiment, the toxin is selected from the group consisting of:
auristatins (e.g., auristatin E, auristatin F, MMAE and MMAF), aurochloromycin, maytansinoids, ricin, ricin A-chain, combretastatin, duocarmycin, dolastatin, doxorubicin, daunorubicin, paclitaxel, cisplatin, cc1065, ethidium bromide, mitomycin, etoposide, tenoposide, vincristine, vinblastine, colchicine, dihydroxy anthracin dione, actinomycin, diphtheria toxin, Pseudomonas exotoxin (PE) A, PE40, abrin, abrin A-chain, volkensin A-chain, alpha-sarcin, gelonin, mitogellin, restrictocin, phenomycin, enomycin, curicin, crotin, calicheamicin, Sapaonaria officinalis inhibitor, glucocorticoid, or a combination thereof.

In another preferred embodiment, the conjugate moiety is a detectable label.

In another preferred embodiment, the detectable label comprises a radionuclide, and the radionuclide includes:
(i) a diagnostic isotope selected from the group consisting of Tc-99m, Ga-68, F-18, I-123, I-125, I-131, In-111, Ga-67, Cu-64, Zr-89, C-11, Lu-177, Re-188, or a combination thereof; and/or
(ii) a therapeutic isotope selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, 1-131, Ir-192, Fe-59, Pb-212, Mo-99, Pd-103, P-32, K-42, Re-186, Re-188, Sm-153, Ra223, Ru106, Na24, Sr89, Tb-149, Th-227, Xe-133, Yb-169, Yb-177, or a combination thereof.

In another preferred embodiment, the conjugate is selected from the group consisting of: fluorescent or luminescent labels, radioactive labels, MRI (magnetic resonance imaging) or CT (X-ray computed tomography) contrast agents, enzymes capable of producing a detectable product, radionuclides, biotoxins, cytokines (IL-2, etc.), antibodies, antibody Fc fragments, antibody scFv fragments, gold single-domain particles/single-domain rods, viral particles, liposomes, magnetic nanoparticles, prodrug-activating enzymes (e.g., DT-diaphorase (DTD) or biphenyl hydrolase-like protein (BPHL)), chemotherapeutic agents (e.g., cisplatin), or single-domain particles in any form, etc.

In another preferred embodiment, the immunoconjugate comprises: a multivalent (e.g., bivalent) single-domain antibody targeting BAFF-R according to the first aspect of the present invention, or an antibody targeting BAFF-R according to the second aspect of the present invention.

In another preferred embodiment, the term "multivalent" means that the amino acid sequence of the immunoconjugate comprises multiple repeats of the single-domain antibody targeting BAFF-R according to the first aspect of the present invention, or the antibody targeting BAFF-R according to the second aspect of the present invention.

In another preferred embodiment, the detection is in vivo detection or in vitro detection.

In another preferred embodiment, the immunoconjugate is used for the diagnosis and/or treatment of tumors expressing BAFF-R protein.

In another preferred embodiment, the immunoconjugate has the formula as shown below: 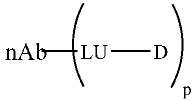 where
nAb represents a single-domain antibody targeting BAFF-R, an antibody targeting BAFF-R or a multispecific antibody;
LU represents a linker;
D represents a drug;
and the subscript p is a value selected from 1 to 10.

In another preferred embodiment, LU is selected from linkers including maleimidocaproyl (MC), maleimide (MAL), succinimidyl 4-(N-maleimidomethyl)cyclohexane-1-carboxylate (SMCC) for conjugation to the antibody moiety, and one or more linkers including valine-citrulline (VC), valine-alanine (VA), glycine-glycine-phenylalanine-glycine (GGFG), alanine-alanine-alanine (AAA), para-aminobenzyloxycarbonyl (PAB), and polyethylene glycol (PEG).

In another preferred embodiment, the antibody moiety is covalently attached to the linker via reaction with a moiety selected from the group consisting of maleimidocaproyl (MC), maleimide (MAL), succinimidyl 4-(N-maleimidomethyl)cyclohexane-1-carboxylate (SMCC), and the like.

In another preferred embodiment, D is an anti-tumor active compound selected from the group consisting of:
(i) tubulin inhibitors, such as maytansine derivatives (DM1, DM4), monomethyl auristatin E (MMAE), monomethyl auristatin F (MMAF);
(ii) DNA-acting toxins, such as duocarmycins, pyrrolobenzodiazepines (PBD);
(iii) topoisomerase inhibitors, such as camptothecin, SN38, ezetecan, Dxd.

A twelfth aspect of the present invention provides a pharmaceutical composition or formulation, which comprises:
(i) the single-domain antibody targeting BAFF-R according to the first aspect of the present invention, the antibody targeting BAFF-R according to the second aspect of the present invention, the multispecific antibody according to the fourth aspect of the present invention, the bispecific antibody according to the fifth aspect of the present invention, the recombinant protein according to the sixth aspect of the present invention, the engineered immune cell according to the tenth aspect of the present invention, or the immunoconjugate according to the eleventh aspect of the present invention; and
(ii) a pharmaceutically acceptable carrier.

In another preferred embodiment, the pharmaceutical composition comprises a single drug, a compound drug, or a synergistic drug.

In another preferred embodiment, the pharmaceutical composition further comprises other biologically active substances, such as anti-tumor drugs.

In another preferred embodiment, the administration route of the pharmaceutical composition is selected from the group consisting of subcutaneous injection, intradermal injection, intramuscular injection, intravenous injection, intraperitoneal injection, microneedle injection, oral administration, or oral-nasal spraying and aerosol inhalation.

In another preferred embodiment, the dosage form of the pharmaceutical composition is selected from the group consisting of liquid, solid, or gel. In another preferred embodiment, that formulation is a liquid formulation.

In another preferred embodiment, the formulation is an injection.

A thirteenth aspect of the present invention provides the use of an active ingredient in (a) preparing a detection reagent, a test plate or a kit; and/or (b) preparing a drug for preventing and/or treating BAFF-R-associated diseases, the active ingredient being selected from the group consisting of: the single-domain antibody targeting BAFF-R according to the first aspect of the present invention, the antibody targeting BAFF-R according to the second aspect of the present invention, the multispecific antibody according to the fourth aspect of the present invention, the bispecific antibody according to the fifth aspect of the present invention, the recombinant protein according to the sixth aspect of the present invention, the engineered immune cell according to the tenth aspect of the present invention, the immunoconjugate according to the eleventh aspect of the present invention, or a combination thereof.

In another preferred embodiment, the BAFF-R-associated diseases include tumorigenesis, growth and/or metastasis, tumor drug resistance-related diseases, autoimmune diseases, inflammation, and metabolism-related diseases.

In another preferred embodiment, the BAFF-R-associated diseases include tumors with high expression of BAFF-R.

In another preferred embodiment, the tumors include leukemia, lymphoma, myeloma, breast cancer (such as triple-negative breast cancer), lung cancer (such as non-small cell lung cancer), pancreatic cancer, malignant glioma, gastric cancer, liver cancer, esophageal cancer, renal cancer, colorectal cancer, bladder cancer, prostate cancer, endometrial cancer, ovarian cancer, cervical cancer, and angiosarcoma.

In another preferred embodiment, the tumor is a hematological tumor.

In another preferred embodiment, the tumor is a plasma cell malignancy or a B cell malignancy.

A fourteenth aspect of the present invention provides a method for preparing an engineered immune cell, wherein the engineered immune cell expresses the chimeric antigen receptor according to the third aspect of the present invention, comprising the following step:
transducing the nucleic acid molecule according to the seventh aspect of the present invention or the vector according to the eighth aspect of the present invention into an immune cell, thereby obtaining the engineered immune cell.

In another preferred embodiment, the immune cell is a T cell or an NK cell.

A fifteenth aspect of the present invention provides a kit for preparing the engineered immune cell according to the tenth aspect of the present invention, wherein the kit comprises a container, and the nucleic acid molecule according to the seventh aspect of the present invention or the vector according to the eighth aspect of the present invention located within the container.

A sixteenth aspect of the present invention provides a method for the in vitro detection (including diagnostic or non-diagnostic) of BAFF-R in a sample, comprising the steps of:
(1) contacting the sample in vitro with the single-domain antibody targeting BAFF-R according to the first aspect of the present invention, the antibody targeting BAFF-R according to the second aspect of the present invention, or the immunoconjugate according to the eleventh aspect of the present invention;
(2) detecting whether an antigen-antibody complex is formed, wherein the formation of the complex indicates the presence of BAFF-R in the sample.

In another preferred embodiment, the test is diagnostic or non-diagnostic.

A seventeenth aspect of the present invention provides a method for preparing a recombinant polypeptide, comprising:
(a) culturing the host cell according to the ninth aspect of the present invention under conditions suitable for expression; and
(b) isolating the recombinant polypeptide from the culture, wherein the recombinant polypeptide is the single-domain antibody targeting BAFF-R according to the first aspect of the present invention, the antibody targeting BAFF-R according to the second aspect of the present invention, the multispecific antibody according to the fourth aspect of the present invention, the bispecific antibody according to the fifth aspect of the present invention, or the recombinant protein according to the sixth aspect of the present invention.

An eighteenth aspect of the present invention provides a method for treating BAFF-R-associated diseases, comprising: administering to a subject in need thereof the single-domain antibody targeting BAFF-R according to the first aspect of the present invention, the antibody targeting BAFF-R according to the second aspect of the present invention, the multispecific antibody according to the fourth aspect of the present invention, the bispecific antibody according to the fifth aspect of the present invention, the recombinant protein according to the sixth aspect of the present invention, the engineered immune cell according to the tenth aspect of the present invention, the immunoconjugate according to the eleventh aspect of the present invention, or the pharmaceutical composition or formulation according to the twelfth aspect of the present invention, or a combination thereof.

In another preferred embodiment, the method further comprises: administering to the subject in need thereof other drugs or therapies for combination therapy.

In another preferred embodiment, the other drugs or therapies include anti-tumor immunotherapeutic drugs, tumor-targeted drugs, tumor chemotherapeutic drugs, and tumor radiotherapy.

In another preferred embodiment, the anti-tumor immunotherapeutic drugs include PD-1 monoclonal antibody and PD-L1 monoclonal antibody.

It is to be understood that within the scope of the present invention, the above technical features of the present invention and those specifically described below (e.g., in the Examples) can be combined with each other to form new or preferred technical solutions. For brevity, they are not described in detail herein.

### DESCRIPTION OF DRAWINGS

FIG. 1 shows the general procedure for screening specific antibodies targeting BAFF-R from a phage antibody library according to the present invention.
FIG. 2 shows the results of enzyme-linked immunosorbent assay (ELISA) of some panned phage monoclones with a target antigen and a control antigen.
FIG. 3 shows the flow cytometric analysis results of the binding of some phage monoclones to K562-BAFF-R and K562 cells.
FIGS. 4A-B show the flow cytometric analysis results (histograms) of the binding of the selected phage monoclones to various BAFF-R-positive and negative cell lines. FIG. 4A shows the flow cytometry histograms of Clones 1, 5, 14 binding to various cell lines; Negative Control represents the negative control phage antibody clone. FIG. 4B shows the flow cytometric histograms of Clones 77, 80 binding to multiple cell lines. VAY736 serves as the positive control phage antibody clone.

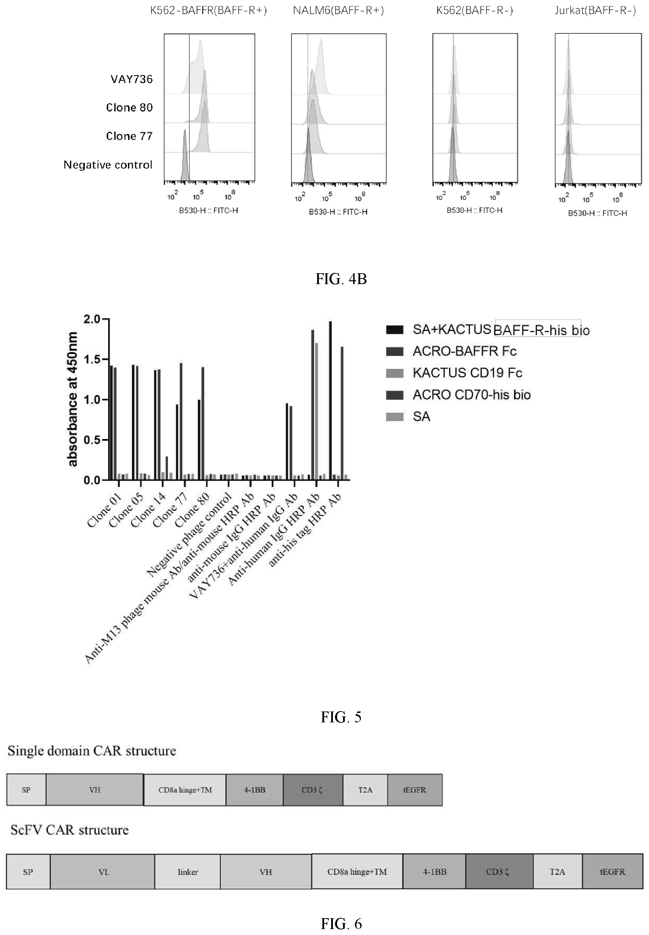

FIG. 5 shows the results of enzyme-linked immunosorbent assay analysis of the screened phage monoclones against BAFF-R antigen proteins from different companies and unrelated antigens. Negative Control is the negative control phage antibody clone. anti-M13 phage mouse Ab/anti-mouse HRP Ab is the negative control without phage and only with the primary antibody (Anti-M13 phage mouse Ab) and the secondary antibody (anti-mouse HRP Ab); anti-mouse HRP Ab is the negative control only with the secondary antibody (anti-mouse HRP Ab), VAY736 Ab/anti-human IgG HRP Ab is the positive control of the target antigen (BAFF-R-his-Bio), anti-human IgG HRP and anti-his HRP are the positive antibody controls for detecting antigen tags. In FIG. 5, the bar graphs corresponding to each test antibody and control group represent, from left to right, the test results against the reagents KACTUS-BAFF-R-his Bio, ACRO-BAFF-R-Fc, KACTUS-CD19-Fc, ACRO-CD70-his, and SA, respectively.
FIG. 6 shows a schematic diagram of the construction of the BAFF-R CAR vector. CAR includes an extracellular signal peptide (SP), a ScFv (VL-linker-VH) or Single-domain (V_{HH}) that binds to the target antigen BAFRR, a hinge region and a transmembrane region (CD8a hinge+TM) between a cell membrane and an extracellular binding region, a 4-1BB co-stimulatory molecule, a CD3ζ intracellular domain, a T2A self-cleaving peptide, and a truncated tEGFR.
FIG. 7 shows the expression of tEGFR in CAR-T cells of different clones.
FIG. 8 shows the killing results of CAR-T cells of different clones against various target cells: JEKO1-luc (FIG. A), MEC1-luc (FIG. B), RS4-luc (FIG. C), NALM6-luc (FIG. D), and MOLT4-luc (FIG. E).

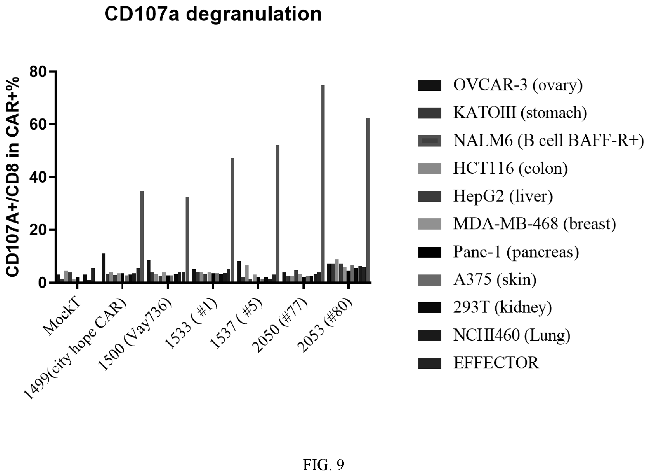

FIG. 9 shows the CD107a degranulation of CAR-T cells of different clones in response to tumor cells derived from various tissues.
FIG. 10 shows the anti-tumor efficacy of CAR-T cells corresponding to different clones in NCG mice bearing JEKO1-luc tumor. FIG. A shows the change in photon flux over time after CAR-T cell infusion, and FIG. B shows the survival rate of mice after CAR-T cell infusion. FIG. C shows the changes in the percentage of T cells among mouse lymphocytes after CAR-T cell infusion.
FIG. 11 shows IVIS images at different time points after infusion of CAR-T cells corresponding to different clones in NCG mice bearing JEKO1-luc tumor.
FIG. 12 shows the schematic diagram of the 1+1 configuration of the α-BAFF-R×α-CD3 bispecific antibody; wherein FIG. 12A shows the schematic diagram of the 1+1 configuration of the α-BAFF-R scFab×α-CD3 scFab bispecific antibody; FIG. 12B shows the schematic diagram of the 1+1 configuration of the α-BAFF-R VH×α-CD3 scFab bispecific antibody.
FIG. 13 shows the activation of TDCC reporter gene cells by K562-BAFF-R mediated by the α-BAFF-R×α-CD3 bispecific antibody.
FIG. 14 shows the activation of TDCC reporter gene cells by K562 mediated by the α-BAFF-R×α-CD3 bispecific antibody.
FIG. 15 shows the killing of Nalm 6 cells by T cells mediated by the α-BAFF-R×α-CD3 bispecific antibody.
FIG. 16 shows the killing of K562 cells by T cells mediated by the α-BAFF-R×α-CD3 bispecific antibody.
FIG. 17 shows the amino acid sequence alignment analysis of the extracellular domain of BAFF-R among human, cynomolgus and mouse.
FIG. 18 the schematic diagrams of different configurations of the α-BAFF-R VH×α-CD3 Fab bispecific antibody.
FIG. 18A shows the schematic diagram of the 1+1 configuration of the α-BAFF-R VH × α-CD3 Fab bispecific antibody.
FIG. 18B shows the schematic diagram of the 2+1 configuration of the α-BAFF-R VH × α-CD3 Fab bispecific antibody.
FIG. 18C shows the schematic diagram of the 2+2 configuration of the α-BAFF-R VH × α-CD3 Fab bispecific antibody.
FIG. 19 shows the activation of TDCC reporter gene cells by K562-BAFF-R mediated by the α-BAFF-R VH×α-CD3 Fab bispecific antibody.
FIG. 20 shows the activation of TDCC reporter gene cells by Nalm6 mediated by the α-BAFF-R VH×α-CD3 Fab bispecific antibody.
FIG. 21 shows the activation of TDCC reporter gene cells by Mec-1 mediated by the α-BAFF-R VH×α-CD3 bispecific antibody.
FIG. 22 shows the activation of TDCC reporter gene cells by Jeko-1 mediated by the α-BAFF-R VH×α-CD3 bispecific antibody.
FIG. 23 shows the activation of TDCC reporter gene cells by K562 mediated by the α-BAFF-R VH×α-CD3 bispecific antibody.
FIG. 24 shows the 24-hour killing of Jeko-1 cells by PBMC cells (Donor 301C) mediated by the α-BAFF-R VH×α-CD3 Fab bispecific antibody.
FIG. 25 shows the 48-hour killing of Jeko-1 cells by PBMC cells (Donor 301C) mediated by the α-BAFF-R VH×α-CD3 Fab bispecific antibody.
FIG. 26 shows the 24-hour killing of Jeko-1 cells by PBMC cells (Donor 605C) mediated by the α-BAFF-R VH×α-CD3 Fab bispecific antibody.
FIG. 27 shows the 48-hour killing of Jeko-1 cells by PBMC cells (Donor 605C) mediated by the α-BAFF-R VH×α-CD3 Fab bispecific antibody.
FIG. 28 shows that the α-BAFF-R VH×α-CD3 Fab bispecific antibody promotes cytokine IFN-γ secretion by PBMC cells (Donor 303C).
FIG. 28A shows the analysis of cytokine IFN-γ secretion by PBMCs (Donor 303C) after 24-hour co-incubation of PBMCs with the bispecific antibody and Jeko-1.
FIG. 28B shows the analysis of cytokine IFN-γ secretion by PBMCs (Donor 303C) after 48-hour co-incubation of PBMCs with the bispecific antibody and Jeko-1.
FIG. 28C shows the analysis of cytokine IFN-γ secretion by PBMCs (Donor 303C) after 24-hour co-incubation of PBMCs with the bispecific antibody.
FIG. 28D shows the analysis of cytokine IFN-γ secretion by PBMCs (Donor 303C) after 48-hour co-incubation of PBMCs with the bispecific antibody.
FIG. 29 shows that the α-BAFF-R VH×α-CD3 Fab bispecific antibody promotes cytokine IFN-γ secretion by PBMC cells (Donor 605C).
FIG. 29A shows the analysis of cytokine IFN-γ secretion by PBMCs (Donor 605C) after 24-hour co-incubation of PBMCs with the bispecific antibody and Jeko-1.
FIG. 29B shows the analysis of cytokine IFN-γ secretion by PBMCs (Donor 605C) after 48-hour co-incubation of PBMCs with the bispecific antibody and Jeko-1.
FIG. 29C shows the analysis of cytokine IFN-γ secretion by PBMCs (Donor 605C) after 24-hour co-incubation of PBMCs with the bispecific antibody.
FIG. 29D shows the analysis of cytokine IFN-γ secretion by PBMCs (Donor 605C) after 48-hour co-incubation of PBMCs with the bispecific antibody.
FIG. 30 shows that the α-BAFF-R VH×α-CD3 Fab bispecific antibody promotes cytokine IL-2 secretion by PBMC cells (Donor 303C).
FIG. 30A shows the analysis of cytokine IL-2 secretion by PBMCs (Donor 303C) after 24-hour co-incubation of PBMCs with the bispecific antibody and Jeko-1.
FIG. 30B shows the analysis of cytokine IL-2 secretion by PBMCs (Donor 303C) after 48-hour co-incubation of PBMCs with the bispecific antibody and Jeko-1.
FIG. 30C shows the analysis of cytokine IL-2 secretion by PBMCs (Donor 303C) after 24-hour co-incubation of PBMCs with the bispecific antibody.
FIG. 30D shows the analysis of cytokine IL-2 secretion by PBMCs (Donor 303C) after 48-hour co-incubation of PBMCs with the bispecific antibody.
FIG. 31 shows that the α-BAFF-R VH×α-CD3 Fab bispecific antibody promotes cytokine IL-2 secretion by PBMC cells (Donor 605C).
FIG. 31A shows the analysis of cytokine IL-2 secretion by PBMCs (Donor 605C) after 24-hour co-incubation of PBMCs with the bispecific antibody and Jeko-1.
FIG. 31B shows the analysis of cytokine IL-2 secretion by PBMCs (Donor 605C) after 48-hour co-incubation of PBMCs with the bispecific antibody and Jeko-1.
FIG. 31C shows the analysis of cytokine IL-2 secretion by PBMCs (Donor 605C) after 24-hour co-incubation of PBMCs with the bispecific antibody.
FIG. 31D shows the analysis of cytokine IL-2 secretion by PBMCs (Donor 605C) after 48-hour co-incubation of PBMCs with the bispecific antibody.
FIG. 32 shows that the α-BAFF-R VH×α-CD3 Fab bispecific antibody promotes cytokine TNF-α secretion by PBMC cells (Donor 303C).
FIG. 32A shows the analysis of cytokine TNF-α secretion by PBMCs (Donor 303C) after 24-hour co-incubation of PBMCs with the bispecific antibody and Jeko-1.
FIG. 32B shows the analysis of cytokine TNF-α secretion by PBMCs (Donor 303C) after 48-hour co-incubation of PBMCs with the bispecific antibody and Jeko-1.
FIG. 32C shows the analysis of cytokine TNF-α secretion by PBMCs (Donor 303C) after 24-hour co-incubation of PBMCs with the bispecific antibody.
FIG. 32D shows the analysis of cytokine TNF-α secretion by PBMCs (Donor 303C) after 48-hour co-incubation of PBMCs with the bispecific antibody.
FIG. 33 shows that the α-BAFF-R VH×α-CD3 Fab bispecific antibody promotes cytokine TNF-α secretion by PBMC cells (Donor 605C).
FIG. 33A shows the analysis of cytokine TNF-α secretion by PBMCs (Donor 605C) after 24-hour co-incubation of PBMCs with the bispecific antibody and Jeko-1.
FIG. 33B shows the analysis of cytokine TNF-α secretion by PBMCs (Donor 605C) after 48-hour co-incubation of PBMCs with the bispecific antibody and Jeko-1.
FIG. 33C shows the analysis of cytokine TNF-α secretion by PBMCs (Donor 605C) after 24-hour co-incubation of PBMCs with the bispecific antibody.
FIG. 33D shows the analysis of cytokine TNF-α secretion by PBMCs (Donor 605C) after 48-hour co-incubation of PBMCs with the bispecific antibody.
FIG. 34 shows that the α-BAFF-R VH×α-CD3 Fab bispecific antibody promotes cytokine IL-6 secretion by PBMC cells (Donor 303C).
FIG. 34A shows the analysis of cytokine IL-6 secretion by PBMCs (Donor 303C) after 24-hour co-incubation of PBMCs with the bispecific antibody and Jeko-1.
FIG. 34B shows the analysis of cytokine IL-6 secretion by PBMCs (Donor 303C) after 48-hour co-incubation of PBMCs with the bispecific antibody and Jeko-1.
FIG. 34C shows the analysis of cytokine IL-6 secretion by PBMCs (Donor 303C) after 24-hour co-incubation of PBMCs with the bispecific antibody.
FIG. 34D shows the analysis of cytokine IL-6 secretion by PBMCs (Donor 303C) after 48-hour co-incubation of PBMCs with the bispecific antibody.
FIG. 35 shows that the α-BAFF-R VH×α-CD3 Fab bispecific antibody promotes cytokine IL-6 secretion by PBMC cells (Donor 605C).
FIG. 35A shows the analysis of cytokine IL-6 secretion by PBMCs (Donor 605C) after 24-hour co-incubation of PBMCs with the bispecific antibody and Jeko-1.
FIG. 35B shows the analysis of cytokine IL-6 secretion by PBMCs (Donor 605C) after 48-hour co-incubation of PBMCs with the bispecific antibody and Jeko-1.
FIG. 35C shows the analysis of cytokine IL-6 secretion by PBMCs (Donor 605C) after 24-hour co-incubation of PBMCs with the bispecific antibody.
FIG. 35D shows the analysis of cytokine IL-6 secretion by PBMCs (Donor 605C) after 48-hour co-incubation of PBMCs with the bispecific antibody.
FIG. 36 shows the concentration-time curve of bispecific antibody 0205-2 in the serum of SD rats after a single dose.
FIG. 37 shows the concentration-time curve of bispecific antibody 0205-3 in the serum of SD rats after a single dose.
FIG. 38 shows the curves depicting changes in subcutaneous tumor volume (mm³) of mice treated with the bispecific antibody 0205-2 and those in the PBS control group over time after the first dose.
FIG. 39 shows the curves depicting changes in subcutaneous tumor volume (mm³) of mice in the PBS group over time after the first dose.
FIG. 40 shows the curves depicting changes in subcutaneous tumor volume (mm³) of mice treated with the bispecific antibody 0205-2 at 1mpk over time after the first dose.
FIG. 41 shows the curves depicting changes in subcutaneous tumor volume (mm³) of mice treated with the bispecific antibody 0205-2 at 10mpk over time after the first dose.
FIG. 42 shows the curves depicting changes in body weight (g) of mice in all the groups over time after the first dose.

### DETAILED DESCRIPTION

Through extensive and in-depth research, the present inventors screened fully human BAFF-R-specific antibodies using a large-capacity phage antibody library, and evaluated the specificity of these antibodies at the phage level by ELISA and FACS assays. Finally, several fully human antibody clones with favorable specificity were obtained. Using fully human antibodies with lower immunogenicity, antibody drugs (including monoclonal antibodies, bispecific antibodies, ADCs, etc.) and cell therapy drugs (including CAR-T, CAR-NK, etc.) were developed.

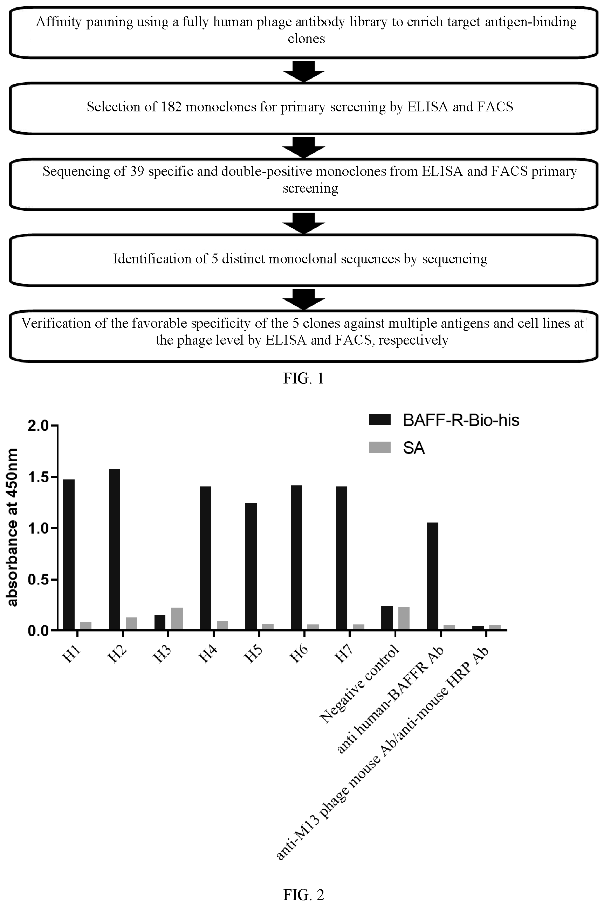

Specifically, after panning with recombinant BAFF-R protein using different antibody libraries, a total of 182 monoclones were selected for primary screening by enzyme-linked immunosorbent assay (ELISA) and fluorescence-activated cell sorting (FACS). Among them, 39 clones specifically bound to BAFF-R-his-Bio antigen and BAFF-R-positive K562-BAFF-R cells, but not the control protein SA or BAFF-R-negative K562 cells. After sequencing, 5 different monoclonal sequences were obtained. Subsequently, these 5 antibodies were identified by fluorescence-activated cell sorting (FACS) with various BAFF-R-positive (K562-BAFF-R, NALM6) and negative cell lines (K562, Jurkat), and by ELISA with BAFF-R proteins from different companies (KACTUS BAFF-R-his bio, ACRO-BAFF-R-Fc, SB-BAFF-R-Fc) and unrelated proteins (KACTUS-CD19-Fc, ACRO-CD70-his, SA). Of them, 4 clones exhibited favorable binding affinity and specificity across multiple cell lines and protein antigens. The overall project workflow is shown in FIG. 1.

The functions of the candidate clones on CAR-T cells were further verified. Clone 5 showed the best in vivo anti-tumor efficacy and favorable safety in mice, making it a superior candidate clone for subsequent clinical development. The heavy chain variable region of clone 5 comprises the following complementarity-determining regions (CDRs): CDR1 set forth in SEQ ID NO. 27, CDR2 set forth in SEQ ID NO. 28, and CDR3 set forth in SEQ ID NO. 29. More specifically, the heavy chain variable region of Clone 5 has an amino acid sequence set forth in SEQ ID NO. 25. Clone 1 also exhibited favorable in vivo anti-tumor efficacy and can serve as a candidate clone. The light chain variable region of clone 1 comprises the following complementarity-determining regions (CDRs): CDR1 set forth in SEQ ID NO. 36, CDR2 set forth in AAS, and CDR3 set forth in SEQ ID NO. 37, and the heavy chain variable region of the antibody comprises the following complementarity-determining regions (CDRs): CDR1 set forth in SEQ ID NO. 38, CDR2 set forth in SEQ ID NO. 39, and CDR3 set forth in SEQ ID NO. 40.

In addition, the present invention further provides fully human single-domain antibodies targeting BAFF-R and bispecific antibodies thereof. Anti-BAFF-R and anti-CD3 bispecific antibodies with different configurations (e.g., 1+1, 2+1, and 2+2 combination configurations) can specifically bind to B-cell activating factor receptor (BAFF-R) on the cell surface and cluster of differentiation 3 (CD3) on immune cells, with high affinity, and can be used for the treatment of BAFFR-expressing neoplastic diseases and autoimmune diseases.

The present invention has been completed on this basis.

### Terms

Unless otherwise defined, all technical and scientific terms used herein have the same meaning as commonly understood by one of ordinary skill in the art to which the invention belongs.

The term "about" may refer to a value or composition within an acceptable error range for the particular value or composition as determined by one of ordinary skill in the art, which will depend in part on how the value or composition is measured or determined.

As used herein, the terms "comprising," "including," or "containing" can be open, semi-closed, or closed. In other words, such terms also include "consisting essentially of" or "consisting of."

Unless otherwise defined, all technical and scientific terms used herein have the meanings as commonly understood by those of ordinary skill in the art.

"Antibodies" refer to immunoglobulins secreted by plasma cells (effector B cells) and used by the body's immune system to neutralize foreign substances (peptides, viruses, bacteria, etc.). The foreign substance is correspondingly called an antigen. The basic structure of an antibody molecule is a 4-mer consisting of 2 identical heavy chains and 2 identical light chains. According to the conservative differences in amino acid sequences, the heavy and light chains are divided into a variable region (V) at the amino terminus and a constant region (C) at the carboxyl terminus. The variable region of one heavy chain (HCVR, also referred to as VH) and the variable region of one light chain (LCVR, also referred to as VL) interact to form the antigen-binding site (Fv). In the variable region, the composition and arrangement of amino acid residues in certain regions are more variable than other regions (framework regions, FRs) in the variable region, these regions are called hypervariable regions (HVRs) and are actually the key sites for binding of antibodies to antigens. Since these hypervariable regions have their sequences complementary to antigenic determinants, they are also called complementarity-determining regions (CDRs). Both heavy and light chains have three complementarity-determining regions, designated HCDR1, HCDR2, HCDR3 and LCDR1, LCDR2, LCDR3, respectively. The heavy chain constant region comprises three constant domains CH1, CH2 and CH3. Each light chain comprises a light chain variable region (abbreviated herein as VL) and a light chain constant region. The light chain constant region comprises one constant domain CL. The VH and VL regions can be further subdivided into hypervariable regions termed complementarity-determining regions (CDRs), interspersed with more conserved regions termed framework regions (FRs). VH and VL each comprise three CDRs and four FRs, arranged from amino-terminus to carboxyl-terminus in the following order: FR1, CDR1, FR2, CDR2, FR3, CDR3, FR4. The variable regions of the heavy and light chains comprise binding domains that interacts with an antigen.

"Single chain fragment variable (scFv)" is composed of antibody heavy and light chain variable regions linked by a short peptide into a peptide chain. Through correct folding, the variable regions from the heavy chain and light chain interact through non-covalent bonds to form the Fv segment, so that scFv can better retain its affinity activity for antigens.

"Single-domain antibody (sdAb, or VHH)" and "nanobody" have the same meaning, referring to a nanobody constructed by cloning the variable region of an antibody heavy chain and consisting of only one heavy chain variable region, which is the smallest antigen-binding fragment with complete function. Generally, an antibody naturally lacking a light chain and heavy chain constant region 1 (CH1) is first obtained, and then the variable region of the antibody heavy chain is cloned to construct a single-domain antibody (VHH) composed of only one heavy chain variable region. As used herein, the terms "nanobody of the present invention", "anti-BAFF-R nanobody of the present invention", and "BAFF-R single-domain antibody of the present invention" are used interchangeably and all refer to single-domain antibodies that specifically recognize and bind to BAFF-R (including human BAFF-R). Particularly preferred is the single-domain antibody wherein the amino acid sequence of the VHH chain is set forth in SEQ ID NO. 25, namely clone 5 in the Examples.

"Murine antibody" refers to an antibody produced by a murine animal against a specific antigen, usually an antibody produced by mouse B lymphocytes. In most cases, the murine antibody is a monoclonal antibody produced by hybridoma cells. The fully human antibody of the present invention is screened and obtained from a human phage antibody library, which has reduced immunogenicity compared with murine antibodies and is more suitable for therapeutic use in humans.

As used herein, "fully human antibody or single-chain antibody or fragment thereof" generally refers to any form of antigen-binding molecule capable of binding to a target antigen. For example, the antigen-binding molecule may be a protein or polypeptide, including, for example, antibodies and antigen-binding fragments thereof, single-chain scFv antibodies, single-domain antibodies, various fusions and conjugates constructed based on scFv, such as scFv-Fc antibodies, immunoconjugates, antibody-drug conjugates (ADCs), multispecific/bispecific antibodies, and chimeric antigen receptors (CARs).

"BAFF-R" or "BAFF-R" refers to the major mediator receptor through which BAFF regulates B-cell differentiation. The BAFF/BAFF-R signaling pathway plays an important role in the proliferation of B lymphocytes. The upregulation of BAFF-R is associated with the progression of B-cell lymphoma and pre-B-ALL. BAFF-R is highly specific, mainly expressed in B cells, and specifically binds only to BAFF, but not to other members of the TNF family. Therefore, BAFF-R is an effective therapeutic target for many cancers, autoimmune diseases, and various other diseases characterized by BAFF-R expression.

As used herein, the term "sequence identity" (also known as sequence consistency) when referring to amino acid or nucleotide sequences refers to the degree of identity between two amino acid or nucleotide sequences (e.g., a query sequence and a reference sequence), usually expressed as a percentage. Typically, before calculating the identity percentage between two amino acid or nucleotide sequences, the sequence alignment is first performed, and a gap (if any) is introduced. If at a certain alignment position, the amino acid residues or bases in the two sequences are the same, the two sequences are considered to be identical or matched at the position; and if the amino acid residues or bases in the two sequences are different, they are considered to be non-identical or mismatched at the position. In some algorithms, the number of matched positions is divided by the total number of positions in the alignment window to obtain sequence identity. In other algorithms, the number of gaps and/or the gap length are also taken into account. For the purposes of the present invention, the published alignment software BLAST (available at ncbi.nlm.nih.gov) can be employed to obtain optimal sequence alignments by using default settings and calculate the sequence identity between two amino acid or nucleotide sequences. In some embodiments, "at least 90% sequence identity" as used in the present invention includes, but is not limited to, at least 95%, at least 98%, at least 99%, or even 100% sequence identity.

In some embodiments, the fully human antibodies provided by the present invention also comprise amino acid sequences having at least 90% sequence identity (e.g., at least 95%, at least 98%, at least 99%, or even 100% sequence identity) to the sequence set forth in SEQ ID NO. 25.

Those skilled in the art can understand that, on the basis of the specific sequences provided herein, the corresponding variants of the antibody targeting BAFF-R provided by the present invention can be obtained by replacing, deleting or adding a few amino acids, and verifying or screening the resultant product for its binding ability with the corresponding antigen BAFF-R or its biological activity, and these variants should also be included within the scope of the present invention. For example, the fully human antibody or single-chain antibody or antigen-binding fragment thereof of the present invention may have at least 1 and no more than 10, or no more than 5, 4, 3, 2, or 1 amino acid alterations in the full-length or CDR sequences.

Those skilled in the art can also understand that, on the basis of the specific heavy chain variable region sequences provided herein, an antibody light chain library (such as a human phage light chain library) can be screened by using BAFF-R as the antigen, so as to obtain light chain variable regions matched with the heavy chain variable region while maintaining BAFF-R binding ability. Anti-BAFF-R antibody molecules obtainable in this manner are also included within the scope of the present invention.

In some embodiments, the antigen-binding molecules of the present invention may further comprise post-translational modifications. Examples of post-translational protein modifications include: phosphorylation, acetylation, methylation, ADP-ribosylation, ubiquitination, glycosylation, carbonylation, SUMOylation, biotinylation, or the addition of polypeptide side chains or hydrophobic groups. Therefore, the modified soluble polypeptides may comprise non-amino acid components such as lipids, polysaccharides or monosaccharides, and phosphates. One preferred form of glycosylation is sialylation modification, which conjugates one or more sialic acid groups to the polypeptide. Sialic acid groups improve the solubility and serum half-life of the protein while reducing potential immunogenicity of the protein. See Raju et al. Biochemistry . 2001 31; 40(30):8868-76.

"Epitope" refers to the portion of a molecule bound by an antigen-binding protein (e.g., an antibody). An epitope may comprise non-adjacent portions of the molecule (e.g., amino acid residues in a polypeptide that are not adjacent in the primary sequence of the polypeptide but are sufficiently close to one another in the tertiary and quaternary structure of the polypeptide to be bound by the antigen-binding protein).

The present invention provides a kit comprising one or more containers containing a plurality of gene constructs encoding the polypeptides of the present invention, and a pharmaceutically acceptable excipient. The kit may also comprise instructions for use. The kit may further bear a notice in a form prescribed by a governmental agency regulating the manufacture, use or sale of pharmaceuticals or biological products, which notice reflects approval by the agency for manufacture, use or sale for human administration.

As used herein, the terms "heavy chain variable region" and "V_{H}" are used interchangeably.

As used herein, the terms "variable region" and "complementarity-determining region" are used interchangeably.

In a preferred embodiment of the present invention, the heavy chain variable region of the antibody comprises three complementarity-determining regions CDR1, CDR2, and CDR3.

In a preferred embodiment of the present invention, the heavy chain of the antibody comprises the heavy chain variable region and heavy chain constant region as described above.

In the present invention, the terms "antibody of the present invention", "protein of the present invention", or "polypeptide of the present invention" are used interchangeably and all refer to polypeptides that specifically bind to the BAFF-R protein, such as proteins or polypeptides having a heavy chain variable region. They may or may not contain initial methionine.

The present invention further provides other proteins or fusion expression products comprising the antibody of the present invention. Specifically, the present invention includes any protein, protein conjugate, or fusion expression product (i.e., immunoconjugate and fusion expression product) having a heavy chain containing a variable region, provided that the variable region is identical to or has at least 90% homology, preferably at least 95% homology, with the heavy chain variable region of the antibody of the present invention.

Generally, the antigen-binding properties of an antibody can be characterized by 3 specific regions located in the heavy chain variable region, known as complementarity-determining regions (CDRs), which are separated by 4 framework regions (FRs). The amino acid sequences of the 4 FRs are relatively conserved and do not directly participate in the binding reaction. These CDRs form loop structures that are spatially close to each other through β-sheets formed by the intervening FRs. The CDRs on the heavy chain and the corresponding CDRs on the light chain constitute the antigen-binding site of the antibody. The amino acids constituting the FR or CDR regions can be determined by comparing the amino acid sequences of antibodies of the same type.

The variable regions of the heavy chains of the antibodies of the present invention are of particular interest, since at least part of them are involved in antigen binding. Accordingly, the present invention includes those molecules comprising an antibody heavy chain variable region with CDRs, provided that the CDRs have more than 90% (preferably more than 95%, most preferably more than 98%) homology with the CDRs identified herein.

The antibody of the present invention refers to a polypeptide having BAFF-R protein-binding activity and comprising the CDR regions described above. The term also includes variant forms of the polypeptide comprising the CDR regions described above that have the same function as the antibody of the present invention. Such variant forms include, but are not limited to: deletion, insertion and/or substitution of one or more (generally 1-50, preferably 1-30, more preferably 1-20, most preferably 1-10) amino acids, and addition of one or several (generally within 20, preferably within 10, more preferably within 5) amino acids at the C-terminus and/or N-terminus. For example, in the art, substitution with amino acids of similar or comparable properties generally does not alter the function of the protein. As another example, addition of one or several amino acids at the C-terminus and/or N-terminus generally does not alter the function of the protein either. The term also includes active fragments and active derivatives of the antibody of the present invention.

The Variant forms of the polypeptide include: homologous sequences, conservative variants, allelic variants, natural mutants, induced mutants, proteins encoded by DNA that hybridizes to the DNA encoding the antibody of the present invention under high or low stringent conditions, and polypeptides or proteins obtained using antisera raised against the antibody of the present invention.

The present invention also provides other polypeptides, such as fusion proteins comprising a single-domain antibody or a fragment thereof. In addition to substantially full-length polypeptides, the present invention also includes fragments of the single-domain antibodies of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, the "conservative variant of the antibody of the present invention" refers to a polypeptide formed by replacing at most 10, preferably at most 8, more preferably at most 5, and most preferably at most 3 amino acids with amino acids of similar or comparable properties compared with the amino acid sequence of the antibody of the present invention. Such conservative variant polypeptides are preferably produced by amino acid substitutions according to Table A.

**Table A**

| Original residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides polynucleotide molecules encoding the aforementioned antibodies or fragments thereof or fusion proteins thereof. The polynucleotides of the present invention may be in the form of DNA or RNA. DNA forms include cDNA, genomic DNA, or synthetic DNA. The DNA can be single-stranded or double-stranded. The DNA may be a coding strand or a non-coding strand.

Polynucleotides encoding the mature polypeptide of the present invention include: coding sequences encoding only the mature polypeptide; coding sequences encoding the mature polypeptide and various additional coding sequences; coding sequences encoding the mature polypeptide (and optional additional coding sequences) and non-coding sequences.

The term "polynucleotide encoding a polypeptide" may refer to a polynucleotide including the coding sequence for the polypeptide, or a polynucleotide further including additional coding and/or non-coding sequences.

The present invention also relates to polynucleotides that hybridize to the aforementioned sequences and have at least 50%, preferably at least 70%, more preferably at least 80% identity between the two sequences. In particular, the present invention relates to polynucleotides that hybridize to the polynucleotides of the present invention under stringent conditions. In the present invention, "stringent conditions" refer to: (1) hybridization and elution at lower ionic strength and higher temperature, such as 0.2×SSC, 0.1% SDS, 60°C; or (2) adding denaturants during hybridization, such as 50% (v/v) formamide, 0.1% calf serum/0.1% Ficoll, 42°C; or (3) hybridization occurs only when the identity between the two sequences is at least 90% or more, preferably 95% or more. Furthermore, the polypeptides encoded by the hybridizable polynucleotides have the same biological functions and activities as the mature polypeptides.

The full-length nucleotide sequence of the antibody of the present invention or fragments thereof can usually be obtained by PCR amplification, recombinant methods, or chemical synthesis. One feasible approach is to synthesize the sequences in question by means of artificial synthesis, especially when the length of the fragment is short. Typically, long sequences can be obtained by first synthesizing multiple small fragments and then ligating them. In addition, the coding sequence of the heavy chain can be fused with an expression tag (such as 6His) to form a fusion protein.

### Bispecific antibody

The present invention also encompasses multispecific antibodies comprising the BAFF-R single-domain antibody of the present invention, wherein the multispecific antibodies further comprise a second antigen-binding region targeting a target selected from the group consisting of: CD3, CD28, 4-1BB, BCMA, CD73, GPC3, HER2, PMSA, OX40, GLP-1, Trop2, FGL1, LFA-3, 2B4, 5T4, α-4 integrin, α-V integrin, α4β7 integrin, α4β7 integrin, α-SMA, AGR2, Apelin J receptor, APRIL, B7-H3, B7-H4, BAFF, BTLA, C5 complement, C-242, CA9, CA19-9, carbonic anhydrase 9, CD2, CD6, CD9, CDlla, CD19, CD20, CD22, CD24, CD25, CD27, CD30, CD33, CD38, CD40, CD40L, CD41, CD44, CD44v6, CD47, CD51, CD52, CD56, CD64, CD69, CD70, CD71, CD74, CD80, CD81, CD86, CD95, CD107a, CD117, CD123, CD125, CD132(IL-2Rg), CD133, CD137, CD138, CD160, CD166, CD172A, CD248, CEACAM5 (CEA), CEACAM6 (NCA-90), CLAUDIN-3, CLAUDIN-4, cMet, collagen, Cripto, CSFR, CSFR-1, CTLA-4, CTGF, CXCL10, CXCL13, CXCR1, CXCR2, CXCR4, CYR61, DL44, DLK1, DLL4, DPP-4, DSG1, EDA, EDB, EGFR, EGFRviii, endothelin B receptor (ETBR), ENPP3, EpCAM, EPHA2, EPHB2, ERBB3, RSV F protein, FAP, FGF-2, FGF8, FGFR1, FGFR2, FGFR3, FGFR4, FLT-3, folate receptor α (FRα), FSP -1, GAL3ST1, G-CSF, G-CSFR, GD2, GITR, GLUT1, GLUT4, GM-CSF , GM-CSFR, GP Ilb/IIIa receptor, Gpl30, GPIIB/IIIA, GPNMB, GRP78, HER2/neu, HER3, HER4, HGF, hGH, HLA-DR, HVEM, hyaluronidase, ICOS, IFNα, IFNβ, IFNγ, IgE , IgE receptor (FceRI), IGF, IGF1R, IL1B, IL1R, IL2, IL11, IL12, IL12p40, IL-12R, IL-12Rβl, IL13, IL13R, IL13Ra2, IL15, IL17, IL18, IL21, IL23, IL23R, IL27 /IL27R (wsxl), IL29, IL-31R, IL31/IL31R, IL2R, IL4, IL4R, IL6, IL6R, IL1 receptor accessory protein (IL1RAP), insulin receptor, Jagged ligand, Jagged 1, Jagged 2, KISS1-R, KLRG1, LAG-3, LIF-R, Lewis X, LIGHT, LRP4, LRRC26, Ly6G6D, LyPD1, MCSP, mesothelin, MRP4, MUC1, mucin-16 (MUC16, CA-125), Na/K ATPase, NGF, Nicastrin, Notch receptor, Notch 1, Notch 2, Notch 3, Notch 4, NOV, OSM-R, OX-40, PAR2, PDGF-AA, PDGF-BB, PDGFRα, PDGFRβ, PD-1, PD-L1, PD-L2, phosphatidylserine, P1GF, PSCA, PSMA, PSGR, RAAG12, RAGE, SLC44A4, Siglecl5, STEAP1, STEAP2, TAG-72, TAPA1, TEM-8, TGFβ, TIGIT, TIM-3, TLR2, TLR4, TLR6, TLR7, TLR8, TLR9, TMEM31, TNFα, TNFR, TNFRS12A, TRAIL-R1, TRAIL-R2, transferrin, transferrin receptor, TRK-A, TRK-B, uPAR, VAP1, VCAM-1, VEGF, VEGF-A, VEGF-B, VEGF-C, VEGF -D, VEGFR1, VEGFR2, VEGFR3, VISTA, WISP-1, WISP-2, WISP-3, or a combination thereof.

Preferably, the multispecific antibody comprises one or more second antigen-binding regions, or further comprises a third antigen-binding region.

A preferred bispecific antibody is an anti-BAFF-R and anti-CD3 bispecific antibody. The bispecific antibody of the present invention can specifically bind to B-cell activating factor receptor (BAFF-R) on the cell surface and cluster of differentiation 3 (CD3) on the surface of immune cells; wherein the anti-BAFF-R antibody is a fully human single-domain antibody. The bispecific antibody of the present invention is a symmetric or asymmetric dimer, such as bispecific antibodies in different combination configurations of 1+1, 2+1 and 2+2.

Preferably, the bispecific antibody has the following structure:
(a) two heavy chains, the first heavy chain comprising, from N-terminus to C-terminus: anti-BAFF-R VH and human IgG Fc region; the second heavy chain comprising, from N-terminus to C-terminus: anti-CD3 VH, antibody heavy chain constant region (CH1), and human IgG Fc region;
(b) one light chain, comprising, from N-terminus to C-terminus: anti-CD3 VL and antibody light chain constant region (CL),

the anti-BAFF-R VH is the heavy chain variable region of a single-domain antibody targeting CD3,
the anti-CD3 VL pairs with the anti-CD3 VH, and the antibody heavy chain constant region (CH1) pairs with the antibody light chain constant region (CL). In an embodiment of the present application, the bispecific antibody (VH-Fab-Fc 1+1) comprises: a first heavy chain having the amino acid sequence set forth in SEQ ID NO. 51, a second heavy chain having the amino acid sequence set forth in SEQ ID NO. 73, and a light chain having the amino acid sequence set forth in SEQ ID NO. 71.

In another preferred embodiment, the bispecific antibody has the following structure:
(a) two heavy chains, the first heavy chain comprising, from N-terminus to C-terminus: anti-BAFF-R VH, anti-CD3 VH, antibody heavy chain constant region (CH1), and human IgG Fc region; the second heavy chain comprising, from N-terminus to C-terminus: anti-BAFF-R VH and human IgG Fc region;
(b) one light chain, comprising, from N-terminus to C-terminus: anti-CD3 VL and antibody light chain constant region (CL),

the anti-BAFF-R VH is the heavy chain variable region of a single-domain antibody targeting CD3,
the anti-CD3 VL pairs with the anti-CD3 VH, and the antibody heavy chain constant region (CH1) pairs with the antibody light chain constant region (CL). In an embodiment of the present application, the bispecific antibody (VH2-Fab-Fc 2+1) comprises: a first heavy chain having the amino acid sequence set forth in SEQ ID NO. 51, a second heavy chain having the amino acid sequence set forth in SEQ ID NO. 75, and a light chain having the amino acid sequence set forth in SEQ ID NO. 71.

In another preferred embodiment, the bispecific antibody has the following structure:
(a) two heavy chains, each comprising, from N-terminus to C-terminus: anti-BAFF-R VH, anti-CD3 VH, antibody heavy chain constant region (CH1), and human IgG Fc region;
(b) two light chains, each comprising, from N-terminus to C-terminus: anti-CD3 VL and antibody light chain constant region (CL),

the anti-BAFF-R VH is the heavy chain variable region of a single-domain antibody targeting CD3,
the anti-CD3 VL pairs with the anti-CD3 VH, and the antibody heavy chain constant region (CH1) pairs with the antibody light chain constant region (CL). In an embodiment of the present application, the bispecific antibody (VH2-Fab2-Fc 2+2) comprises: a first heavy chain having the amino acid sequence set forth in SEQ ID NO. 51, a second heavy chain having the amino acid sequence set forth in SEQ ID NO. 77, and a light chain having the amino acid sequence set forth in SEQ ID NO. 71.

### Chimeric antigen receptor (CAR)

The chimeric antigen receptor (CAR) of the present invention comprises an extracellular domain, a transmembrane domain, and an intracellular domain. The extracellular domain comprises a target-specific binding element (also called an antigen-binding domain). The intracellular domain comprises a co-stimulatory signaling region and a ζ chain moiety. The co-stimulatory signaling region refers to a portion of the intracellular domain that comprises a co-stimulatory molecule. Co-stimulatory molecules are cell-surface molecules required for an effective response of lymphocytes to an antigen, other than antigen receptors or their ligands.

The linker can be incorporated between the extracellular domain and the transmembrane domain of the CAR, or between the cytoplasmic domain and the transmembrane domain of the CAR. As used herein, the term "linker" generally refers to any oligopeptide or polypeptide that functions to link the transmembrane domain to the extracellular domain or the cytoplasmic domain of a polypeptide chain. The linker may comprise 0 to 300 amino acids, preferably 2 to 100 amino acids, and most preferably 3 to 50 amino acids.

In a preferred embodiment of the present invention, the extracellular domain of the CAR provided by the present invention comprises an antigen-binding domain targeting BAFF-R. When expressed in T cells, the CAR of the present invention is capable of antigen recognition based on antigen-binding specificity. When it binds to its cognate antigen, it affects tumor cells, resulting in inhibition of tumor cell growth, induction of tumor cell death, or other effects, and leads to reduction or elimination of tumor burden in a patient. The antigen-binding domain is preferably fused with one or more intracellular domains from the co-stimulatory molecule and the ζ chain. Preferably, the antigen-binding domain is fused to an intracellular domain combining a 4-1BB signaling domain and a CD3ζ signaling domain.

As used herein, the "antigen-binding domain" means a Fab fragment, a Fab' fragment, an F(ab')2 fragment, or a single Fv fragment with antigen-binding activity.

In the present invention, fully human phage libraries including single-domain libraries are used for antibody screening, and fully human single-domain antibodies with lower immunogenicity than humanized murine antibodies are obtained. CAR vectors whose antigen-binding domain comprises one or more single-domain antibodies that specifically recognize BAFF-R are constructed, and CAR-T cells are further prepared.

### Vector

The present invention includes a DNA construct comprising a CAR sequence, wherein the sequence comprises a nucleic acid sequence of an antigen-binding domain operably linked to a nucleic acid sequence of a signaling domain.

Nucleic acid sequences encoding desired molecules can be obtained using recombinant methods known in the art, such as, for example, by screening libraries from cells expressing the gene, by deriving the gene from a vector known to comprise the gene, or by direct isolation from cells and tissues containing the gene using standard techniques. Alternatively, genes of interest can be produced synthetically.

The present invention also provides a vector into which the DNA of the present invention is inserted. Vectors derived from retroviruses such as lentiviruses are suitable tools to achieve long-term gene transfer, since they allow long-term, stable integration of the transgene and its propagation in daughter cells.

Briefly, expression of a native or synthetic nucleic acid encoding a CAR is generally achieved by operably linking a nucleic acid encoding a CAR polypeptide or a portion thereof to a promoter and incorporating the construct into an expression vector. The vector is suitable for replication and integration in eukaryotic cells. Typical cloning vectors comprise transcription and translation terminators, initiation sequences, and promoters useful for regulating the expression of the desired nucleic acid sequence.

The nucleic acid can be cloned into many types of vectors. For example, the nucleic acid can be cloned into such vectors, including but not limited to plasmids, phagemids, phage derivatives, animal viruses, and cosmids. Specific vectors of interest include expression vectors, replication vectors, probe generating vectors and sequencing vectors.

Further, the expression vector can be provided to the cell in the form of a viral vector. Viruses useful as vectors include, but are not limited to, retroviruses, adenoviruses, adeno-associated viruses, herpesviruses, and lentiviruses. In general, suitable vectors comprise an origin of replication functional in at least one organism, a promoter sequence, convenient restriction endonuclease sites, and one or more selectable markers.

Many virus-based systems have been developed for transferring genes into mammalian cells. For example, retroviruses provide a convenient platform for gene delivery systems. The selected genes can be inserted into vectors and packaged into retroviral particles using techniques known in the art. The recombinant virus can then be isolated and delivered to target cells in vivo or ex vivo. Many retroviral systems are known in the art. In some embodiments, adenoviral vectors are used. Many adenoviral vectors are known in the art. In an embodiment, lentiviral vectors are used.

Additional promoter elements, such as enhancers, can adjust the frequency at which transcription begins. Typically, these are located in the region 30-110 bp upstream of the initiation site, although it has recently been shown that many promoters also contain functional elements downstream of the initiation site. The spacing between promoter elements is often flexible so that the promoter function is maintained when the element is inverted or moved relative to one another. In the thymidine kinase (tk) promoter, activity begins to decrease only when the spacing between promoter elements is increased to 50 bp apart. Depending on the promoter, individual elements may act cooperatively or independently to initiate transcription.

An example of a suitable promoter is the immediate-early cytomegalovirus (CMV) promoter sequence. The promoter sequence is a strong constitutive promoter sequence capable of driving high level expression of any polynucleotide sequence operably linked thereto. Another example of a suitable promoter is elongated growth factor-1α (EF-1α). However, other constitutive promoter sequences may also be used, including, but not limited to, simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate-early promoter, Rous sarcoma virus promoter, and human gene promoters such as, but not limited to, actin promoter, myosin promoter, hemoglobin promoter, and creatine kinase promoter. Further, the present invention should not be limited to the use of constitutive promoters. Inducible promoters are also considered as part of the present invention. The use of inducible promoters provides molecular switches that can turn on expression of a polynucleotide sequence operably linked to an inducible promoter when such expression is desired, or turn off expression when expression is undesired. Examples of inducible promoters include, but are not limited to, metallothionein promoters, glucocorticoid promoters, progesterone promoters, and tetracycline promoters.

To evaluate the expression of a CAR polypeptide or a portion thereof, the expression vector introduced into a cell may also comprise either or both of a selectable marker gene or a reporter gene, to facilitate the identification and selection of expressing cells from a population of cells sought to be transfected or infected via a viral vector. In other aspects, selectable markers may be carried on a separate segment of DNA and used in co-transfection procedures. Both the selectable marker and the reporter gene may be flanked by appropriate regulatory sequences to enable expression in a host cell. Useful selectable markers include, for example, antibiotic resistance genes, such as neo, and the like.

The reporter gene is used to identify potentially transfected cells and to evaluate the functionality of regulatory sequences. Generally, a reporter gene is a gene that is not present in, or expressed by, a recipient organism or tissue, and encodes a polypeptide whose expression is clearly indicated by some readily detectable properties, such as enzymatic activity. After the DNA has been introduced into the recipient cell, the expression of the reporter gene is measured at an appropriate time. Suitable reporter genes may include genes encoding luciferase, β-galactosidase, chloramphenicol acetyltransferase, secreted alkaline phosphatase, or green fluorescent protein (e.g., Ui-Tei et al., 2000 FEBS Letters 479:79-82). Suitable expression system are known and can be prepared use known techniques or obtained commercially. Typically, constructs with at least 5 flanking regions that show the highest levels of reporter gene expression are identified as promoters. Such promoter regions may be linked to reporter genes and used to evaluate the ability of reagents to regulate promoter-driven transcription.

Methods of introducing genes into cells and of expressing genes into cells are known in the art. In the context of an expression vector, the vector can be readily introduced into a host cell, e.g., a mammalian, bacterial, yeast, or insect cell, by any method known in the art. For example, expression vectors can be transferred into host cells by physical, chemical or biological means.

Physical methods for introducing polynucleotides into host cells include calcium phosphate precipitation, lipid transfection, particle bombardment, microinjection, electroporation and so on. Methods of producing cells comprising vectors and/or exogenous nucleic acids are known in the art. See e.g. Sambrook et al. (2001, Molecular Cloning: A Laboratory Manual, Cold Spring Harbor Laboratory, New York). A preferred method of introducing polynucleotides into host cells is calcium phosphate transfection.

Biological methods of introducing a polynucleotide of interest into a host cell include the use of DNA and RNA vectors. Viral vectors, especially retroviral vectors, have become the most widely used method for inserting genes into mammalian, e.g., human, cells. Other viral vectors may be derived from lentivirus, poxvirus, herpes simplex virus I, adenovirus, adeno-associated virus, and the like.

Chemical means for introducing a polynucleotide into a host cell include colloidal dispersion systems, such as macromolecular complexes, nanocapsules, microspheres, beads; and lipid-based systems including oil-in-water emulsions, micelles, mixed micelles, and liposomes. Exemplary colloidal systems used as delivery vehicles in vitro and in vivo are liposomes (e.g., artificial membrane vesicles).

In the case of using a non-viral delivery system, an exemplary delivery vehicle is a liposome. The use of lipid formulations is contemplated for the introduction of nucleic acids into host cells (in vitro, ex vivo, or in vivo). In another aspect, the nucleic acid may be associated with a lipid. Nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a lipid-containing solution, mixed with a lipid, combined with a lipid, contained as a suspension in a lipid, contained in or complexed with a micelle, or otherwise associated with a lipid. The lipids, lipid/DNA or lipid/expression vectors associated with the compositions are not limited to any specific structure in the solution. For example, they may exist in bilayer structures as micelles or have a "collapsed" structure. They can also be simply interdispersed in a solution, possibly forming aggregates of varying size or shape. Lipids are fatty substances which may be naturally occurring or synthetic lipids. For example, lipids include fatty droplets that naturally occur in the cytoplasm as well as those compounds containing long-chain aliphatic hydrocarbons and their derivatives such as fatty acids, alcohols, amines, amino alcohols, and aldehydes.

In a preferred embodiment of the invention, the vector is a lentiviral vector. The present inventors have confirmed through research that the use of this lentiviral vector to construct the CAR of the present invention results in high transfection efficiency into T cells and high reproducibility.

In a preferred embodiment of the present invention, the vector further comprises a signal peptide sequence. Preferably, the signal peptide sequence is linked upstream of the nucleic acid sequence encoding the antigen-binding domain.

### Preparation of antibodies

The DNA molecule sequences of the antibodies or fragments thereof of the present invention can be obtained by conventional techniques, such as PCR amplification or genomic library screening. In addition, the coding sequences for the light and heavy chains can be fused together to form a single-chain antibody.

Once the relevant sequences are obtained, they can be obtained in large quantities by recombination. This generally involves cloning them into a vector, transferring the vector into a cell, and then isolating the relevant sequences from the propagated host cells by conventional methods.

In addition, the relevant sequences can also be synthesized by artificial synthesis, especially when the fragment length is short. Typically, long sequences can be obtained by first synthesizing multiple small fragments and then ligating them.

At present, the DNA sequence encoding the antibody (or fragment thereof, or derivative thereof) of the present invention can be obtained entirely by chemical synthesis. The DNA sequence can then be introduced into various existing DNA molecules (or vectors, for example) and cells known in the art. In addition, mutations can also be introduced into the protein sequence of the present invention by chemical synthesis.

The present invention also relates to vectors comprising the appropriate DNA sequences described above, together with appropriate promoters or control sequences. These vectors can be used to transform appropriate host cells to enable them to express proteins.

Host cells can be prokaryotic cells, such as bacterial cells; lower eukaryotic cells, such as yeast cells; or higher eukaryotic cells, such as mammalian cells. Preferred animal cells include (but are not limited to): CHO-S, HEK-293 cells.

Typically, the transformed host cells are cultured under conditions suitable for the expression of the antibodies of the present invention. The antibodies of the present invention are then purified by conventional immunoglobulin purification procedures, such as protein A-Sepharose, hydroxyapatite chromatography, gel electrophoresis, dialysis, ion exchange chromatography, hydrophobic chromatography, size-exclusion chromatography, or affinity chromatography, which are routine separation and purification means well known to those skilled in the art.

The resulting monoclonal antibody can be identified by conventional means. For example, the binding specificity of monoclonal antibodies can be determined by immunoprecipitation or in vitro binding assays such as radioimmunoassay (RIA) or enzyme-linked immunosorbent assay (ELISA). The binding affinity of monoclonal antibodies can be determined, for example, by Scatchard analysis as described by Munson et al., Anal. Biochem., 107:220 (1980).

The antibodies of the present invention can be expressed intracellularly, on the cell membrane, or secreted extracellularly. If desired, the recombinant proteins can be isolated and purified by various separation methods utilizing their physical, chemical, and other properties. These methods are well known to those skilled in the art. Examples of such methods include, but are not limited to: conventional renaturation treatment, treatment with protein precipitants (salting-out method), centrifugation, osmotic lysis, sonication, ultracentrifugation, size-exclusion chromatography (gel filtration), adsorption chromatography, ion exchange chromatography, high performance liquid chromatography (HPLC), and various other liquid chromatography techniques, as well as combinations of these methods.

### Immunoconjugates

The present invention also provides immunoconjugates (ADCs) based on the antibodies of the present invention, preferably nanobody-drug conjugates (NDCs).

Typically, the antibody-drug conjugate comprises the antibody and an effector molecule, wherein the antibody is conjugated to the effector molecule, preferably by chemical conjugation. The effector molecule is preferably a therapeutically active drug. In addition, the effector molecule may be one or more of a toxic protein, a chemotherapeutic drug, a small molecule drug or a radionuclide.

The antibody of the present invention may be conjugated to the effector molecule via a coupling agent. Examples of the coupling agent include any one or more of non-selective coupling agents, carboxyl-utilizing coupling agents, peptide chains, and disulfide bond-utilizing coupling agents. The non-selective coupling agent refers to a compound such as glutaraldehyde, etc., which enables the effector molecule and the antibody to form a covalent bond. The carboxyl-utilizing coupling agent may be any one or more of cis-aconitic anhydride-based coupling agents (e.g., cis-aconitic anhydride) and acylhydrazone-based coupling agents (with an acylhydrazone as the conjugation site).

Certain residues on the antibody (such as Cys or Lys) are used for attachment to various functional groups, including imaging agents (e.g., chromophores and fluorophores), diagnostic agents (e.g., MRI contrast agents and radioisotopes), stabilizing agents (e.g., ethylene glycol polymers), and therapeutic agents. The antibody may be conjugated to a functional agent to form an antibody-functional agent conjugate. The functional agent (e.g., drug, detection reagent, stabilizer) is conjugated (covalently linked) to the antibody. The functional agent may be attached to the antibody directly or indirectly through a linker.

A nanobody may be conjugated to a drug to form an antibody-drug conjugate (NDC). Typically, the NDC comprises a linker located between the drug and the antibody. The linker may be a degradable or nondegradable linker. A degradable linker is readily degradable under intracellular conditions, for example, the linker degrades at the target site, thereby releasing the drug from the antibody. Suitable degradable linkers include, for example, enzymatically degradable linkers, including peptide-containing linkers degradable by intracellular proteases (e.g., lysosomal proteases or endosomal proteases), or sugar linkers such as glucuronide-containing linkers degradable by glucuronidase. The peptide-based linker may include, for example, dipeptides, such as valine-citrulline, phenylalanine-lysine, or valine-alanine. Other suitable degradable linkers include, for example, a pH sensitive linker (e.g., a hydrazone linker when pH is less than 5.5) and a disulfide linker that degrades under reducing conditions (e.g., a disulfide linker). The nondegradable linker typically releases the drug under conditions where the antibody is hydrolyzed by the protease.

Prior to conjugation to the antibody, the linker bears reactive functional groups capable of reacting with certain amino acid residues, and conjugation is achieved via such reactive groups. Thiol-reactive functional groups are preferred and include, for example: maleimide compounds; haloacetamides (e.g., iodo-, bromo-, or chloro-substituted); haloesters (e.g., iodo-, bromo-, or chloro-substituted); halomethyl ketones (e.g., iodo-, bromo-, or chloro-substituted); benzyl halides (e.g., iodo-, bromo-, or chloro-substituted); vinyl sulfones; pyridyl disulfides; mercury derivatives such as 3,6-di(mercuromethyl)dioxane, wherein the counterion is acetate, chloride, or nitrate; and polymethylene dimethyl sulfide thiosulfonates. The linker may include, for example, maleimide linked to an antibody by thiosuccinimide.

The drug may be any cytotoxic, cytostatic, or immunosuppressive drug. In embodiments, the linker links the antibody to the drug, and the drug bears a functional group capable of forming a bond with the linker. For example, the drug may have an amino group, carboxyl group, thiol group, hydroxyl group, or ketone group capable of bonding to the linker. When the drug is directly attached to the linker, the drug bears a reactive functional group prior to conjugation to the antibody.

Useful classes of drugs include, for example, antitubulin drugs, DNA minor groove-binding agents, DNA replication inhibitors, alkylating agents, antibiotics, folate antagonists, antimetabolites, chemosensitizers, topoisomerase inhibitors, vinca alkaloids, and the like. Examples of particularly useful cytotoxic drug classes include, for example, DNA minor groove-binding agents, DNA alkylating agents, and tubulin inhibitors. Representative cytotoxic drugs include, for example, auristatins, camptothecins, duocarmycins, etoposides, maytansines and maytansinoids (e.g., DM1 and DM4), taxanes, benzodiazepines or benzodiazepine-containing drugs (e.g., pyrrolo[1,4]benzodiazepines (PBDs), indolinobenzodiazepines, and oxazolidinobenzodiazepines), and vinca alkaloids.

In the present invention, the drug-linker may be use to form an NDC in a single step. In other embodiments, the bifunctional linker compound may be used to form an NDC in a two-step or multi-step process. For example, a cysteine residue reacts with the reactive moiety of the linker in a first step, and in a subsequent step, a functional group on the linker reacts with the drug to form the NDC.

Generally, functional groups on the linker are chosen to facilitate specific reaction with suitable reactive groups on the drug moiety. As a non-limiting example, azide-based moieties can be used to react specifically with reactive alkyne groups on the drug moiety. The drug is covalently attached to the linker via a 1,3-dipolar cycloaddition between the azide and the alkyne. Other useful functional groups include, for example, ketones and aldehydes (suitable for reaction with hydrazides and alkoxyamines), phosphines (suitable for reaction with azides); isocyanates and isothiocyanates (suitable for reaction with amines and alcohols); and activated esters, such as N-hydroxysuccinimide esters (suitable for reaction with amines and alcohols). These and other conjugation strategies, such as those described in Bioconjugate Techniques, Second Edition (Elsevier), are well known to those skilled in the art. Those skilled in the art will appreciate that for selective reaction of the drug moiety and the linker, when a complementary pair of reactive functional groups is selected, each member of the complementary pair can be used either on the linker or on the drug.

The present invention also provides a method for preparing an NDC, further comprising: combining the antibody with a drug-linker compound under conditions sufficient to form the antibody conjugate (NDC).

In some embodiments, the method of the present invention comprises: combining the antibody with a bifunctional linker compound under conditions sufficient to form an antibody-linker conjugate. In these embodiment, the method of the present invention further comprises: combining the antibody-linker conjugate with the drug moiety under conditions sufficient to covalently attach the drug moiety to the antibody via the linker.

In some embodiments, the structure of the immunoconjugate, preferably a nanobody-drug conjugate NDC, is represented by the following formula: 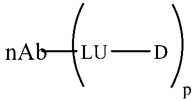 where
nAb represents the aforementioned single-domain antibody targeting BAFF-R, antibody targeting BAFF-R or multispecific antibody,
LU represents a linker;
D represents a drug;
and the subscript p is a value selected from 1 to 10.

### Uses

The present invention further provides the use of the antibodies of the present invention, for example in the preparation of diagnostic formulations, or in the preparation of drugs for the prevention and/or treatment of BAFF-R-associated diseases. The BAFF-R-associated diseases include tumorigenesis, growth and/or metastasis, tumor drug resistance-related diseases, autoimmune diseases, inflammation, and metabolism-related diseases, etc.

The uses of the antibodies or ADCs of the present invention include, but are not limited to:
(i) Diagnosis, prophylaxis and/or treatment of tumorigenesis, growth and/or metastasis, especially tumors with high BAFF-R expression. The tumors include (but are not limited to): leukemia, lymphoma, myeloma, breast cancer (e.g., triple-negative breast cancer), lung cancer (e.g., non-small cell lung cancer), pancreatic cancer, malignant glioma, gastric cancer, liver cancer, esophageal cancer, renal cancer, colorectal cancer, bladder cancer, prostate cancer, endometrial cancer, ovarian cancer, cervical cancer, angiosarcoma, etc.; especially triple-negative breast cancer, non-small cell lung cancer, pancreatic cancer, malignant glioma, and more preferably triple-negative breast cancer and/or non-small cell lung cancer.
(ii) Diagnosis, prophylaxis and/or treatment of autoimmune diseases. The autoimmune diseases include (but are not limited to): systemic lupus erythematosus, rheumatoid arthritis, ulcerative colitis, type I diabetes, psoriasis, multiple sclerosis, myasthenia gravis, Sjögren's syndrome, immune thrombocytopenia, immune hemolysis.
(iii) Diagnosis, prophylaxis and/or treatment of inflammation. The inflammation includes (but is not limited to): rheumatoid arthritis, osteoarthritis, ankylosing spondylitis, gout, Reiter's syndrome, psoriatic arthropathy, infectious arthritis, tuberculous arthritis, viral arthritis, fungal arthritis, glomerulonephritis, systemic lupus erythematosus, Crohn's disease, ulcerative colitis, acute lung injury, chronic obstructive pulmonary disease, idiopathic pulmonary fibrosis.
(iv) Diagnosis, prophylaxis and/or treatment of metabolism-related diseases. The metabolism-related diseases include (but are not limited to): diabetes, diet-induced obesity and adipose inflammation.

### Pharmaceutical compositions

The present invention also provides a composition. In a preferred embodiment, the composition is a pharmaceutical composition comprising the aforementioned antibody, or an active fragment thereof, or a fusion protein thereof, or an NDC thereof, or corresponding CAR-T cells, and a pharmaceutically acceptable carrier. In general, these substances can be formulated in non-toxic, inert and pharmaceutically acceptable aqueous carrier media. The formulated pharmaceutical composition can be administered by conventional routes including (but not limited to): intratumoral, intraperitoneal, intravenous, or topical administration.

The antibody of the present invention can also be expressed intracellularly from a nucleotide sequence for use in cell therapy, for example, the antibody can be used in chimeric antigen receptor T-cell immunotherapy (CAR-T) and the like.

The pharmaceutical composition of the present invention can be directly used for binding to BAFF-R protein molecules, and thus can be used for the prevention and treatment of diseases such as tumors. In addition, other therapeutic agents may be used concurrently.

The pharmaceutical composition of the present invention comprises a safe and effective amount (e.g., 0.001-99wt%, preferably 0.01-90wt%, preferably 0.1-80wt%) of the aforementioned monoclonal antibody (or conjugate thereof) of the present invention, and a pharmaceutically acceptable carrier or excipient. Such carriers include, but are not limited to, saline, buffer, glucose, water, glycerol, ethanol, and combinations thereof. The pharmaceutical formulation should be compatible with the mode of administration. The pharmaceutical composition of the present invention can be prepared as an injection, for example, by conventional methods using physiological saline or an aqueous solution containing glucose and other adjuvants. Pharmaceutical compositions such as injections and solutions are preferably produced under sterile conditions. The dosage of the active ingredient is a therapeutically effective amount, for example, about 1 µg/kg body weight to about 5 mg/kg body weight per day. In addition, the polypeptide of the present invention can also be used in combination with other therapeutic agents.

When administering the pharmaceutical composition, a safe and effective amount of the immunoconjugate is administered to a mammal, wherein such safe and effective amount is typically at least about 10 ug/kg bw, and in most cases not more than about 50 mg/kg bw, preferably from about 10 ug/kg bw to about 20 mg/kg bw. Of course, the specific dosage should also take into account factors such as the route of administration and the patient's health status, which are within the skill of a skilled physician.

For NDCs, since the nanobody-drug conjugates provided by the present invention can target specific cell populations and bind to specific proteins (antigens) on the cell surface, thereby releasing the drug in an active form into the cell via conjugate internalization or drug penetration, the nanobody-drug conjugates of the present invention can be used for the treatment of target diseases. The antibody-drug conjugates mentioned above can be administered to a subject (e.g., a human) in a therapeutically effective amount via an appropriate route. The subject in need of treatment may be a patient at risk of, or suspected of having, a disorder associated with the activity or expression level of a particular antigen. Such patients can be identified by routine physical examination.

When treating with the nanobody-drug conjugates of the present invention, delivery can be performed by conventional methods in the art. For example, they can be introduced into cells using liposomes, hydrogels, cyclodextrins, biodegradable nanocapsules, or bioadhesive microspheres. Alternatively, the nucleic acid or vector can be delivered locally by direct injection or by means of an infusion pump.

### Main advantages of the present invention

(1) The present invention employs fully human phage for antibody screening, and directly obtains a series of fully human monoclonal antibodies with excellent performance. Compared with traditional hybridoma technology, the difficult humanization step of murine antibodies is eliminated. Moreover, fully human antibodies exhibit lower immunogenicity than humanized murine antibodies, and have great application potential in the development of antibody drugs (including monoclonal antibodies, bispecific antibodies, ADCs, etc.) and cell therapy drugs (including CAR-T, CAR-NK, etc.).
(2) The high-affinity specific antibodies provided by the present invention can also be used for the development of detection reagents.
(3) The nanobodies targeting BAFF-R of the present invention show cross-species reactivity and can bind to human, cynomolgus, and mouse BAFF-R proteins.
(4) The bispecific antibodies targeting both BAFF-R nanobodies and CD3 of the present invention can specifically and effectively mediate the killing of BAFF-R-positive target cells by PBMCs, and exhibit favorable biological activity and safety in vivo.

The present invention will be further described with reference to specific examples. It should be understood that these examples are for illustrative purposes only and are not intended to limit the scope of the present invention. The experimental methods without specifying detailed conditions in the following examples are generally performed according to conventional conditions, such as those described in Sambrook et al., Molecular Cloning: A Laboratory Manual (New York: Cold Spring Harbor Laboratory Press, 1989), or according to the conditions recommended by the manufacturer. Unless otherwise stated, percentages and parts are by weight.

### Example 1. Enrichment of specific antibody clones targeting BAFF-R protein from phage antibody library by affinity panning

Appropriate negative and positive panning strategies were used to enrich the specific antibody clones we needed from a phage antibody library.

### Construction of phage antibody library

The phage antibody libraries we constructed include natural libraries, semi-synthetic libraries and single-domain libraries. The semi-synthetic phage antibody library, used together with the natural library, solves the problem that the natural library may lack BAFF-R high affinity antibody clones. The single-domain phage antibody libraries are antibody libraries composed only of variable region amino acids of heavy chain antibodies, which have the molecular weight of only 12-15 kDa, but have similar or higher specificity and affinity than traditional antibodies. In addition, single-domain antibodies have attracted much attention due to their stable physicochemical properties, high affinity, easy recombinant expression and preparation, and ready combination with antibodies against other targets or epitopes.

### BAFF-R protein panning

Multiple rounds of panning were performed using BAFF-R-his-Bio as the positive panning protein to obtain a phage pool enriched with antibody clones of interest. The experimental steps are briefly described as follows:
1) Incubate SA magnetic beads with blocking buffer for 2 h, then bind the target antigen (BAFF-R-his-Bio) to the blocked SA magnetic beads;
2) Add the phage library (containing 5x10¹² phage particles) and an aliquot of unbound SA magnetic beads, and incubate to subtract phage antibody clones that non-specifically bind to SA magnetic beads;
3) After incubation, transfer the supernatant to the SA magnetic beads conjugated with the target antigen, and continue incubation to allow phage binding to the target antigen;
4) Wash the magnetic beads with washing buffer to remove unbound phages;
5) Elute the positive phages from the target antigen with elution buffer, then neutralize with neutralization buffer;
6) Re-infect host bacteria XL1-Blue with the eluted phages to amplify the recovered phages. Take a small portion of the sample for serial dilution, infect host bacteria, spread on Amp-resistant plates, and count the number of recovered phages;
7) Repeat steps 1) to 6). Typically, 3 to 4 rounds of panning are required until a significant increase in phage recovery rate (eluted phage count / input phage count) is observed.

The enriched phage pool can be used for subsequent monoclonal selection and ELISA/FACS screening.

### Main materials and reagents:

Fully humanized phage antibody library, including natural library, semi-synthetic library and single-domain library;
Helper phage KO7, Thermo/Invitrogen, 18311019;
Recombinant biotinylated Human BAFF-R Protein, KACTUS, BAFF-R-HM401;
BeaverBeads^{™} Streptavidin, Beaver Bio, 22307-10;
High binding ELSIA plate, Costar, #3590;
Blocking buffer: PBS + 3% BSA;
Washing buffer: PBS + 0.1% Tween 20;
Elution buffer: 0.2M Glycine, pH 2.2; and
Neutralization buffer: 1M Tris, pH 9.1.

### Experimental results:

Using different antibody libraries, 4 rounds of protein panning were performed, and a significant increase in the recovery rate was observed in each panning (Table 1), demonstrating that the antibody clones were effectively enriched.

**Table 1 Experimental results of protein panning**

| Antibody library | Rounds | Recovery rate | Enrichment fold |
|---|---|---|---|
| XL-SD-1 | 1st | 1.20E-05 | / |
| | 2nd | 2.00E-04 | 16.67 |
| | 3rd | 5.00E-04 | 2.50 |
| | 4th | 2.28E-03 | 4.56 |
| XL-NVH | 1st | 5.80E-05 | / |
| | 2nd | 1.30E-04 | 2.24 |
| | 3rd | 1.20E-04 | 0.92 |
| | 4th | 1.72E-03 | 14.33 |
| XL-V1 | 1st | 3.60E-05 | / |
| | 2nd | 5.36E-05 | 1.49 |
| | 3rd | 1.34E-04 | 2.50 |
| | 4th | 2.00E-03 | 14.93 |
| XL-V2 | 1st | 2.20E-05 | / |
| | 2nd | 5.36E-05 | 2.44 |
| | 3rd | 6.00E-05 | 1.12 |
| | 4th | 1.48E-03 | 24.67 |

It can be seen that after 4 rounds of panning, different antibody libraries have been enriched (the recovery rate in the 4th round was significantly higher than that in the previous round).

### Example 2. Screening for specific clones from the enriched phage pool using enzyme-linked immunosorbent assay (ELISA) and fluorescence-activated cell sorting (FACS)

Purpose and principle: The phage pool enriched by affinity panning steps contains phage antibodies of various properties: specific clones, non-specific clones, and negative clones. To obtain specific clones, we perform monoclone isolation, package them into monoclonal phages, and conduct primary screening of a large number of monoclonal phages by enzyme-linked immunosorbent assay (ELISA) and fluorescence-activated cell sorting (FACS) so as to select clones that specifically bind to both the BAFF-R protein and the BAFF-R-positive cell line K562-BAFF-R. The specific monoclone is further confirmed by DNA sequencing to identify the contained unique antibody sequence therein.

In the ELISA primary screening, the biotinylated target antigen (BAFF-R-his-Bio) is maintained in a conformation closer to its native state in the reaction solution via the binding between streptavidin and biotin. Clones that bind specifically to BAFF-R-his-Bio but not to the control protein SA are identified as specific clones. For the FACS primary screening, the BAFF-R-highly expressing positive cell line K562-BAFF-R and the BAFF-R-negative cell line K562 are used. Clones that bind to K562-BAFF-R cells but not to K562 cells are designated as specific clones. Through dual primary screening by ELISA and FACS, we can obtain candidate antibodies that bind both recombinantly expressed BAFF-R protein and native BAFF-R molecules on the cell surface, which are then subjected to subsequent rounds of screening.

### Brief steps of ELISA experiment:

1) Culture and package monoclonal phages in deep-well 96-well plates;
2) Dilute streptavidin to 2 µg/mL with PBS, add 100 µL per well to a high-binding ELISA plate, and incubate for 2 h at room temperature;
3) Discard the coating solution, add 250 µL of blocking buffer per well, and block overnight at 4°C;
4) Wash the plate twice with 250 µL of washing buffer;
5) Dilute the biotin-labeled target protein and control protein to 2 µg/mL with PBS, add 100 µL per well to the streptavidin-precoated ELISA plate, and incubate for 1 h at room temperature;
6) Wash the plate twice with 250 µL of washing buffer;
7) Add 100 µL of the cultured phage supernatant from step 1 to the wells coated with the target antigen, and incubate for 2 h at room temperature;
8) Wash the plate four times with 250 µL of washing buffer;
9) Add 100 µL of 1:2000 diluted mouse anti M13 primary antibody per well, and incubate at a room temperature for 45 min;
10) Wash the plate four times with 250 µL of washing buffer;
11) Add 100 µL of 1:2000 diluted HRP Donkey anti-mouse IgG per well, and incubate at a room temperature for 45 min;
12) Wash the plate six times with 250 µL of washing buffer;
13) Add 100 µL of TMB chromogenic substrate, and develop for 5 to 10 min; and
14) Add 100 µL of 2M H₂SO₄ to terminate the reaction, and read the results using a microplate reader.

### Brief steps of FACS primary screening experiment:

1) Culture and package monoclonal phages in deep-well 96-well plates;
2) Wash Raji and Jurkat cells twice with PBS, resuspend in PBS at a density of 1x10⁷/mL cells/mL, and aliquot at 50 µL into a 96-well deep-well plate;
3) Add 50 µL of packaged monoclonal phages to each well, mix well, and incubate at 4°C for 2 h;
4) Wash with 200 µL of PBS twice;
5) Add 100 µL of 1:2000 diluted mouse anti M13 primary antibody per well, pipette to mix well, and then, incubate at a room temperature for 45 min;
6) Wash with 200 µL of PBS twice;
7) Add 100 µL of 1:300 diluted FITC horse anti mouse-IgG (H+L) per well, pipette to mix well, and then, incubate at a room temperature for 45 min;
8) Wash with 200 µL of PBS twice, and finally, resuspend the cells with 200 µL of PBS; and
9) Detect a sample FITC channel for the fluorescence intensity on a flow cytometer, and analyze results.

### Main materials and reagents:

Helper phage KO7, Thermo/Invitrogen, 18311019;
Streptavidin, Pierce, 21125;
Recombinant biotinylated Human BAFF-R Protein, KACTUS, BAFF-R-HM401;
High binding ELSIA plate, Costar, #3590;
Corning 96-Well Clear Round Bottom TC-Treated Microplate, Costar, #3799;
Blocking buffer: PBS + 3% BSA;
Washing buffer: PBS + 0.1% Tween20;
Soluble one-component TMB substrate solution, Tiangen, PA-107-02;
Anti-M13 Bacteriophage Coat Protein g8p antibody, abcam, ab9225;
HRP Goat anti-mouse IgG(minimal x-reactivity) Antibody, Biolegend, 405306;
FITC horse anti mouse-IgG(H+L), Vector, FI2000.

### Experimental results:

Monoclones were randomly picked from the enriched phage antibody pool and packaged into phages, and then, the binding of monoclonal phages to BAFF-R-his-Bio protein and control protein SA was detected by phage ELISA to find BAFF-R-specific phage antibody clones. The ELISA results of some clones were as shown in FIG. 2. As shown in the figure, clones H1-H2 and H4-H7 exhibited strong binding to the target antigen BAFF-R (BAFF-R-Bio-his) and no binding to the control antigen SA, demonstrating good specificity. Clone H3 showed no binding to either the target antigen BAFF-R-his-Bio or the control protein SA, and was thus identified as a negative clone.

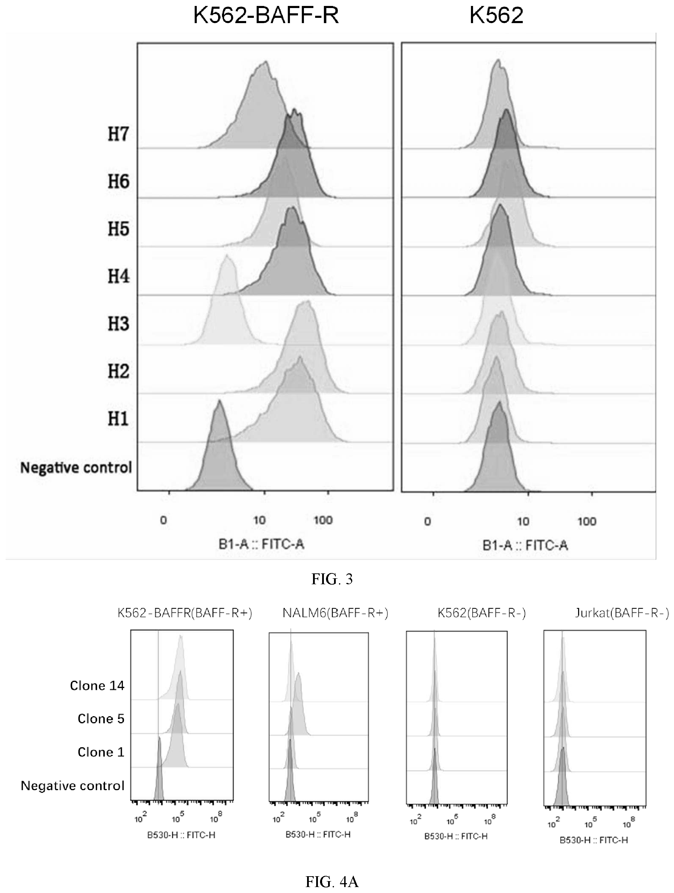

The FACS primary screening results for some clones are presented in FIG. 3. Negative Control denotes the negative control phage antibody clone. Clones H1-H2 and H4-H7 did not bind to K562 cells but bound to K562-BAFF-R cells, indicating they are specific clones. Clone H3 showed no binding to either K562-BAFF-R or K562 cells and was a negative clone.

A total of 39 specific clones were obtained through primary screening by ELISA and FACS.

### Example 3. Identification of monoclonal specificity by FACS using multiple cell lines

Experimental purpose and principle: Antibodies used for treatment must have very good target specificity, only binding to the target antigen and not to any unrelated antigens. On the other hand, the amino acid sequence of the same antigen on different cell lines may be different (isoforms or mutants) or the bound ligands may be different, so it is also necessary to examine whether our antibodies can bind to multiple target protein-positive cells. In order to further analyze the specificity and universality of these monoclones and find the best candidate clones, we further assessed the specificity of the clones from primary screening by flow cytometry. In this experiment, we used multiple BAFF-R-positive cell lines and multiple BAFF-R-negative cell lines to react with these monoclonal phage antibodies to analyze whether these clones can bind to BAFF-R antigens on different cell lines and whether there is any non-specific binding to other cell lines that do not express BAFF-R. Through this experiment, we obtained several clones with excellent specificity.
Experimental method: same as FACS primary screening;

### Main samples and reagents:

K562-BAFF-R cell line: BAFF-R-positive cell line;
K562 cell line: BAFF-R-negative cell line;
NALM6 cell line: BAFF-R-positive cell line;
Jurkat cell line: BAFF-R-negative cell line;
Other reagents: the same as those used in FACS primary screening.

### Experimental results:

Antibodies used for treatment must have very good target specificity. In order to further analyze the specificity of these monoclonal antibodies, we identified the unique clone obtained in Example 2 using enzyme-linked immunosorbent assay and flow cytometry on more antigens and cell lines. The results are shown in FIGS. 4A-B. FIG. 4A shows the flow cytometric histograms of Clones 1, 5, 14 binding to multiple cell lines. Negative Control represents the negative control phage antibody clone. FIG. 4B shows the flow cytometric histograms of Clones 77, 80 binding to multiple cell lines. VAY736 serves as the positive control phage antibody clone. VAY736 antibody bound to all BAFF-R-positive cell lines and showed no binding to BAFF-R-negative cell lines, indicating that the 4 selected cell lines expressed BAFF-R antigen at normal levels. Clones 5, 77, 80 bound to all BAFF-R-positive cell lines. Clones 1, 14 only bound to the BAFF-R-overexpressing cell line K562-BAFF-R and showed no binding to NALM6. All these clones exhibited no binding to BAFF-R-negative cell lines, demonstrating good specificity.

### Example 4. Identification of monoclonal specificity by ELISA using antigens from different companies

Experimental purpose and principle: Antibodies intended for therapeutic use must exhibit excellent target specificity, i.e., binding only to the target antigen but not to any irrelevant antigens. On the other hand, the amino acid sequences of the same antigen produced by different companies may vary (isoforms or mutants). Therefore, it is also necessary to verify whether our antibodies can bind to various forms of the target protein. In order to further analyze the specificity and universality of these monoclones and find the best candidate clones, we further assessed the specificity of the clones from primary screening by enzyme linked immunosorbent assay (ELISA). In this experiment, BAFF-R antigens purchased from different companies and various BAFF-R-unrelated antigens were allowed to react with these monoclonal phage antibodies to determine whether the clones could bind to different BAFF-R antigens and whether there was any non-specific binding to other BAFF-R-unrelated antigens. Through this experiment, we obtained several clones with excellent specificity.
Experimental method: same as ELISA primary screening.

### Main samples and reagents:

**Table 2**

| Abbreviation | Name | Manufacturer | Cat. No. |
|---|---|---|---|
| ACRO-BAFF-R-Fc | Acro-human BAFF-R Protein,his tag | ACRObiosystem | BAFF-R-H52H5 |
| ACRO-CD70-his | Biotinylated Human CD27 Ligand ,His,Avitag | ACRObiosystem | CDL-H82Q9 |
| KACTUS-BAFF-R-his-Bio | Recombinant biotinylated Human BAFF-R Protein | KACTUS | BAFF-R-HM401 |
| KACTUS-CD19-Fc | Recombinant biotinylated Human CD19 Protein | KACTUS | CD2-HE121 |
| SA | Streptavidin | Pierce | 21125 |
| PE anti-human BAFF-R Ab | PE/Dazzle^{™} 594 anti-human CD268 (BAFF-R) Antibody | Biolegend | 316922 |

The remaining reagents are the same as those for the ELISA primary screening.

### Experimental results:

Antibodies used for treatment must have very good target specificity. In order to further analyze the specificity of these monoclonal antibodies, we identified the multiple clones obtained in Example 2 using enzyme-linked immunosorbent assay (ELISA) on various antigens. The results were as shown in FIG. 5. Negative control is a negative control phage antibody clone. anti-M13 phage mouse Ab/anti-mouse HRP Ab is the negative control without phage, with only primary antibody (Anti-M13 phage mouse Ab) and secondary antibody (anti-mouse HRP Ab) added; anti-mouse HRP Ab is the negative control with only secondary antibody (anti-mouse HRP Ab) added, VAY736 Ab/anti-human IgG HRP Ab is the positive control with BAFF-R detection antibody added; and anti-human IgG HRP Ab/anti-his HRP Ab is the positive control for detection of antigen tag antibodies. Clones 1, 5, 77, and 80 bound to both of the two BAFF-R antigens but showed no binding to the non-relevant antigens KACTUS-CD19-Fc, Acro-CD70-his, and SA, indicating that these four clones can recognize BAFF-R antigens in different conformations with good specificity. Clone 14 exhibited weak binding to the non-relevant antigen Acro-CD70-his, and its specificity requires further confirmation.

### Example 5. Detection of EGFRt expression on surface of CAR-T cells

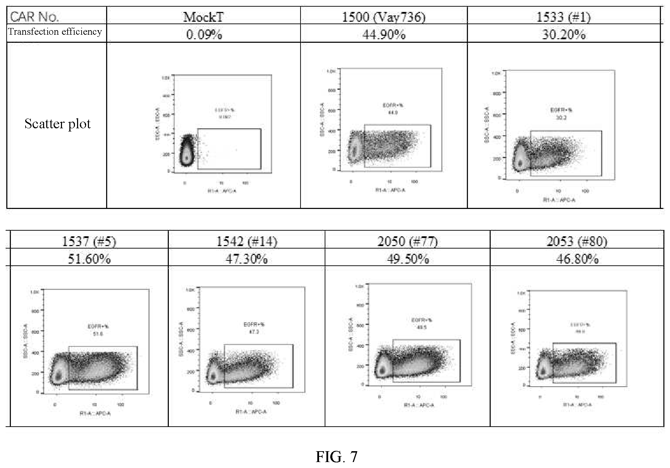

To verify the functions of candidate clones on CAR-T cells, based on the candidate antibody sequences, we constructed a CAR vector with the structure as shown in FIG. 6. The CAR structure comprises a CD8α signal peptide, V_{HH} or scFv, a CD8α hinge region, a CD8α transmembrane region, a 4-1BB co-stimulatory molecule and CD3ζ, and a truncated EFGR molecule (EGFRt) was linked via T2A, which can be used as a safety switch in clinical transformation. The CAR vector was then packaged with a lentivirus, and the expression of EGFRt was detected 5 to 7 days after T cells were transfected with the CAR lentivirus (FIG. 7). Since EGFRt is co-expressed with CAR molecules, it can be used as an indirect detection indicator for the distribution of CAR molecules on the surface of T cells without affecting the structure and function of the CAR.

Brief experimental steps for CAR-T/EGFRt expression detection are as follows:
1) Take 1×10⁶ CAR-T/T cells per well, add PBS to wash once, centrifuge at 300 g for 5 min, and discard the supernatant.
2) Resuspend the cell pellet with 100 µL of PBS, add 5 µL of APC-CD5 antibody and 5 µL of APC-EGFR antibody respectively, and incubate at 4°C in the dark for 15 min.
3) Wash with PBS twice, and centrifuge at 300 g for 5 min.
4) Resuspend with 200 µL of PBS, and detect on flow cytometer.

### Main materials and reagents:

APC anti-human EGFR Antibody, Clone AY13, BioLegend, Cat. No.352906; and
Fetal bovine serum (FBS), Gibco, Cat. No.10099141;

### Experimental results:

As shown in FIG. 7, detection was performed on the seventh day, and the expression of EGFR in T cells in each group was ≥30%.

### Example 7 CD107a degranulation assay

### Experimental principle and purpose:

CD107a is a marker for intracellular microvesicles. CD107a on the cell membrane increases after granzyme-loaded microvesicles fuse with the cell membrane, and when its recovery is blocked by monesin (purchased from BioLegend), it can quantitatively reflect the strength of microvesicle release. When CAR -T cells are stimulated by target antigens on target cells, granzymes are released, and the activation of T cells can be determined by flow cytometric detection of the increase in CD107a.

### Brief experimental steps of CD107a degranulation:

1) Centrifuge the CAR-T cells to be detected and target cells respectively at a room temperature and 300 g for 5 min, discard the supernatant, and then, resuspend the cells with 1640 medium + 10%FBS to 2x10⁶ cells/mL;
2) Add 100 µL of the CAR-T cells to be detected and 100 µL of the target cells to 96-well plates respectively, and mix well;
3) Add 1.5 µL of PE/Cy7 anti-human CD107a antibody and 0.2 µL of monensin to the cells in each well, then incubate in a cell culture incubator at 37°C, 5% CO₂ for 4 h;
4) After completion of incubation, centrifuge at 300 g for 5 min at 4°C, discard the supernatant, and wash the cells twice with 200 µL of PBS;
5) Resuspend the cells with 100 µL of PBS, add 1.5 µL of BV421 anti-human CD8 and 1.5 µL of APC anti-human EGFR antibodies respectively, mix well, and then, incubate on ice in the dark for 20 min; and
6) After completion of incubation, wash the cells with 200 µL of PBS 3 times, resuspend the cells with 100 µL of PBS, and then, detect by a flow cytometer.

### Main samples and reagents:

Target cell NALM6-luc (BAFF-R+), RS4-luc (BAFF-R+), MEC1-luc (BAFF-R+)
JEKO1-luc(BAFF-R+), THP1-luc(BAFF-R-), Molm13-luc(BAFF-R-), CCRF-luc(BAFF-R-), MOLT4-luc(BAFF-R-);
Fetal bovine serum (FBS), Gibco, Cat. No.10099141;
Monensin, BioLegend, Cat. No. 420701;
PE/Cy7 mouse anti-human CD107a, BD, Cat. No. 561348;
BV421 mouse anti-human CD8, BD, Cat. No. 301036;
APC anti-human EGFR Antibody, Clone AY13, BioLegend, Cat. No.35290.

### Experimental results:

CAR-T cells were obtained by lentiviral transduction, and the CAR-T cells were cultured in vitro for 9-12 days before a CD107a degranulation assay. The CAR-T cells to be detected were incubated with target cells, monensin and CD107a antibodies for 4 h, and the cell density of the CAR-T cells and target cells was both 2×10⁵ cells/mL. The samples were then labeled with CD8 antibodies and EGFR antibodies and then subjected to flow cytometric detection. The data were analyzed using FlowJo software. Live cells were gated (P1) in the dot plot to exclude cell debris. Single, dispersed cells were gated (P2) from the P1 population. CD8-positive cells were further gated (P3) from the P2 population. Finally, within the P3 gate, the percentage of CD107a-positive cells among EGFR antibody-stained positive cells (i.e., CAR-positive cells) was analyzed. The analysis results were as shown in Table 3. The degranulation level of BAFF-R CAR-T was significantly increased only after co-incubation with BAFF-R-positive cells, while there was no significant increase in the degranulation level after co-incubation with negative cell lines. Among them, clones 1, 5, 14, 77, and 80 showed high degranulation levels against BAFF-R-positive cells, all of which were higher than those of the control Vay736 CAR (the CAR scFv sequence was derived from Ianalumab).

**Table 3 Results of CD107a degranulation of CAR-T cells in response to different target cells in each group**

| CD107a/CD8+ CAR+% | MockT | 1500 (Vay736 CAR) | 1533 (#1) | 1537 (#5) | 1542 (#14) | 2050 (#77) | 2053 (#80) |
|---|---|---|---|---|---|---|---|
| NALM6-luc (BAFF-R+) | 8.08% | 46.10% | 87.10% | 69.40% | 92.90% | 90.60% | 82.00% |
| RS4-luc (BAFF-R+) | 8.40% | 35.80% | 72.20% | 50.40% | 85.20% | 78.60% | 70.70% |
| MECl-luc (BAFF-R+) | 8.29% | 42.80% | 85.90% | 72.00% | 89.20% | 80.90% | 78.70% |
| JEKO1-luc (BAFF-R+) | 6.54% | 63.50% | 82.90% | 66.70% | 80.10% | 76.70% | 79.30% |
| THP1-luc (BAFF-R-) | 8.25% | 11.00% | 10.70% | 22.10% | 16.40% | 5.87% | 16.10% |
| Molm13-luc (BAFF-R-) | 8.33% | 10.60% | 10.00% | 22.00% | 13.40% | 7.28% | 12.40% |
| CCRF-luc (BAFF-R-) | 6.62% | 11.00% | 7.75% | 11.10% | 10.10% | 5.76% | 6.51% |
| MOLT4-luc (BAFF-R-) | 6.29% | 11.50% | 11.00% | 9.83% | 17.60% | 8.29% | 8.08% |

### Example 8. In vitro cell killing assay

### Experimental purpose and principle:

In the in vitro cell killing assay, human acute B-lymphoblastic leukemia cells NALM6, human acute lymphoblastic leukemia cells RS4, human mucoepidermoid carcinoma cells MEC1, and human mantle cell lymphoma cells JEKO1 were used as BAFF-R-positive target cells, while MOLT4 cells were used as BAFF-R-negative target cells to evaluate the antigen-specific cytotoxicity of BAFF-RCAR-T cells. The above target cells were respectively transduced by lentiviral transduction to obtain target cells that stably express firefly luciferase, so the activity of luciferase in the sample can reflect the number of target cells. CAR-T cells and target cells were co-incubated and cultured. When target cells were killed by CAR-T cells, luciferase was released and quickly inactivated (firefly luciferase has a half-life of about 0.5 h). If the target cells are not killed or inhibited by CAR-T cells, more luciferases will be produced as the target cells proliferate and continue to express luciferase. Therefore, the killing of target cells by CAR-T can be detected by the activity of luciferase.

### Brief experimental steps of in vitro cell killing:

1) Centrifuge the above-mentioned target cells at room temperature and 500 g for 5 min respectively, discard the supernatant, then resuspend the cells with 1640 + 10%FBS medium to 1x10⁵ cells/mL, and add 100 µL of target cells to each well of a 96-well plate, respectively;
2) According to the CAR positive rate and effector-target ratio of the CAR-T sample to be detected, add 100 µL of CAR-T cells to each well of the 96-well plates respectively, and mix well with the target cells; and then, put the mixture in a carbon dioxide incubator to incubate and culture for 24h; and
3) Detect the luciferase activity of the sample in each well using a luciferase assay kit.

### Main samples and reagents:

BAFF-R-positive target cells: NALM6-luc, RS4-luc, MEC1-luc, JEKO1-luc
BAFF-R-negative target cells Raji-luc, CCRF-luc; and
Steady-Glo Luciferase Assay System, Promega, Cat. No. E2520.

### Experimental results:

CAR-T cell samples were mixed with a fixed number of target cells (1x10⁴ cells) at various effector-to-target ratios (E:T), co-incubated for 24 h, and then the luciferase activity (RLU) in the samples was detected. Since the luciferase activity can reflect the number of target cells in a sample, the killing/inhibition ability of CAR-T cells on target cells can be obtained by changes in luciferase activity in the sample. The lower the luciferase activity readout (RLU), the more the target cells killed.

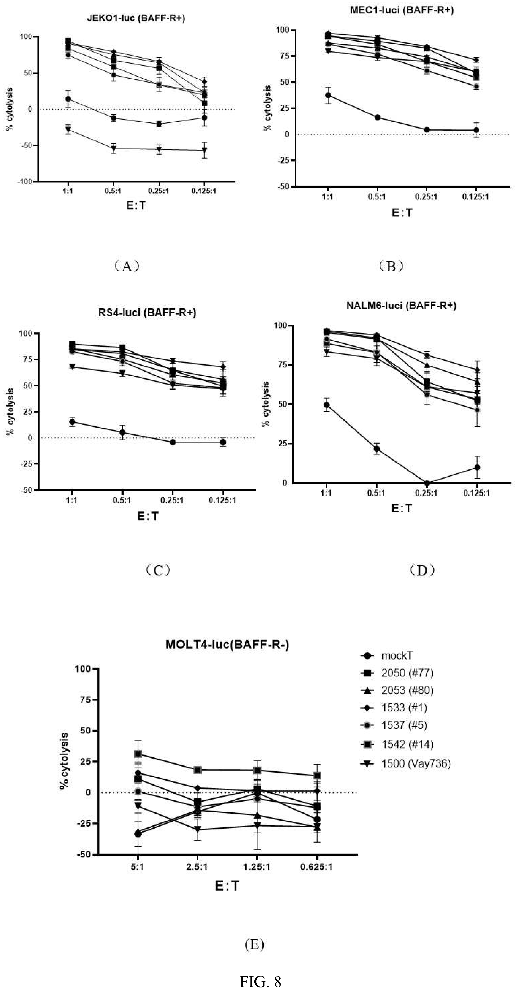

As shown in FIG. 8, all CAR-T cell samples effectively killed BAFF-R-positive target cells, with cytotoxicity comparable to that of the control CAR Vay736. Of them, clone 14 exhibited a certain degree of killing against the negative cells MOLT4. Clones 1, 5, 77, and 80 showed no non-specific killing against negative cells.

Based on the CD107a degranulation assay and in vitro cell killing assay, Clones 1, 5, 77, and 80 exhibited in vitro functions similar to the control CAR Vay736. Next, further evaluation of the CAR specificity and in vivo efficacy of the three clones will be performed.

### Example 9. Determination of performance of organ-derived tumor cells to stimulate CAR cell degranulation

CD107a is a marker for intracellular microvesicles. CD107a on the cell membrane increases after granzyme-loaded microvesicles fuse with the cell membrane, and when its recovery is blocked by monesin (purchased from BioLegend), it can quantitatively reflect the strength of microvesicle release. Upon stimulation of CAR-T cells by the target antigen on target cells, granzyme release occurs, and the activation of T cells by target cells can be determined by detecting an increase in CD107a via flow cytometry.

### Brief experimental steps of CD107a degranulation:

1) Centrifuge the CAR-T cells to be detected and target cells respectively at a room temperature and 300 g for 5 min, discard the supernatant, and then, resuspend the cells with 1640 medium + 10%FBS to 2x10⁶ cells/mL;
2) Add 100 µL of the CAR-T cells to be detected and 100 µL of the target cells to 96-well plates respectively, and mix well;
3) Add 1.5 µL of PE/Cy7 anti-human CD107a antibody and 0.2 µL of monensin to the cells in each well, then incubate in a cell culture incubator (37°C, 5%CO₂, 4 h);
4) After completion of incubation, centrifuge at 300 g for 5 min at 4°C, discard the supernatant, and wash the cells twice with 200 µL of PBS;
5) Resuspend the cells with 100 µL of PBS, add 1.5 µL of BV421 anti-human CD8 and 1.5 µL of APC anti-human EGFR antibodies respectively, mix well, and then, incubate on ice in the dark for 20 min; and
6) After completion of incubation, wash the cells with 200 µL of PBS 3 times, resuspend the cells with 100 µL of PBS, and then, detect by a flow cytometer.

### Main samples and reagents:

Target cells NALM6, 293T, A375, HepG2 , NCHI460, OVCAR-3 , Raji, HCT116 , Panc-1, MDA-MB-468, KATOIII;
Fetal bovine serum (FBS), Gibco, Cat. No.10099141;
Monensin, BioLegend, Cat. No. 420701;
PE/Cy7 mouse anti-human CD107a, BD, Cat. No. 561348;
BV421 mouse anti-human CD8, BD, Cat. No. 301036;
APC anti-human EGFR Antibody, Clone AY13, BioLegend, Cat. No.35290.

### Experimental results:

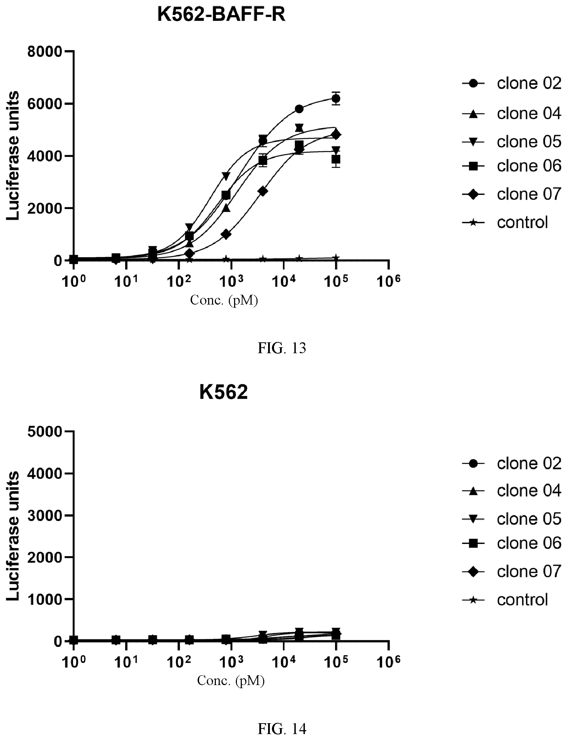

CAR-T cells were obtained by lentiviral transduction, and the CAR-T cells were cultured in vitro for 9-12 days before a CD107a degranulation assay. The CAR-T cells to be detected were incubated with target cells, monensin and CD107a antibodies for 4 h, and the cell density of the CAR-T cells and target cells was both 2×10⁶ cells/mL. The samples were then labeled with CD8 antibodies and EGFR antibodies and then subjected to flow cytometric detection. The data were analyzed using FlowJo software. Live cells were gated (P1) in the dot plot to exclude cell debris. Single, dispersed cells were gated (P2) from the P1 population. CD8-positive cells were further gated (P3) from the P2 population. Finally, within the P3 gate, the percentage of CD107a-positive cells among EGFR antibody-stained positive cells (i.e., CAR-positive cells) was analyzed. The analysis results are shown in FIG. 13. The degranulation levels of BAFF-R CAR-T cells from clones 1, 5, 77, and 80 were significantly increased only after co-incubation with BAFF-R-positive cells, whereas no obvious elevation in degranulation was observed following co-incubation with cell lines derived from organ-origin tumor cells. These results indicate that BAFF-R CAR-T cells are not easily activated by normal tissue or organ cells, thus exhibiting high safety.

The above in vitro experiments demonstrate that CAR-T cells of candidate clones 1, 5, 77, and 80 can effectively recognize BAFF-R antigen on tumor cells and exert potent cytotoxicity. No non-specific activation or killing activity was observed in the in vitro validation using negative cells, suggesting favorable expected clinical safety. Repetitive stimulation assays showed that the 4 candidate clones can be efficiently amplified under in vitro target antigen stimulation, and have the potential to maintain long-term effective killing of tumor cells.

### Example 10 Anti-tumor efficacy assay on NCG tumor-bearing mice

To further verify the anti-tumor efficacy of the 4 candidate clones in mice, we used the tumor cell line JEKO1-Luc stably expressing firefly luciferase (ffLuc), which was confirmed to express ffLuc normally. JEKO1 is a natural cell line with high endogenous BAFF-R expression. JEKO1-luc cells were inoculated into immunodeficient NCG mice via the tail vein. After tumor establishment, candidate clone CAR-T cells were inoculated to verify their anti-tumor effects.

### Brief test processes:

a) Establish the cell line JEKO1-luc overexpressing firefly luciferase (ffLuc), and identify that it can normally express the firefly luciferase;
b) Culture the above cells to the logarithmic growth phase and harvest the cells;
c) Purchase 48 NCG mice, acclimate for 1 week, inject JEKO1-luc cells via the tail vein at 5×10⁶ cells/200 µL PBS per mouse, and monitor the general condition daily after injection;
d) On day 10, divide the mice into groups, and inject via the tail vein with: CAR-T cells (3E+06 CAR+ cells/mouse), control non-transfected Mock-T cells (2E+07 cells/mouse), or PBS buffer.
e) Regularly observe images, weigh body weights, observe and record the general conditions of the mice, and regularly determine the proliferation of T cells in mouse blood.

### Experimental results:

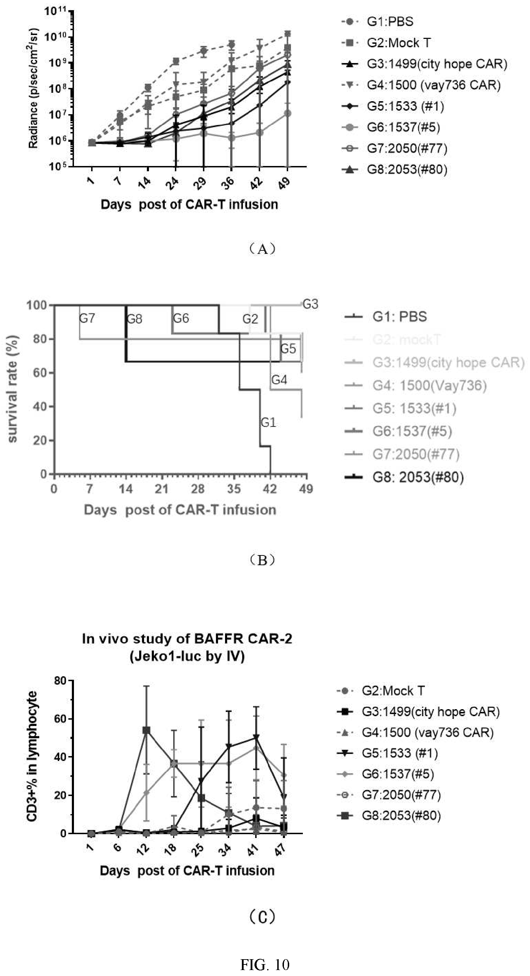

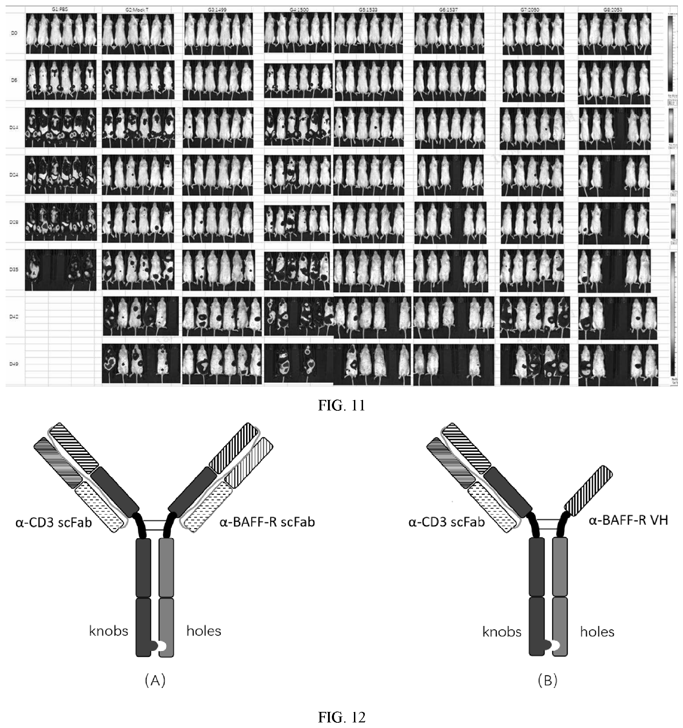

FIGS. 10 and 11 show the in vivo anti-tumor efficacy of the candidate clones. As shown in FIG. 10A and FIG. 11, tumor burden continued to increase after infusion of PBS, mockT, 1500 (Vay CAR), 1499 (city of hope CAR), clone 77, and clone 80 CAR-T cells. Tumor burden in mice infused with Clone 1 CAR-T cells was slightly lower than that in the PBS and mock-T groups, but the inhibitory effect on tumor growth was weak. For clone 5, tumor burden was significantly inhibited. As shown in FIG. 10B, the 35-day survival rate of mice in the clone 5 group was greater than 80%. FIG. 10C shows T-cell proliferation in mouse blood. No obvious in vivo T-cell expansion was observed in the BS, mockT, 1500 (Vay CAR), 1499 (city of hope CAR), and clone 77 groups. Clone 80 showed T-cell proliferation only in the first two weeks followed by a rapid decline, which may account for its relatively poor efficacy. Clones 1 and 5 maintained sustained and effective expansion in vivo, which was significantly superior to other clones. Moreover, the expansion of clone 5 started earlier than that of clone 1.

In summary, clone 5 exhibited the best in vivo anti-tumor efficacy and favorable safety, making it a superior candidate clone for subsequent clinical development. Clone 1 also showed certain anti-tumor effect and good safety, and can be used as an alternative candidate clone.

### Example 11 Screening of specific antibodies targeting BAFF-R protein

A fully human phage antibody library (including fully human single-chain antibody library and fully human single-domain antibody library) was used to enrich antibodies specifically binding to BAFF-R by affinity panning. Enzyme-linked immunosorbent assay (ELISA) and flow cytometry (FCM) were employed to screen antibody clones that specifically bind to BAFF-R antigen and BAFF-R-positive cells. The brief method is as follows: Biotinylated BAFF-R antigen (BAFFR-His-biotin, BAF-HM40RB, Kactus) was immobilized on SA-coupled magnetic beads (22307-10, Beaver Bio). Streptavidin-coupled magnetic beads were used as negative antigen to remove non-specifically bound phage clones from the phage library. Magnetic beads coated with BAFF-R antigen were used as positive antigen to enrich bound phage clones. This enrichment process was repeated for 3-4 rounds. As shown in Table 4, after 4 rounds of enrichment, the recovery rates of all four fully human phage antibody libraries reached 10⁻³, indicating that monoclonal screening and identification could be performed.

**Table 4. Recovery rate and enrichment fold of the phage antibody library after each round of panning**

| **Antibody library** | **Rounds** | **Recovery** rate | **Enrichment** fold |
|---|---|---|---|
| XL-SD-1 | 1st | 1.20E-05 | / |
| | 2nd | 2.00E-04 | 16.67 |
| | 3rd | 5.00E-04 | 2.50 |
| | 4th | 2.28E-03 | 4.56 |
| XL-NVH | 1st | 5.80E-05 | / |
| | 2nd | 1.30E-04 | 2.24 |
| | 3rd | 1.20E-04 | 0.92 |
| | 4th | 1.72E-03 | 14.33 |
| XL-V1 | 1st | 3.60E-05 | / |
| | 2nd | 5.36E-05 | 1.49 |
| | 3rd | 1.34E-04 | 2.50 |
| | 4th | 2.00E-03 | 14.93 |
| XL-V2 | 1st | 2.20E-05 | / |
| | 2nd | 5.36E-05 | 2.44 |
| | 3rd | 6.00E-05 | 1.12 |
| | 4th | 1.48E-03 | 24.67 |

Selected phage monoclones were subjected to ELISA using BAFFR-His-biotin as the positive antigen and streptavidin (21125, Pierce) as the negative antigen, to identify phage clones that specifically bound to only BAFFR-His-biotin but not streptavidin. Meanwhile, flow cytometry (FCM) was performed using the BAFF-R-overexpressing cell line K562-BAFF-R as the positive cell and K562 as the negative cell, to identify phage clones that specifically bound to K562-BAFF-R but not K562. Based on the results of ELISA and FCM, phage clones showing dual-specific binding were identified as candidate positive clones. The amino acid sequences of the candidate positive clones were determined after DNA sequencing.

### Example 12 Analysis of cytotoxic activity of candidate clones

To identify clones with superior T-cell dependent cellular cytotoxicity (TDCC) activity, candidate clones were constructed into bispecific antibodies of 1+1 configuration with the CD3 antibody OKT3, i.e., both the BAFF-R antibody and CD3 antibody are monovalent in one bispecific antibody molecule. The molecular configuration is shown in FIG. 12: the OKT3 molecule was designed as scFab, and the BAFF-R candidate clones were designed as VH or scFab (single-domain antibodies were constructed as VH, and single-chain antibodies as scFab). To facilitate antibody purification, the antibody Fc was retained. To eliminate ADCC, ADCP, and CDC effects, an IgG4 subtype with S228P/F234A/L235A mutation was used for Fc. To reduce the risk of Fc mispairing, a knobs-into-holes structure was introduced, with the "knobs" design on the CD3 antibody side and the "holes" design on the BAFF-R antibody side. After codon optimization, the antibody amino acid sequences were constructed into the pcDNA3.4 plasmid, namely OKT3 scFab - Fc PAA Ks, α-BAFF-R VH - Fc PAA Hs, and α-BAFF-R scFab - Fc PAA Hs. The expression plasmids for expressing OKT3 scFab antibody and BAFF-R VH or scFab antibody were co-transfected into CHOS cells, and the expression supernatant was collected after 7 days of suspension culture. The supernatant was centrifuged at 10,000 g for 30 min to remove debris, filtered through a 0.22µm filter membrane, and then purified by Protein A affinity chromatography. The purified antibody was concentrated and exchanged into PBS for TDCC activity analysis.

Activity analysis of the α-BAFF-R×α-CD3 bispecific antibody was performed in two steps:
First, the activation activity of the α-BAFF-R×α-CD3 bispecific antibody on the CD3 signaling pathway was evaluated using a TDCC reporter gene cell line (Jurkat-NFAT-luc2, Nanjing IASO Biotechnology Co., Ltd);

Then, the cytotoxic activity of the α-BAFF-R×α-CD3 bispecific antibody against BAFF-R-positive target cells was evaluated using T cells as effector cells. Based on the activation activity and cytotoxic activity, the α-BAFF-R×α-CD3 bispecific antibodies were ranked to identify α-BAFF-R clones with superior activity. The brief experimental method for the CD3 signaling pathway activation assay based on the TDCC reporter gene cell line is as follows: K562 stably overexpressing BAFF-R (K562-BAFF-R) was used as the positive target cell, K562 as the negative control cell, and Jurkat-NFAT-luc2 as the effector cell. 2E4 positive target cells or control cells were added to each well of a 96-well plate, together with an equal number of effector cells. The bispecific antibody was serially diluted 5-fold and added to the 96-well plate, with final concentrations of 100 nM, 20 nM, 4 nM, 0.8 nM, 0.16 nM, 0.032 nM, 0.0064 nM, and 0.00 nM. After incubation at 37°C for 14-16 hours, luciferase substrate (ONE-Glo^{™} EX Luciferase Assay System, E8120, Promega) was added, luminescence intensity was measured, and the EC50 curve of luminescence intensity versus antibody concentration was calculated.

As shown in FIG. 13, the bispecific antibodies formed by clone 02, clone 04, clone 05, clone 06, and clone 07 all mediated the activation of the CD3 signaling pathway by K562-BAFF-R target cells, among which clone 02, clone 05, and clone 06 exhibited stronger activation activity. As shown in FIG. 14, when BAFF-R was not expressed on the surface of target cells, none of the 5 bispecific antibodies induced activation of the CD3 signaling pathway.

The brief experimental method for the T-cell-based target cell killing assay is as follows: Nalm6 stably overexpressing luciferase (Nalm6-luc) was used as the positive target cell, and K562 stably overexpressing luciferase (K562-luc) was used as the negative control cell. T cells were used as effector cells. T cells were positively isolated from PBMCs using CD3 magnetic beads, activated with CD3/CD28 activation beads, and cultured in T-cell medium for 9-11 days. 2E4 positive target cells or control cells were added to each well of a 96-well plate, together with 4-fold effector cells to each well to achieve an effector-to-target ratio of 4:1. The bispecific antibodies were serially diluted 5-fold and added to the 96-well plate at final concentrations of 10 nM, 2 nM, 0.4 nM, 0.08 nM, 0.016 nM, 0.0032 nM, 0.00064 nM, and 0.00 nM. After incubation at 37°C for 14-16 h, luciferase substrate (ONE-Glo^{™} EX Luciferase Assay System, E8120, Promega) was added, luminescence intensity was measured, target cell killing efficiency was calculated, and the EC50 curve of killing efficiency versus concentration was plotted.

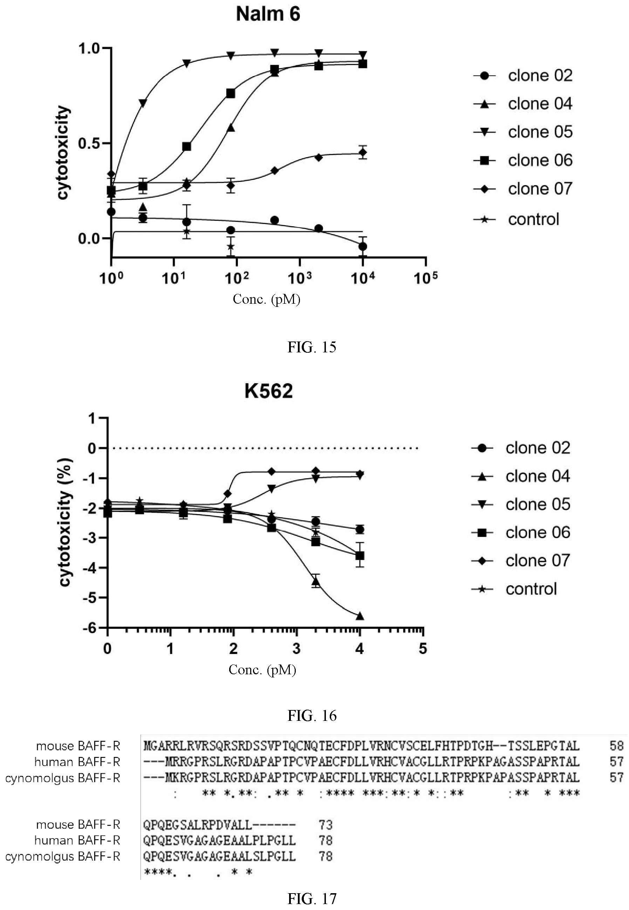

As shown in FIG. 15, among the bispecific antibodies formed by the combination of clone 02, clone 04, clone 05, clone 06, and clone 07, clone 04, clone 05, and clone 06 mediated the killing of target Nalm6 cells by PBMCs. Clone 05 showed the strongest activity, clone 07 showed weak activity, and no activity was observed for clone 02. As shown in FIG. 16, when BAFF-R was not expressed on the target cell surface, none of the 5 bispecific antibodies induced target cell killing.

### Example 13 Study on species cross-reactivity of antibodies

According to the BAFF-R protein information provided by Uniprot, alignment analysis showed that the amino acid similarity of the human BAFF-R extracellular domain was 96.16% with cynomolgus monkey and 48.57% with mouse, respectively. The similarity between cynomolgus monkey and mouse BAFF-R extracellular domain was 47.14% (Table 5, FIG. 17).

**Table 5. Amino acid similarity analysis of the BAFF-R extracellular domain among human, cynomolgus and mouse**

| | Human BAFF-R | Cynomolgus BAFF-R | Mouse BAFF-R |
|---|---|---|---|
| Human BAFF-R | 100% | / | / |
| Cynomolgus BAFF-R | 96.15% | 100% | / |
| Mouse BAFF-R | 48.57% | 47.14% | 100% |

To determine the cross-reactivity of the BAFF-R antibody between cynomolgus and mouse, the binding activity of the antibody to cynomolgus and mouse BAFF-R antigens was detected by enzyme-linked immunosorbent assay (ELISA). Clone 5 was constructed into a monoclonal antibody with Fc comprising S228P/L235E. The antibody was expressed and produced in CHOS cells and used for the species cross-reactivity study. BAFF-R antigens of different species (BAFFR-human-bio-his, BAF-HM40RB, KACTUS; BAFFR-cyno-His, BAF-CM10R, KACTUS; BAFFR-mouse-rFc, 50353-M31H, Sino) were diluted to 2 µg/mL with PBS, and coated onto 96-well ELISA plates at 100 µL/well, then incubated at 4°C overnight. The plates were blocked with 3% BSA at 37°C for 2 h. The antibody was serially diluted 5-fold to 500 nM, 100 nM, 20 nM, 4 nM, 0.8 nM, 0.16 nM, 0.032 nM, 0.0064 nM, and 0.00 nM, added at 100 µL/well, and incubated at 37°C for 2 h. HRP-labeled anti-human IgG antibody (HRP Donkey anti-human IgG, 410902, Biolegend) was diluted 1:2000, added at 100 µL/well, and incubated at 37°C for 1 h. After washing three times with 0.01% PBST, 100 µL/well of TMB substrate was added and the system was allowed to react in the dark for 3 min. The reaction was stopped with 50 µL/well of 2 M H₂SO₄, and A450 was measured using a microplate reader.

The maximum binding value and binding EC₅₀ value were calculated based on A450 changes along the concentration gradient. As shown in Table 6, the EC₅₀ values of clone 5 binding to human, cynomolgus, and mouse BAFF-R were 0.965 nM, 1.906 nM, and 3.402 nM, respectively. These results indicate that clone 5 exhibits cross-reactivity with both cynomolgus and mouse BAFF-R, with strong cross-reactivity to cynomolgus and relatively weaker cross-reactivity to mouse.

**Table 6. Cross-binding data of clone 5 antibody with human, cynomolgus and mouse BAFF-R**

| **Antigen** | **EC₅₀ (nM)** | **Top Value** |
|---|---|---|
| Human BAFF-R | 0.965 | 1.473 |
| Cyno BAFF-R | 1.906 | 1.228 |
| Mouse BAFF-R | 3.402 | 1.487 |

### Example 14 Structural design and preparation of bispecific antibodies of preferred clones

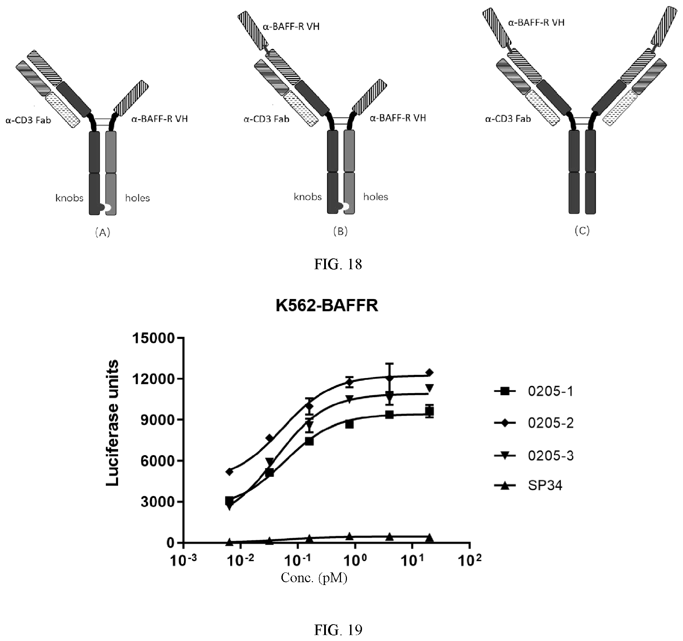

The preferred BAFF-R single-domain antibody clone was combined with the CD3 antibody SP34 to construct bispecific antibodies, and 3 configurations are shown in FIG. 18. Configuration 1 is a 1+1 structure: both the BAFF-R antibody and CD3 antibody are monovalent in one bispecific antibody molecule (FIG. 18A). Configuration 2 is a 2+1 structure: the BAFF-R antibody is bivalent while the CD3 antibody is monovalent (FIG. 18B). Configuration 3 is a 2+2 structure: both the BAFF-R antibody and CD3 antibody are bivalent in one bispecific antibody molecule (FIG. 18C). In each of the 3 configurations, the BAFF-R antibody is a single-domain antibody, and the CD3 antibody SP34 is a Fab structure.

Similarly, to prolong the antibody half-life and facilitate antibody purification, the Fc moiety of the antibody was retained. Meanwhile, to eliminate ADCC, ADCP and CDC effects, the Fc was of IgG4 isotype with S228P/F234A/L235A mutation. In an asymmetric structure, to reduce Fc mispairing, a knobs-into-holes structure was introduced, where the CD3 antibody side was designed as knobs and the BAFF-R antibody side as holes. A pair of disulfide bonds was also introduced into the Fc structure. The knobs side had S354C/T366W combined mutation, and the holes side had Y349C/T366S/L368A/Y407V combined mutation. After codon optimization, the antibody amino acid sequences were constructed into the pcDNA3.4 plasmid. The plasmids were designated as SP34-H PAA Ks, 05VH-SP34-H PAA Ks, 05VH-SP34-H PAA, SP34-L and 05VH-hFc PAA Hs, respectively. The plasmid combination expressing the 1+1 bispecific antibody is SP34-H PAA Ks, SP34-L and 05VH-hFc PAA Hs; the plasmid combination expressing the 2+1 bispecific antibody is 05VH-SP34-H PAA Ks, SP34-L and 05VH-hFc PAA Hs; the plasmid combination expressing the 2+2 bispecific antibody is 05VH-SP34-H PAA and SP34-L; the bispecific antibodies with 1+1, 2+1 and 2+2 configurations are respectively named VH-Fab-Fc 1+1, VH2-Fab-Fc 2+1 and VH2-Fab2-Fc 2+2; the antibody codes are respectively 0205-1, 0205-2 and 0205-3 (Table 7).

According to the plasmid combinations in Table 7, the plasmids were co-transfected into CHOS cells. The expression supernatant was harvested after 7 days of suspension culture. The supernatant was centrifuged at 10,000 g for 30 min to remove debris, then filtered through a 0.22 µm filter membrane. The antibody was subjected to Protein A affinity chromatography. The purified antibody was concentrated and buffer-exchanged into MES buffer for cation-exchange chromatography. The Protein A-purified antibody was highly purified by cation-exchange chromatography to remove mispaired antibodies. The highly purified antibody was used for binding kinetics, in vitro and in vivo biological activity assays, etc.

**Table 7. Plasmid combinations for bispecific antibody expression**

| **Antibody code** | **Antibody name** | **Plasmid 1** | **Plasmid 2** | **Plasmid 3** |
|---|---|---|---|---|
| 0205-1 | VH-Fab-Fc 1+1 | SP34-H PAA Ks (SEQ ID NO. 73) | SP34-L (SEQ ID NO. 71) | 05VH-hFc PAA Hs (SEQ ID NO. 51) |
| 0205-2 | VH2-Fab-Fc 2+1 | 05VH-SP34-H PAA Ks (SEQ ID NO. 75) | SP34-L (SEQ ID NO. 71) | 05VH-hFc PAA Hs (SEQ ID NO. 51) |
| 0205-3 | VH2-Fab2-Fc 2+2 | 05VH-SP34-H PAA (SEQ ID NO. 77) | SP34-L (SEQ ID NO. 71) | N/A |

### Example 15 Binding kinetics analysis of antibodies to antigens

To investigate the binding kinetics of α-BAFF-R×α-CD3 bispecific antibodies to human BAFF-R and human CD3 proteins, bio-layer interferometry (BLI) was employed. The association-dissociation constants and affinities of the bispecific antibodies to BAFF-R and CD3 proteins were analyzed using the ForteBio Octet molecular interaction analysis system.

His-tagged human BAFF-R antigen (BAR-H52H3, Acro) was diluted to 5 µg/mL in binding buffer (1×PBS containing 0.02% Tween and 0.1% BSA, PH=7.4) and loaded onto HIS1K biosensors (18-0038, ForteBio) to a binding thickness of 0.25 nm. The antigen-loaded sensors were equilibrated in sample dilution buffer (1×PBS containing 0.02% Tween and 0.1% BSA, PH=7.4) for 180 sec, then incubated in serially diluted antibody solutions (200 nM, 100 nM, 50 nM, 25 nM, 12.5 nM, 6.25 nM) for association for 60 sec, followed by dissociation in sample dilution buffer for 60-180 sec. Finally, the sensors were regenerated sequentially with 10 mM glycine and binding buffer (1×PBS containing 0.02% Tween and 0.1% BSA, PH=7.4). Data were analyzed using the built-in data analysis software of the Octet molecular interaction analysis system to obtain association, dissociation constants and affinity values. As shown in Table 8, the affinity of clone 05 monoclonal antibody to human BAFF-R was 2.63 nM, and the affinities of bispecific antibodies 0205-1, 0205-2 and 0205-3 to human BAFF-R were 1.78 nM, 1.12 nM and 1.27 nM, respectively.

**Table 8. Kinetic parameters of α-BAFF-R monoclonal antibody and α-BAFF-R×α-CD3 bispecific antibodies binding to BAFF-R**

| **Antibody** | **Antigen** | **KD (M)** | **Kon (1/Ms)** | **Kdis (1/s)** | **Rmax** | **R^2** |
|---|---|---|---|---|---|---|
| Clone 05 | | 2.63E-09 | 6.22E+05 | 1.63E-03 | 1.1603 | 0.9969 |
| 0205-1 | Human | 1.78E-09 | 1.38E+06 | 2.45E-03 | 1.3379 | 0.9949 |
| 0205-2 | BAFF-R | 1.12E-09 | 8.75E+05 | 9.76E-04 | 1.3606 | 0.9984 |
| 0205-3 | | 1.27E-09 | 1.07E+06 | 1.36E-03 | 1.6546 | 0.9971 |

His-tagged human CD3E&D (CDD-H52W1, Acro) and human CD3E&G (CDG-H52W6, Acro) was diluted to 5 µg/mL in binding buffer (1×PBS containing 0.02% Tween and 0.1% BSA, PH=7.4) and loaded onto HIS1K biosensors (18-0038, ForteBio) to a binding thickness of 0.4 nm. The antigen-loaded sensors were equilibrated in sample dilution buffer (1×PBS containing 0.02% Tween and 0.1% BSA, PH=7.4) for 180 sec, then incubated in serially diluted antibody solutions (100 nM, 50 nM, 25 nM, 12.5 nM, 6.25 nM, 3.125 nM) for association for 60 sec, followed by dissociation in sample dilution buffer for 60-180 sec. Finally, the sensors were regenerated sequentially with 10 mM glycine and binding buffer (1×PBS containing 0.02% Tween and 0.1% BSA, PH=7.4). Data were analyzed using the built-in data analysis software of the Octet molecular interaction analysis system to obtain association, dissociation constants and affinity values. As shown in Table 9, the affinities of monoclonal antibody SP34 to human CD3E&D and human CD3E&G were 6.38 nM and 7.87 nM, respectively; the affinities of bispecific antibody 0205-1 to CD3E&D and human CD3E&G were 10.7 nM and 6.68 nM, respectively; the affinities of bispecific antibody 0205-2 to CD3E&D and human CD3E&G were 10.3 nM and 11.1 nM, respectively; and the affinities of bispecific antibody 0205-3 to CD3E&D and human CD3E&G were 10.6 nM and 9.03 nM, respectively. Although the single-domain antibody was linked to the N-terminus of the anti-CD3 antibody heavy chain in 0205-2 and 0205-3, their binding to the CD3E&D or CD3E&G heterodimer complex was not affected or only minimally affected by the N-terminal single-domain antibody.

**Table 9. Kinetic parameters of α-CD3 monoclonal antibody and α-BAFF-R×α-CD3 bispecific antibodies binding to CD3E&D and CD3E&G**

| **Antibody** | **Antigen** | **KD (M)** | **Kon (1/Ms)** | **Kdis (1/s)** | **Rmax** | **R^2** |
|---|---|---|---|---|---|---|
| SP34 | CD3E&D | 6.38E-09 | 4.73E+05 | 3.01E-03 | 0.3714 | 0.9976 |
| | CD3E&G | 7.87E-09 | 3.69E+05 | 2.90E-03 | 0.4351 | 0.998 |
| 0205-1 | CD3E&D | 1.07E-08 | 7.59E+05 | 8.11E-03 | 0.419 | 0.9967 |
| | CD3E&G | 6.68E-09 | 4.16E+06 | 2.78E-02 | 0.2178 | 0.9916 |
| 0205-2 | CD3E&D | 1.03E-08 | 3.06E+05 | 3.14E-03 | 0.2003 | 0.9948 |
| | CD3E&G | 1.11E-08 | 2.62E+05 | 2.91E-03 | 0.2594 | 0.9965 |
| 0205-3 | CD3E&D | 1.06E-08 | 3.02E+05 | 3.20E-03 | 0.3429 | 0.9946 |
| | CD3E&G | 9.03E-09 | 2.77E+05 | 2.51E-03 | 0.443 | 0.9967 |

### Example 16 Study on CD3 signaling pathway activation activity of bispecific antibodies

The primary step for α-BAFF-R×α-CD3 bispecific antibodies to exert biological function is T-cell activation mediated by the bispecific antibodies. T-cell activation occurs only in the presence of BAFF-R-positive target cells, while no T-cell activation is observed in the absence of BAFF-R-positive cells. The activation activity and specificity of α-BAFF-R×α-CD3 bispecific antibodies on the CD3 signaling pathway were evaluated based on a TDCC reporter gene cell line (Jurkat-NFAT-luc2). Human chronic myelogenous leukemia cell line K562 stably overexpressing BAFF-R (K562-BAFF-R), human acute lymphoblastic leukemia cell line Nalm6, human chronic lymphocytic leukemia cell line MEC-1, and human mantle cell lymphoma cell line Jeko-1 were used as positive target cells. K562 was used as the negative control cell, and Jurkat-NFAT-luc2 as the effector cell. 2E4 positive target cells or negative control cells were added to each well of a 96-well plate, together with an equal number of effector cells. The bispecific antibodies were serially diluted 5-fold and added to the 96-well plate at final concentrations of 100 nM, 20 nM, 4 nM, 0.8 nM, 0.16 nM, 0.032 nM, 0.0064 nM, and 0.00 nM. After incubation at 37°C for 14-16 h, luciferase substrate (ONE-Glo^{™} EX Luciferase Assay System, E8120, Promega) was added, luminescence intensity was measured, and the EC50 curve of luminescence intensity versus antibody concentration was calculated.

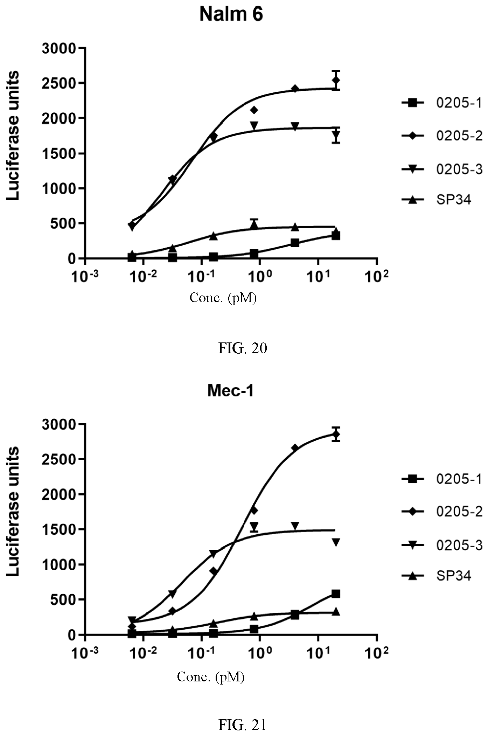

As shown in FIG. 19-20 and Tables 10-13, when K562-BAFF-R with high BAFF-R expression was used as the target cell, 0205-1, 0205-2, and 0205-3 all mediated activation of the TDCC reporter gene cells, with similar EC₅₀ values. When the target cells were native tumor cell lines expressing BAFF-R such as Nalm6, Mec-1, and Jeko-1, the 3 bispecific antibodies exhibited significantly different activities in mediating TDCC reporter gene cell activation: 0205-3 showed the optimal activity with EC₅₀ ranging from 0.02 nM to 0.065 nM; 0205-2 was moderate with EC₅₀ ranging from 0.06 nM to 0.5 nM; 0205-1 showed the weakest activity.

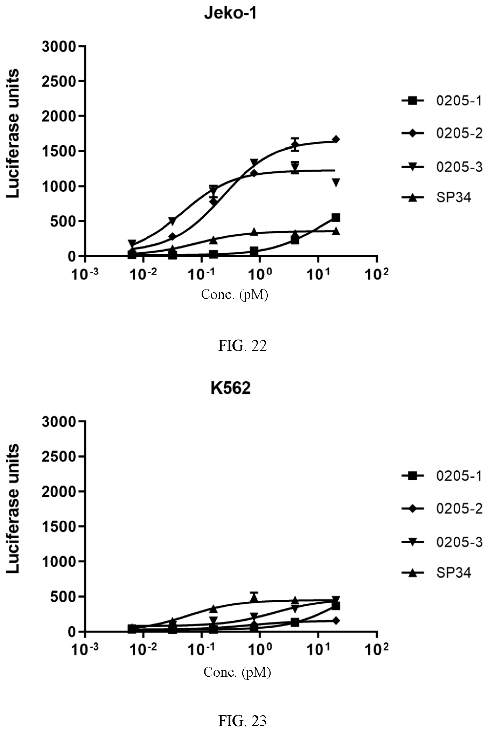

As shown in FIG. 23 and Table 14, all 3 bispecific antibodies mediated low levels of TDCC reporter gene activation in BAFF-R-negative cells. Slight activation of TDCC reporter gene cells was observed only at the highest concentrations of 0205-1 and 0205-3. Since 0205-3 is a bivalent CD3 antibody, these results suggest that 0205-3 may carry certain safety risks, which require further evaluation in subsequent experiments.

**Table 10. Parameters of TDCC reporter gene cell activation by K562-BAFF-R mediated by α-BAFF-R VH×α-CD3 Fab bispecific antibodies**

| | K562-BAFF-R | |
|---|---|---|
| | EC₅₀(nM) | top |
| 0205-1 | 0.059 | 9426 |
| 0205-2 | 0.054 | 12251 |
| 0205-3 | 0.041 | 10910 |
| SP34 | 0.059 | 450.7 |

**Table 11. Parameters of TDCC reporter gene cell activation by Nalm6 mediated by α-BAFF-R VH×α-CD3 Fab bispecific antibodies**

| | Nalm6 | |
|---|---|---|
| | EC50(nM) | top |
| 0205-1 | 3.2 | 381.1 |
| 0205-2 | 0.068 | 2431 |
| 0205-3 | 0.021 | 1870 |
| SP34 | 0.059 | 450.7 |

**Table 12. Parameters of TDCC reporter gene cell activation by Mec-1 mediated by α-BAFF-R VH×α-CD3 Fab bispecific antibodies**

| | Mec-1 | |
|---|---|---|
| | EC50(nM) | top |
| 0205-1 | 7.311 | 792.8 |
| 0205-2 | 0.5 | 2931 |
| 0205-3 | 0.047 | 1489 |
| SP34 | 0.16 | 317.3 |

**Table 13. Parameters of TDCC reporter gene cell activation by Jeko-1 mediated by α-BAFF-R VH×α-CD3 Fab bispecific antibodies**

| | Jeko-1 | |
|---|---|---|
| | EC50(nM) | top |
| 0205-1 | 11.67 | 863.5 |
| 0205-2 | 0.23 | 1654 |
| 0205-3 | 0.046 | 1227 |
| SP34 | 0.083 | 359.7 |

**Table 14. Parameters of TDCC reporter gene cell activation by K562 mediated by α-BAFF-R VH×α-CD3 Fab bispecific antibodies**

| | K562 | |
|---|---|---|
| | EC50(nM) | top |
| 0205-1 | 25.45 | 796.1 |
| 0205-2 | 0.65 | 155.7 |
| 0205-3 | 1.86 | 466.1 |
| SP34 | 0.059 | 450.7 |

### Example 17 Study on target cell killing activity of PBMCs mediated by bispecific antibodies

To simulate the activity of bispecific antibody-mediated immune cell killing of BAFF-R-positive target cells in vivo, PBMCs were used as effector cells, Jeko-1 stably overexpressing luciferase (Jeko-1-luc) as positive target cells, and K562 stably overexpressing luciferase (K562-luc) as negative control cells. 2E4 positive target cells or control cells were added to each well of a 96-well plate, together with 10-fold effector cells to each well to achieve an effector-to-target ratio of 10:1. The antibodies were serially diluted 5-fold and added to the 96-well plate at final concentrations of 1 nM, 0.4 nM, 0.04 nM, 0.008 nM, 0.0016 nM, 0.00032 nM, and 0.0 nM. After incubation at 37°C for 24 h and 48 h, luciferase substrate (ONE-Glo^{™} EX Luciferase Assay System, E8120, Promega) was added, respectively, luminescence intensity was measured, target cell killing efficiency was calculated, and the EC50 curve of killing efficiency versus concentration was plotted. In this assay, a REGN1979 analog, a bispecific antibody targeting CD20 and CD3 from Regeneron, was used as the positive control.

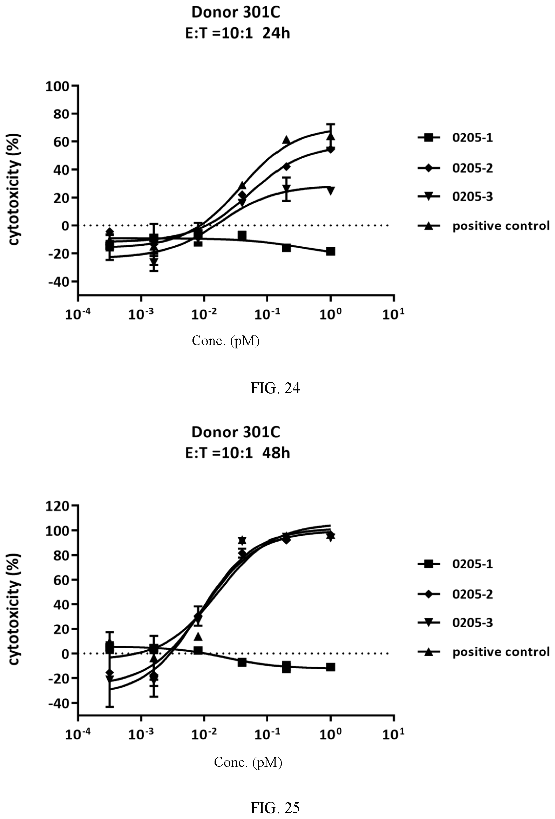

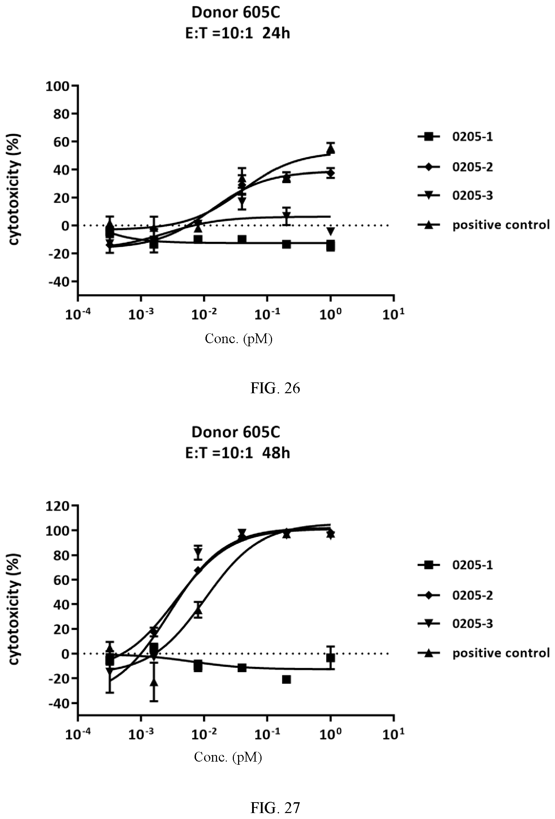

As shown in FIGS. 24-27 and Tables 15 and 16, the 3 bispecific antibodies exhibited a consistent activity rank in PBMCs from two donors. No killing activity against BAFF-R-positive tumor cells was observed for 0205-1 in each of the 2 donors. 0205-2 and 0205-3 showed relatively weak tumor cell killing at 24 h, but achieved complete tumor cell clearance at 48 h.

By comprehensive comparison of the activation activity on TDCC reporter gene cells and the clearance activity on BAFF-R-positive tumor cells, the potency rank of the α-BAFF-R VH×α-CD3 Fab bispecific antibodies was 0205-3 ≥ 0205-2 > 0205-1.

**Table 15: Killing parameters of PBMC cells (Donor 301C) against Jeko-1 cells mediated by α-BAFF-R VH×α-CD3 Fab bispecific antibody**

| | Donor 301C | | | |
|---|---|---|---|---|
| | 24hr | | 48hr | |
| | EC50(pM) | top (%) | EC50(pM) | top (%) |
| 0205-1 | / | -22.31 | / | -12.22 |
| 0205-2 | 52.83 | 57.79 | 9.744 | 99.86 |
| 0205-3 | 18.04 | 28.51 | 8.775 | 101.8 |
| positive control | 37.99 | 70.72 | 16.9 | 105.5 |

**Table 16: Killing parameters of PBMC cells (Donor 605C) against Jeko-1 cells mediated by α-BAFF-R VH×α-CD3 Fab bispecific antibody**

| | Donor 605C | | | |
|---|---|---|---|---|
| | 24hr | | 48hr | |
| | EC50(pM) | top (%) | EC50(pM) | top (%) |
| 0205-1 | / | -12.61 | / | -12.76 |
| 0205-2 | 13.1 | 39.01 | 3.585 | 101.2 |
| 0205-3 | 2.478 | 6.344 | 2.814 | 102.2 |
| positive control | 39.36 | 52.9 | 10.38 | 105.7 |

### Example 18 Study on cytokine release from PBMCs mediated by bispecific antibodies

PBMCs were used as effector cells, and Jeko-1 cells stably overexpressing luciferase (Jeko-1-luc) were used as positive target cells. Meanwhile, a vehicle control without target cells was designed. 2E4 positive target cells or control cells were added to each well of a 96-well plate, together with 10-fold amount of effector cells, resulting in an effector-to-target (E:T) ratio of 10:1. An equal number of effector cells was added to the vehicle control wells. The bispecific antibodies were serially diluted 5-fold and added to the 96-well plate, with final concentration gradients of 1 nM, 0.4 nM, 0.016 nM, and 0.0 nM. After incubation at 37°C for 24 h and 48 h, respectively, the plates were centrifuged at 500 g for 10 min, and the supernatants were carefully collected. The levels of cytokines IFN-γ (62HIFNGPEG, cisbio), IL-2 (62HIL02PEG, cisbio), TNFα (62HTNFAPEG, cisbio), and IL-6 (62HIL06PEG, cisbio) in the supernatants were determined by time-resolved fluorescence-based assay, to evaluate the magnitude of cytokine release from PBMCs mediated by the bispecific antibodies. In this study, an analog of REGN1979 (a bispecific antibody targeting CD20 and CD3 from Regeneron) was used as a positive control, and the SP34 monoclonal antibody was used as a negative control antibody.

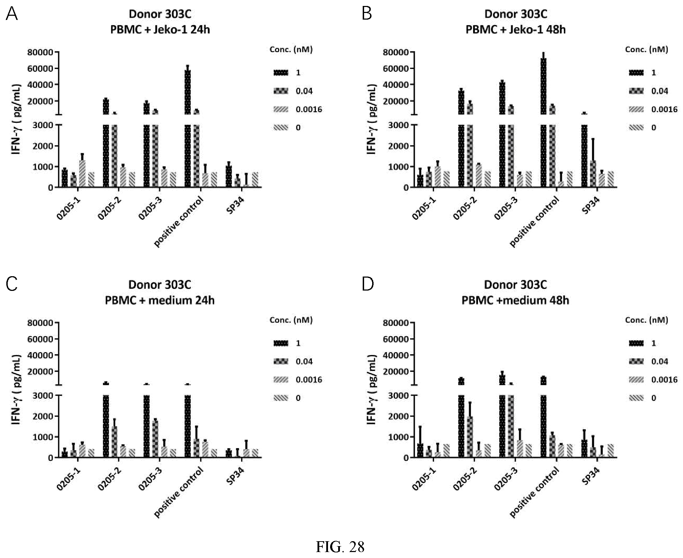

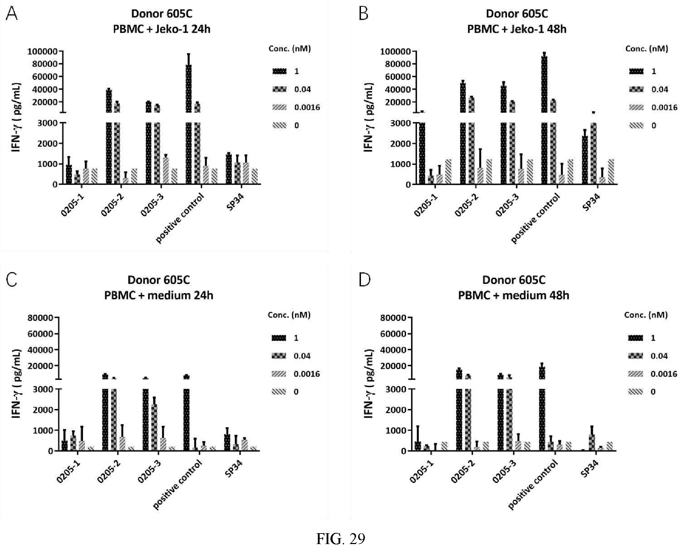

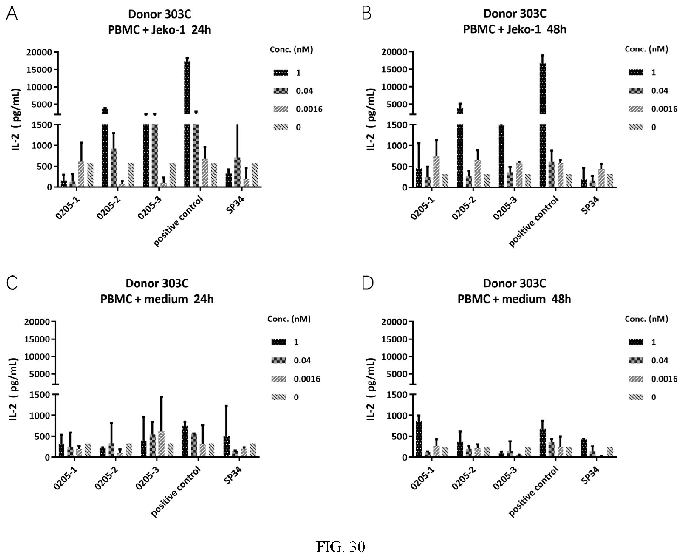

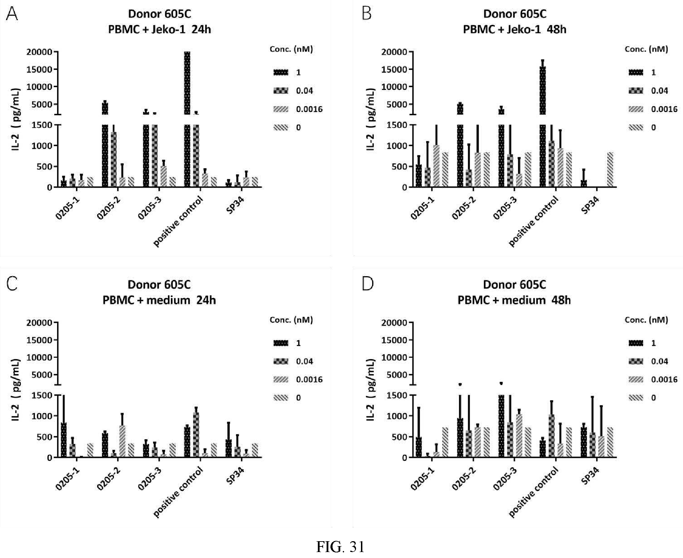

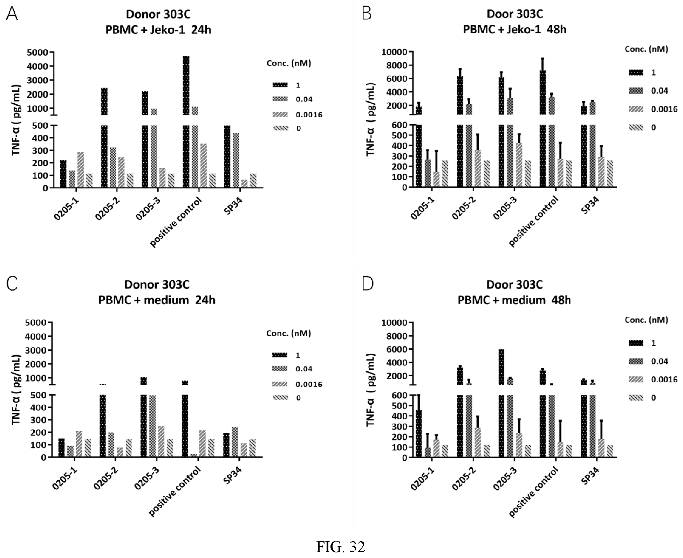

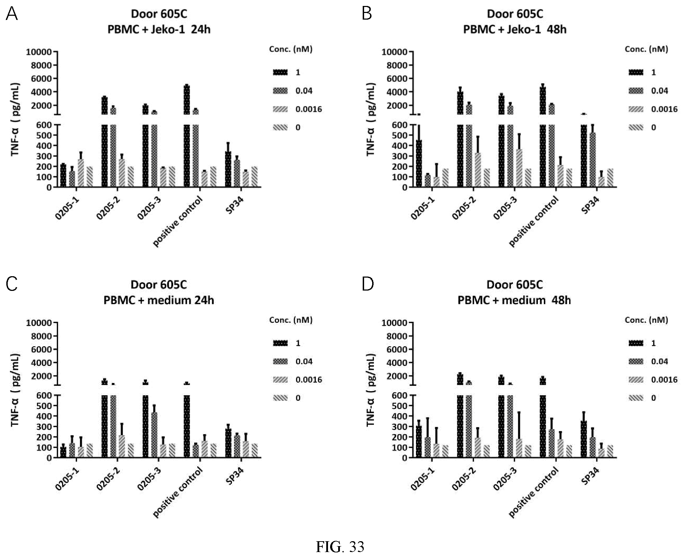

As shown in FIG. 28-33, when co-incubated with BAFF-R-positive target cells and PBMCs, bispecific antibodies 0205-2 and 0205-3 significantly induced the release of IFN-γ, IL-2, and TNFα from PBMCs, with levels slightly lower than those of the positive control antibody. In contrast, the secretion of IFN-γ, IL-2, and TNFα was markedly reduced in the absence of BAFF-R-positive target cells.

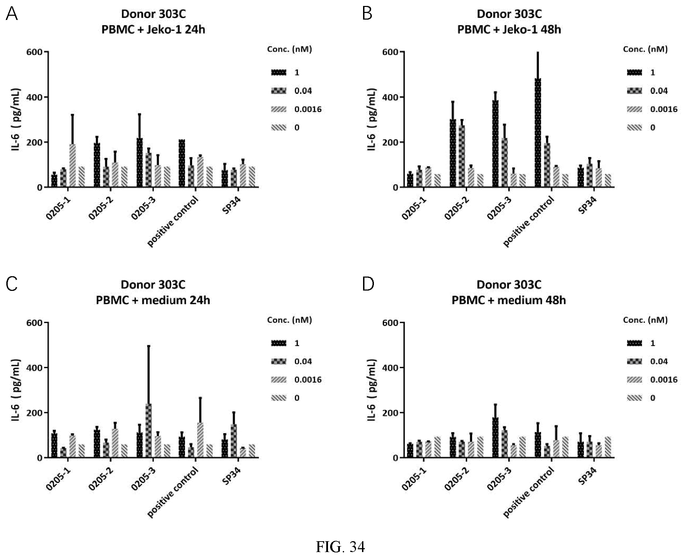

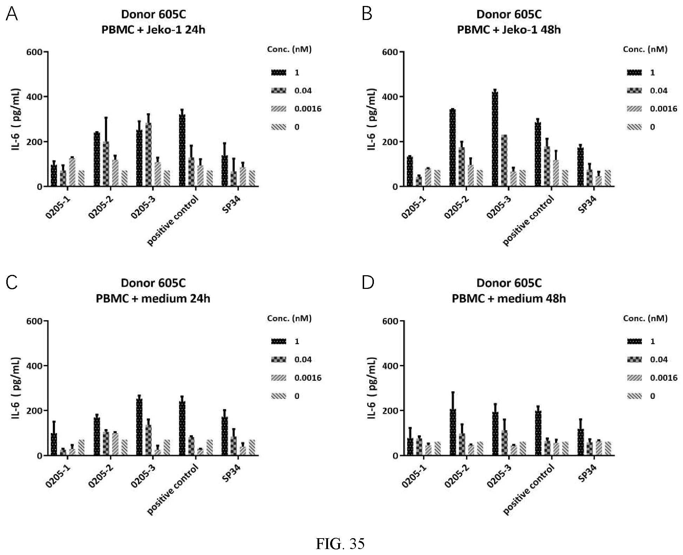

As shown in FIGS. 34 and 35, all 3 bispecific antibodies and the positive control antibody induced low levels of IL-6 secretion from PBMCs, regardless of the presence or absence of BAFF-R-positive target cells.

### Example 19 Pharmacokinetic study of antibodies

To investigate the pharmacokinetics of the antibodies, 6-8-week-old female SD rats weighing 220-240 g were used. The SD rats were purchased from Shanghai Jihui Laboratory Animal Care Co. LTD). The antibody dose was 1 mg/kg, administered intravenously. Blood samples (200 µL) were collected from the jugular vein before administration and at various time points after dosing (5 min, 30 min, 2 hr, 6 hr, 24 hr, 72 hr, 120 hr, 168 hr, 240 hr, 336 hr). After standing at room temperature for approximately 1 hour, the blood samples were centrifuged to harvest serum, which was stored at -70°C. Serum antibody concentrations were determined using the sandwich ELISA method. Goat anti-human IgG Fc antibody (609-101-017, Rockland) was diluted to 2.5 µg/mL and coated onto ELISA plates. Sera were diluted 20-fold for quantitative detection. Peroxidase-conjugated secondary antibody Anti-Human IgG (Fab specific)-peroxidase (A0293, Sigma) was diluted 1:5000 to bind to the Fab region of serum antibodies. Serum antibody concentrations at each time point were calculated based on the standard curve and dilution factor.

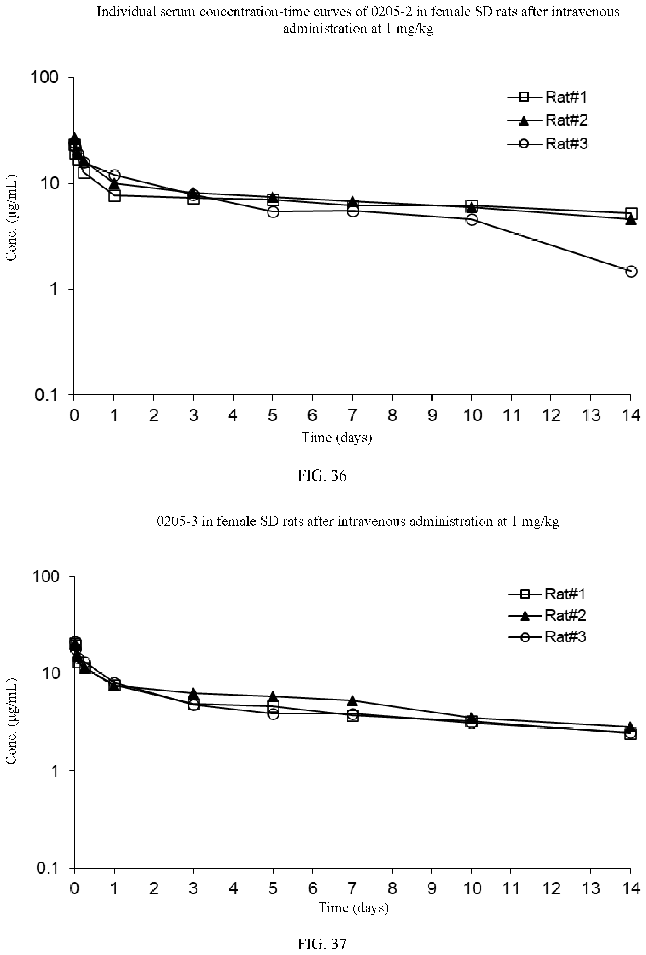

No abnormal reactions were observed in any SD rats after dosing. The serum concentrations of bispecific antibody 0205-2 at each time point are shown in Table 17 and FIG. 36, with a half-life of about13.7 days. The serum concentrations of bispecific antibody 0205-3 at each time point are shown in Table 18 and FIG. 37, with a half-life of about 10.3 days.

**Table 17. Serum concentration and pharmacokinetic parameters of bispecific antibody 0205-2 in SD rats**

| **Individual and mean serum concentration-time data of 0205-2 after an IV dose at 1 mg/kg in female SD rats** | | | | | | | | |
|---|---|---|---|---|---|---|---|---|
| Dose (mg/kg) | Dose route | Sampling time (Day) | Concentration (µg/mL) | | | Mean (µg/mL) | SD | CV(%) |
| | | | Rat# 1 | Rat#2 | Rat#3 | | | |
| 1 | IV | 0 | BQL | BQL | BQL | BQL | NA | NA |
| | | 0.003 | 23.3 | 27.2 | 24.4 | 25.0 | 1.99 | 7.98 |
| | | 0.021 | 19.7 | 26.1 | 22.0 | 22.6 | 3.27 | 14.5 |
| | | 0.083 | 17.2 | 19.3 | 19.0 | 18.5 | 1.13 | 6.08 |
| | 16.6 | 0.250 | 12.8 | 16.6 | 15.9 | 15.1 | 2.03 | 13.5 |
| | | 1 | 7.71 | 9.95 | 11.9 | 9.87 | 2.12 | 21.5 |
| | | 3 | 7.35 | 8.24 | 7.85 | 7.81 | 0.447 | 5.73 |
| | | 5 | 7.08 | 7.51 | 5.40 | 6.66 | 1.11 | 16.7 |
| | 12.4 | 7 | 6.15 | 6.74 | 5.50 | 6.13 | 0.619 | 10.1 |
| | 17.0 | 10 | 6.16 | 6.02 | 4.58 | 5.59 | 0.873 | 15.6 |
| | 5.33 | 14 | 5.20 | 4.62 | 1.49 | 3.77 | 1.99 | 52.9 |

| PK parameters | | Unit | Rat# 1 | Rat#2 | Rat#3 | Mean | SD | CV(%) |
|---|---|---|---|---|---|---|---|---|
| C₀ | | µg/mL | 24.1 | 27.4 | 25.0 | 25.5 | 1.71 | 6.70 |
| CL | | mL/day/kg | 3.72 | 5.18 | 10.4 | 6.44 | 3.53 | 54.8 |
| Vₛₛ | | mL/kg | 122 | 96.3 | 66.6 | 94.8 | 27.6 | 29.1 |
| T_{1/2} | | day | 23.1 | 13.4 | 4.42 | 13.7 | 9.36 | 68.5 |
| AUCₗₐₛₜ | | day*µg/mL | 95.7 | 104 | 86.4 | 95.2 | 8.58 | 9.02 |
| AUC_{INF} | | day*µg/mL | 269 | 193 | 95.9 | 186 | 86.8 | 46.7 |
| MRT_{INF} | | day | 32.7 | 18.6 | 6.38 | 19.2 | 13.2 | 68.6 |
| MRTₗₐₛₜ | | day | 6.23 | 5.78 | 4.84 | 5.62 | 0.708 | 12.6 |

**Table 18. Concentration of bispecific antibody 0205-3 in SD rat serum and its pharmacokinetic parameters**

| **Individual and mean serum concentration-time data of 0205-3 after an IV dose at 1 mg/kg in female SD rats** | | | | | | | | |
|---|---|---|---|---|---|---|---|---|
| Dose (mg/kg) | Dose route | Sampling time (Day) | Concentration (µg/mL) | | | Mean (µg/mL) | SD | CV(%) |
| | | | Rat# 1 | Rat#2 | Rat#3 | | | |
| 1 | IV | 0 | BQL | BQL | BQL | BQL | NA | NA |
| | | 0.003 | 20.6 | 20.5 | 21.5 | 20.9 | 0.588 | 2.82 |
| | | 0.021 | 20.0 | 20.3 | 17.9 | 19.4 | 1.31 | 6.74 |
| | | 0.083 | 13.3 | 15.1 | 14.5 | 14.3 | 0.914 | 6.39 |
| | 16.6 | 0.250 | 11.6 | 11.6 | 13.1 | 12.1 | 0.867 | 7.16 |
| | | 1 | 7.67 | 7.45 | 8.17 | 7.77 | 0.369 | 4.76 |
| | | 3 | 4.94 | 6.29 | 4.83 | 5.35 | 0.816 | 15.2 |
| | | 5 | 4.58 | 5.79 | 3.91 | 4.76 | 0.954 | 20.1 |
| | 12.4 | 7 | 3.70 | 5.26 | 3.86 | 4.27 | 0.861 | 20.2 |
| | 17.0 | 10 | 3.29 | 3.53 | 3.12 | 3.31 | 0.204 | 6.16 |
| | 5.33 | 14 | 2.45 | 2.85 | 2.47 | 2.59 | 0.229 | 8.85 |

| PK parameters | | Unit | Rat# 1 | Rat#2 | Rat#3 | Mean | SD | CV(%) |
|---|---|---|---|---|---|---|---|---|
| C₀ | | µg/mL | 20.7 | 20.5 | 22.4 | 21.2 | 1.02 | 4.79 |
| CL | | mL/day/kg | 9.88 | 8.98 | 9.85 | 9.57 | 0.507 | 5.30 |
| Vₛₛ | | mL/kg | 143 | 115 | 143 | 134 | 16.2 | 12.1 |
| T_{1/2} | | day | 10.8 | 9.12 | 10.9 | 10.3 | 0.986 | 9.61 |
| AUCₗₐₛₜ | | day*µg/mL | 63.1 | 73.7 | 62.9 | 66.6 | 6.20 | 9.32 |
| AUC_{INF} | | day*µg/mL | 101 | 111 | 102 | 105 | 5.72 | 5.47 |
| MRT_{INF} | | day | 14.4 | 12.8 | 14.5 | 13.9 | 0.982 | 7.05 |
| MRTₗₐₛₜ | | day | 5.32 | 5.46 | 5.20 | 5.33 | 0.133 | 2.50 |

### Example 20 In vivo efficacy study of antibodies

Considering the safety profile of CD3 antibodies, we selected the bispecific antibody 0205-2 containing a monovalent CD3 antibody to evaluate its in vivo antitumor efficacy in a subcutaneous tumor model based on PBMC-reconstituted NCG mice. As shown in Table 19, on Day 0, Jeko-1 tumor cells were mixed 1:1 with matrigel and inoculated subcutaneously at 5E6 cells per mouse. On Day 3, PBMC cells were infused intraperitoneally at 1E7 cells per mouse. Mice were randomized into groups when tumor volume reached 110 mm³: Group 1: PBS control group, Group 2: 0205-2 low-dose group (1 mpk), Group 3: 0205-2 high-dose group (10 mpk). Antibodies were administered intraperitoneally twice weekly for two consecutive weeks. Body weight and tumor volume were measured twice weekly. In accordance with animal research ethics guidelines, mice were euthanized when tumor volume reached the predefined endpoint.

**Table 19. Study design for bispecific antibody efficacy in a subcutaneous tumor model based on PBMC-reconstituted NCG mice**

| **Group** | **Mice No.** | **Jeko-1** | **PBMCs** | **Dosage** | **ROA** | **Dosing Frequency & Duration** |
|---|---|---|---|---|---|---|
| 1 | 6 | Day 0, *s.c,* Jeko-1 (5x10⁶/100 µL(PBS with matrigel (1:1))/mouse) | Day 3, *i.p.* PBMCs (1x10⁷/100 µL PBS/mouse ) | PBS, N/A | | biw × 4 weeks |
| 2 | 6 | | | 0205-2, 1mpk | *i.p.* | |
| 3 | 6 | | | 0205-2, 10mpk | | |

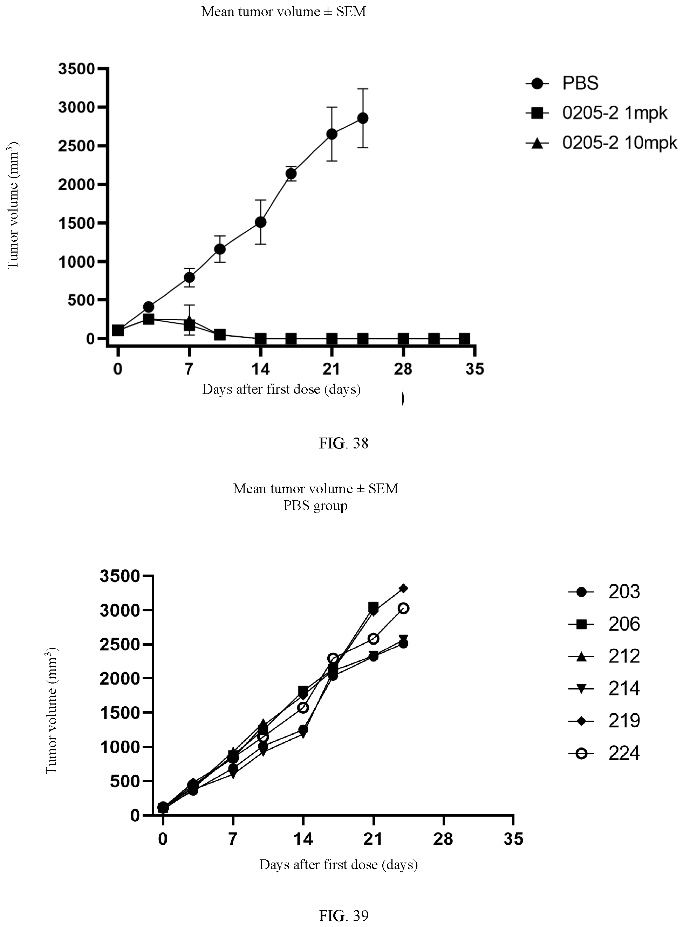

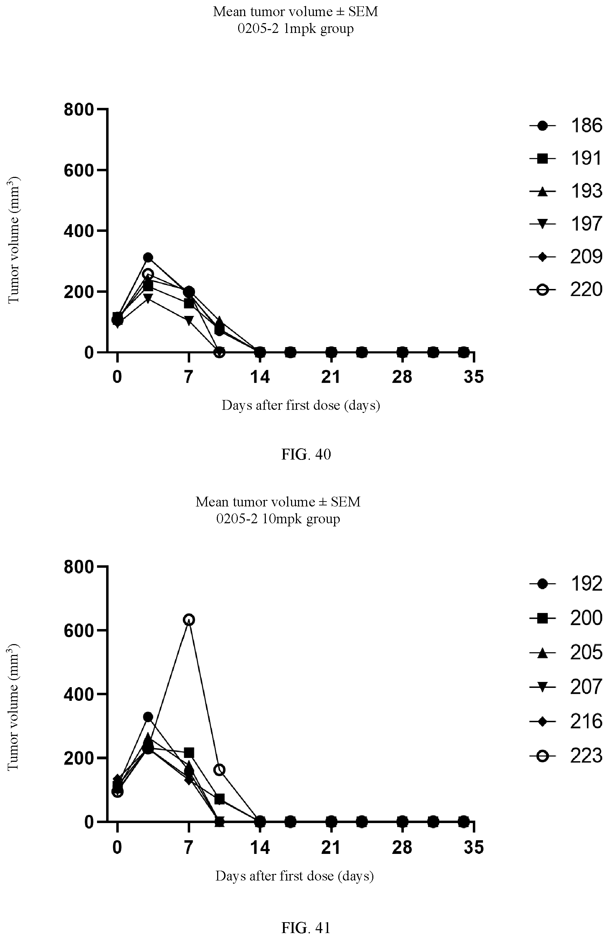

As shown in FIG. 38, administration was initiated when tumor volume reached 110 mm³. Rapid tumor shrinkage and subsequent complete tumor regression were observed in mice in both dose groups (1 mpk and 10 mpk), with no significant difference between the two groups. Tumor volume changes in the 1 mpk group were similar to those in the PBS group. In the 10 mpk group, one mouse still achieved rapid and complete tumor regression even when its tumor volume reached 650 mm³ (FIGS. 39-41).

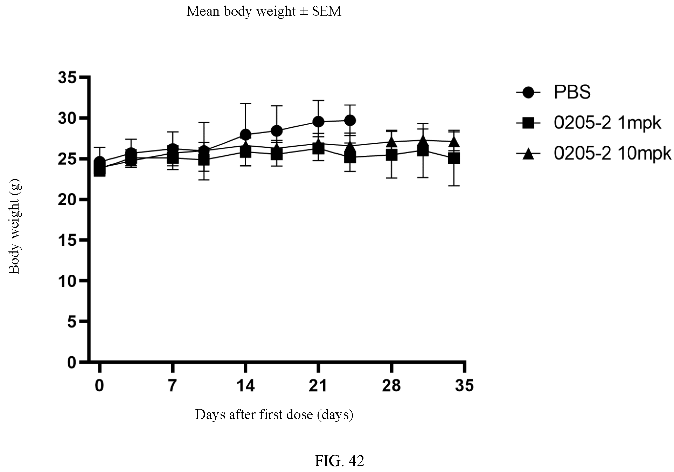

As shown in FIG. 42, no mouse death was observed in either the 1 mpk or 10 mpk group during the observation period. No obvious weight loss, decreased activity, hunched back, ruffled fur or other adverse signs were detected in mice, indicating that the mice were well-tolerated to the drug.

### References

1. Wang S, Wang L, Hu J, Qian W, Zhang X, Hu Y, Zhu Q, Chen B, Wu D, Chang CH et al: Outcomes in refractory diffuse large B-cell lymphoma: results from a multicenter real-world study in China. Cancer Commun (Lond) 2021, 41(3):229-239.
2. Spiegel JY, Patel S, Muffly L, Hossain NM, Oak J, Baird JH, Frank MJ, Shiraz P, Sahaf B, Craig J et al: CAR T cells with dual targeting of CD19 and CD22 in adult patients with recurrent or refractory B cell malignancies: a phase 1 trial. Nat Med 2021, 27(8): 1419-1431.
3. Rodig SJ, Shahsafaei A, Li B, Mackay CR, Dorfman DM: BAFF-R, the major B cell-activating factor receptor, is expressed on most mature B cells and B-cell lymphoproliferative disorders. Hum Pathol 2005, 36(10):1113-1119.

### Sequences of the Invention

CD8α signal peptide Nucleic acid sequence SEQ ID NO. 1 
ATGGCCCTGCCTGTGACAGCTCTGCTCCTCCCTCTGGCCCTGCTGCTCCATGCCGCCAGA
CCC
CD8α Signal peptide Protein sequence SEQ ID NO. 2
   MALPVTALLLPLALLLHAARP
CD8α Hinge region Nucleic acid sequence SEQ ID NO. 3 
TTCGTGCCCGTGTTCCTGCCCGCCAAACCTACTACTACCCCTGCACCTAGGCCTCCCACC
CCAGCCCCAACAATCGCCAGCCAGCCTCTGTCTCTGCGGCCCGAAGCCTGTAGACCTGCTGC
CGGCGGAGCCGTGCACACCAGAGGCCTGGACTTCGCCTGCGAC
CD8α Hinge region Protein sequence SEQ ID NO. 4
   FVPVFLPAKPTTTPAPRPPTPAPTIASQPLSLRPEACRPAAGGAVHTRGLDFACD
CD8α Transmembrane region Nucleic acid sequence SEQ ID NO. 5 
ATCTACATCTGGGCCCCTCTGGCCGGCACCTGTGGCGTGCTGCTGCTGAGCCTGGTGATC
ACCCTGTACTGCAACCACCGGAAC
CD8α Transmembrane region Protein sequence SEQ ID NO. 6
   IYIWAPLAGTCGVLLLSLVITLYCNHRN
4-1BB Intracellular domain Nucleic acid sequence SEQ ID NO. 7 
AAACGGGGCAGAAAGAAACTCCTGTATATATTCAAACAACCATTTATGAGACCAGTACA
AACTACTCAAGAGGAAGATGGCTGTAGCTGCCGATTTCCAGAAGAAGAAGGAGGATGT
GAACTG
4-1BB Intracellular domain Protein sequence SEQ ID NO. 8
   KRGRKKLLYIFKQPFMRPVQTTQEEDGCSCRFPEEEEGGCEL
CD3ζ Intracellular signaling domain Nucleic acid sequence SEQ ID NO. 9 
AGAGTGAAGTTCAGCAGATCCGCCGACGCCCCTGCCTACCAGCAGGGACAGAACCAGCT
GTACAACGAGCTGAACCTGGGCAGACGGGAAGAGTACGACGTGCTGGACAAGCGGAGAGG
CCGGGACCCCGAGATGGGCGGAAAGCCCAGACGGAAGAACCCCCAGGAAGGCCTGTATAA
CGAACTGCAGAAAGACAAGATGGCCGAGGCCTACAGCGAGATCGGCATGAAGGGCGAGCG
GAGGCGCGGCAAGGGCCACGATGGCCTGTACCAGGGCCTGAGCACCGCCACCAAGGACACC
TACGACGCCCTGCACATGCAGGCCCTGCCCCCCAGA
CD3ζ Intracellular signaling domain Protein sequence SEQ ID NO. 10 
RVKFSRSADAPAYQQGQNQLYNELNLGRREEYDVLDKRRGRDPEMGGKPRRKNPQEGLYN
ELQKDKMAEAYSEIGMKGERRRGKGHDGLYQGLSTATKDTYDALHMQALPPR
Cleaving peptide T2A Nucleic acid sequence SEQ ID NO. 11
   GAGGGAAGGGGCAGCTTATTAACATGTGGCGATGTGGAAGAGAACCCCGGTCCC
Cleaving peptide T2A Protein sequence SEQ ID NO. 12
   EGRGSLLTCGDVEENPGP
CSF2RA signal Nucleic acid sequence SEQ ID NO. 13 
ATGCTGCTGCTCGTGACCTCTTTACTGTTATGTGAGCTGCCCCACCCCGCTTTTTTACTGA
TCCCT
CSF2RA signal protein sequence SEQ ID NO. 14 MLLLVTSLLLCELPHPAFLLIP
EGFRt Nucleic acid sequence SEQ ID NO. 15 
CGTAAGGTGTGTAACGGAATCGGCATTGGCGAGTTCAAGGACTCTTTAAGCATCAACGC
CACAAACATCAAGCACTTCAAGAATTGTACCTCCATCAGCGGCGATTTACACATTCTCCCCG
TGGCTTTTCGTGGCGATTCCTTCACCCACACCCCCCCTCTGGACCCCCAAGAGCTGGACATTT
 
TAAAAACCGTGAAGGAGATCACCGGCTTTCTGCTGATCCAAGCTTGGCCCGAGAATCGTACC
GACCTCCACGCCTTCGAGAATTTAGAGATTATTCGTGGAAGGACCAAGCAGCACGGCCAGTT
CTCTTTAGCCGTCGTGTCTTTAAACATTACCAGCCTCGGTTTAAGGTCTTTAAAGGAGATTAG
CGACGGCGACGTGATCATCTCCGGCAACAAGAACCTCTGCTACGCCAACACCATCAACTGG
AAGAAGCTGTTCGGAACCAGCGGCCAAAAGACCAAGATCATCAGCAATCGTGGAGAGAACT
CTTGTAAGGCCACTGGTCAAGTTTGCCACGCCCTCTGTAGCCCCGAAGGATGTTGGGGCCCC
GAGCCTAGGGACTGTGTTAGCTGCAGAAACGTGAGCAGAGGCAGAGAGTGTGTGGACAAAT
GCAATTTACTGGAAGGAGAGCCTAGGGAGTTCGTGGAGAACAGCGAATGTATCCAGTGCCA
CCCCGAGTGTTTACCTCAAGCCATGAACATCACTTGTACCGGAAGGGGCCCCGATAACTGCA
TCCAATGCGCCCACTACATCGACGGACCCCACTGCGTGAAAACTTGTCCCGCCGGAGTGATG
GGAGAGAATAACACTTTAGTGTGGAAGTACGCCGACGCTGGCCACGTCTGCCATCTGTGCCA
CCCCAACTGTACCTACGGCTGCACTGGTCCCGGTTTAGAGGGATGTCCTACCAACGGCCCCA
AGATCCCCTCCATCGCCACCGGAATGGTGGGCGCTCTGTTATTACTGCTGGTGGTGGCTCTG
GGCATCGGTTTATTCATG
EGFRt Protein sequence SEQ ID NO. 16 
RKVCNGIGIGEFKDSLSINATNIKHFKNCTSISGDLHILPVAFRGDSFTHTPPLDPQELDILKTV
KEITGFLLIQAWPENRTDLHAFENLEIIRGRTKQHGQFSLAVVSLNITSLGLRSLKEISDGDVIISGN
KNLCYANTINWKKLFGTSGQKTKIISNRGENSCKATGQVCHALCSPEGCWGPEPRDCVSCRNVS
RGRECVDKCNLLEGEPREFVENSECIQCHPECLPQAMNITCTGRGPDNCIQCAHYIDGPHCVKTC
PAGVMGENNTLVWKYADAGHVCHLCHPNCTYGCTGPGLEGCPTNGPKIPSIATGMVGALLLLL
VVALGIGLFM
P2A Nucleic acid sequence SEQ ID NO. 17
   gctactaacttcagcctgctgaagcaggctggtgacgtcgaggagaatcctggcccc
P2A Protein sequence SEQ ID NO. 18
   ATNFSLLKQAGDVEENPGP
BAFF-R-5 CAR VHH Nucleic acid sequence SEQ ID NO. 19 
GAGGTGCAGCTGGTGGAGTCTGGGGGAGGCGTGGTCCAGCCTGGGAGGTCCCTGAGACT
CACCTGTGCAGCCTCTGGATTCACGTTCAGTACCTCTGGCATGTACTGGATTCGCCAGGCTCC
AGGCAAGGGGCTGGAGTGGGTTTCATACATTAGTAGTAGTAGTAGTACCATATACTACGCAG
ACTCCGTGAAGGGCCGGTTCACCATCTCCAGAGACAATTCCAAGAACACGCTGTATCTGCAA
ATGAACAGCCTGAGAGCTGAGGACACGGCTGTGTATTATTGTGTGACTCAGTCGTGTGGTGC
TGGTAAATGCTACCCGGGGGTCTGGGGCCAAGGGACCACGGTCACTGTCTCCTCA
BAFF-R-5 CARVHH Amino acid sequence SEQ ID NO. 20 
EVQLVESGGGVVQPGRSLRLTCAASGFTFSTSGMYWIRQAPGKGLEWVSYISSSSSTIYYADS
VKGRFTISRDNSKNTLYLQMNSLRAEDTAVYYCVTQSCGAGKCYPGVWGQGTTVTVSS
Vay736 Nucleic acid sequence SEQ ID NO. 21 
CAGGTGCAGCTGCAGCAGAGCGGCCCCGGCCTGGTGAAGCCCAGCCAGACCCTGAGCCT
GACCTGCGCCATCAGCGGCGACAGCGTGAGCAGCAACAGCGCCGCCTGGGGCTGGATCCGC
 
CAGAGCCCCGGCCGCGGCCTGGAGTGGCTGGGCCGCATCTACTACCGCAGCAAGTGGTACA
ACAGCTACGCCGTGAGCGTGAAGAGCCGCATCACCATCAACCCCGACACCAGCAAGAACCA
GTTCAGCCTGCAGCTGAACAGCGTGACCCCCGAGGACACCGCCGTGTACTACTGCGCCCGCT
ACCAGTGGGTGCCCAAGATCGGCGTGTTCGACAGCTGGGGCCAGGGCACCCTGGTGACCGT
GAGCAGCGGAGGAGGCGGCTCCGGCGGCGGCGGCAGCGGCGGAGGAGGCTCTGACATCGT
GCTGACCCAGAGCCCCGCCACCCTGAGCCTGAGCCCCGGCGAGCGCGCCACCCTGAGCTGC
CGCGCCAGCCAGTTCATCCTGCCCGAGTACCTGAGCTGGTACCAGCAGAAGCCCGGCCAGG
CCCCCCGCCTGCTGATCTACGGCAGCAGCAGCCGCGCCACCGGCGTGCCCGCCCGCTTCAGC
GGCAGCGGCAGCGGCACCGACTTCACCCTGACCATCAGCAGCCTGGAGCCCGAGGACTTCG
CCGTGTACTACTGCCAGCAGTTCTACAGCAGCCCCCTGACCTTCGGCCAGGGCACCAAGGTG
GAGATCAAG
Vay736 Amino Acid Sequence SEQ ID NO. 22 
QVQLQQSGPGLVKPSQTLSLTCAISGDSVSSNSAAWGWIRQSPGRGLEWLGRIYYRSKWYNS
YAVSVKSRITINPDTSKNQFSLQLNSVTPEDTAVYYCARYQWVPKIGVFDSWGQGTLVTVSSGG
GGSGGGGSGGGGSDIVLTQSPATLSLSPGERATLSCRASQFILPEYLSWYQQKPGQAPRLLIYGSS
SRATGVPARFSGSGSGTDFTLTISSLEPEDFAVYYCQQFYSSPLTFGQGTKVEIK
city of hope CAR scFV Nucleic Acid Sequence SEQ ID NO. 23 
GTGCAGCTGCAGGAGAGCGGCCCCGGCCTGGTGAAGCCCAGCCAGACCCTGAGCCTTAC
TTGCACGGTGAGCGGTGACAGCATCACCAGCGGCTACTGGAACTGGATCCGCCAGCACCCC
GGCAAGGGCCTGGAGTACATCGGCTACATCAGCTACAGCGGCAGCACCTACTACAACCCCA
GCCTGAAGAGCCGCGTGACCATCAGCCGCGACACCAGCAAGAACCAGTACAGCCTGAAGCT
GAGCAGCGTGACCGCCGCCGACACCGCCGTGTACTACTGCGCCAGCCCCAACTACCCCTTCT
ACGCCATGGACTACTGGGGCCAGGGCACCCTGGTGACCGTGAGCAGCGGCGGCGGCGGCAG
CGGCGGCGGCGGCAGCGGCGGCGGCGGCAGCGACATCGTGCTGACCCAGAGCCCCGCCACC
CTGAGCCTGAGCCCCGGCGAGCGCGCCACCCTGAGCTGCCGCGCCAGCGAGAGCGTGGACA
ACTACGGCATCAGCTTCATGAACTGGTTCCAGCAGAAGCCCGGCCAGGCCCCCCGCCTGCTG
ATCTACGCCGCCAGCAACCGCGCCACCGGCATCCCCGCCCGCTTCAGCGGCAGCGGCAGCG
GCACCGACTTCACCCTGACCATCAGCAGCCTGGAGCCCGAGGACTTCGCCGTGTACTACTGC
CAGCAGAGCAAGGAGGTGCCCTGGACCTTCGGCGGCGGCACCAAGGTGGAGATCAAGCGC
city of hope CAR scFV Amino Acid Sequence SEQ ID NO. 24 
VQLQESGPGLVKPSQTLSLTCTVSGDSITSGYWNWIRQHPGKGLEYIGYISYSGSTYYNPSLK
SRVTISRDTSKNQYSLKLSSVTAADTAVYYCASPNYPFYAMDYWGQGTLVTVSSGGGGSGGGG
SGGGGSDIVLTQSPATLSLSPGERATLSCRASESVDNYGISFMNWFQQKPGQAPRLLIYAASNRA
TGIPARFSGSGSGTDFTLTISSLEPEDFAVYYCQQSKEVPWTFGGGTKVEIKR
05VH Amino Acid Sequence SEQ ID NO. 25 
EVQLVESGGGVVQPGRSLRLTCAASGFTFSTSGMYWIRQAPGKGLEWVSYISSSSSTIYYADS
VKGRFTISRDNSKNTLYLQMNSLRAEDTAVYYCVTQSCGAGKCYPGVWGQGTTVTVSS
05VH Nucleotide sequence SEQ ID NO. 26 
GAGGTGCAGCTGGTGGAGTCTGGGGGAGGCGTGGTCCAGCCTGGGAGGTCCCTGAGACT
CACCTGTGCAGCCTCTGGATTCACGTTCAGTACCTCTGGCATGTACTGGATTCGCCAGGCTCC
AGGCAAGGGGCTGGAGTGGGTTTCATACATTAGTAGTAGTAGTAGTACCATATACTACGCAG
ACTCCGTGAAGGGCCGGTTCACCATCTCCAGAGACAATTCCAAGAACACGCTGTATCTGCAA
ATGAACAGCCTGAGAGCTGAGGACACGGCTGTGTATTATTGTGTGACTCAGTCGTGTGGTGC
TGGTAAATGCTACCCGGGGGTCTGGGGCCAAGGGACCACGGTCACTGTCTCCTCA
05VH CDR1 SEQ ID NO. 27 GFTFSTSG
05VH CDR2 SEQ ID NO. 28 ISSSSSTI
05VH CDR3 SEQ ID NO. 29 VTQSCGAGKCYPGV
01VL Amino acid sequence SEQ ID NO.30 
DIQMTQSPSSLSASVGDRVTITCRASQKISSYLNWYQQKPGKAPKLLIYAASSLQSGVPSRFS
GSGSGADFTLTISSLQPEDFATYYCQQSYSPPTTFGGGTKVDIKR
01VL Nucleotide sequence SEQ ID NO.31 
GACATCCAGATGACCCAGTCTCCATCCTCCCTGTCTGCATCTGTTGGAGACAGAGTCACC
ATCACTTGCCGGGCAAGTCAGAAAATTAGCAGCTATTTAAATTGGTATCAGCAGAAACCAG
GGAAAGCCCCTAAGCTCCTGATCTATGCTGCATCCAGTTTGCAAAGTGGGGTCCCATCAAGG
TTCAGTGGCAGTGGATCTGGGGCAGATTTCACTCTCACCATCAGCAGTCTGCAACCTGAAGA
TTTTGCAACTTACTACTGTCAGCAGAGTTACAGTCCCCCGACCACTTTCGGCGGAGGGACCA
AAGTGGATATCAAACGT
01VH Amino acid sequence SEQ ID NO.32 
MAEVQLVESGAEVKKPGASVKVSCKASGYTFTSYGISWVRQAPGQGLEWMGWISAYNGNT
NYAQKLQGRVTMTTDTSTSTAYMELRSLRSDDTAVYYCARNTYYYDSSGYPNDAFDIWGQGTT
VTVSS
01VH Nucleotide sequence SEQ ID NO.33 
ATGGCCGAGGTGCAGCTGGTGGAGTCTGGAGCTGAGGTGAAGAAGCCTGGGGCCTCAGT
GAAGGTCTCCTGCAAGGCTTCTGGTTACACCTTTACCAGCTATGGTATCAGCTGGGTGCGAC
AGGCCCCTGGACAAGGGCTTGAGTGGATGGGATGGATCAGCGCTTACAATGGTAACACAAA
CTATGCACAGAAGCTCCAGGGCAGAGTCACCATGACCACAGACACATCCACGAGCACAGCC
TACATGGAGCTGAGGAGCCTGAGATCTGACGACACGGCCGTGTATTACTGTGCGAGAAATA
CGTATTACTATGATAGTAGTGGTTACCCAAATGATGCTTTTGATATCTGGGGCCAAGGGACC
ACGGTCACCGTCTCCTCA
Amino acid sequence of the scFv(VL-VH) contained in the CAR constructed from clone 1 (01) SEQ ID NO.34 
DIQMTQSPSSLSASVGDRVTITCRASQKISSYLNWYQQKPGKAPKLLIYAASSLQSGVPSRFS
GSGSGADFTLTISSLQPEDFATYYCQQSYSPPTTFGGGTKVDIKRSRGGGGSGGGGSGGGGSLEM
AEVQLVESGAEVKKPGASVKVSCKASGYTFTSYGISWVRQAPGQGLEWMGWISAYNGNTNYA
QKLQGRVTMTTDTSTSTAYMELRSLRSDDTAVYYCARNTYYYDSSGYPNDAFDIWGQGTTVTV
SS
Nucleotide sequence of the scFv(VL-VH) contained in the CAR constructed from clone 1 (01) SEQ ID NO.35 
GACATCCAGATGACCCAGTCTCCATCCTCCCTGTCTGCATCTGTTGGAGACAGAGTCACC
ATCACTTGCCGGGCAAGTCAGAAAATTAGCAGCTATTTAAATTGGTATCAGCAGAAACCAG
GGAAAGCCCCTAAGCTCCTGATCTATGCTGCATCCAGTTTGCAAAGTGGGGTCCCATCAAGG
TTCAGTGGCAGTGGATCTGGGGCAGATTTCACTCTCACCATCAGCAGTCTGCAACCTGAAGA
TTTTGCAACTTACTACTGTCAGCAGAGTTACAGTCCCCCGACCACTTTCGGCGGAGGGACCA
AAGTGGATATCAAACGTTCTAGAGGTGGTGGTGGTAGCGGCGGCGGCGGCTCTGGTGGTGG
TGGATCCCTCGAGATGGCCGAGGTGCAGCTGGTGGAGTCTGGAGCTGAGGTGAAGAAGCCT
GGGGCCTCAGTGAAGGTCTCCTGCAAGGCTTCTGGTTACACCTTTACCAGCTATGGTATCAG
CTGGGTGCGACAGGCCCCTGGACAAGGGCTTGAGTGGATGGGATGGATCAGCGCTTACAAT
GGTAACACAAACTATGCACAGAAGCTCCAGGGCAGAGTCACCATGACCACAGACACATCCA
CGAGCACAGCCTACATGGAGCTGAGGAGCCTGAGATCTGACGACACGGCCGTGTATTACTG
TGCGAGAAATACGTATTACTATGATAGTAGTGGTTACCCAAATGATGCTTTTGATATCTGGG
GCCAAGGGACCACGGTCACCGTCTCCTCA
01VL CDR1 SEQ ID NO.36 QKISSY
01VL CDR2 AAS
01VL CDR3 SEQ ID NO.37 QQSYSPPTT
01VH CDR1 SEQ ID NO.38 GYTFTSYG
01VH CDR2 SEQ ID NO.39 ISAYNGNT
01VH CDR3 SEQ ID NO.40 ARNTYYYDSSGYPNDAFDI
SEQ ID NO. 41 hFc PAA Hs Amino acid sequence 
ESKYGPPCPPCPAPEAAGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAKGQPR
EPQVCTLPPSQEEMTKNQVSLSCAVKGFYPSDIAVEWESNGQPENNYKTTPPVLDSDGSFFLVSR
LTVDKSRWQEGNVFSCSVMHEALHNHYTQKSLSLSLGK
SEQ ID NO. 42 hFc PAA Hs Nucleotide sequence 
GAGAGCAAGTACGGCCCCCCCTGCCCCCCCTGCCCCGCCCCCGAGGCCGCCGGCGGCCC
CAGCGTGTTCCTGTTCCCCCCCAAGCCCAAGGACACCCTGATGATCAGCCGCACCCCCGAGG
TGACCTGCGTGGTGGTGGACGTGAGCCAGGAGGACCCCGAGGTGCAGTTCAACTGGTACGT
GGACGGCGTGGAGGTGCACAACGCCAAGACCAAGCCCCGCGAGGAGCAGTTCAACAGCACC
TACCGCGTGGTGAGCGTGCTGACCGTGCTGCACCAGGACTGGCTGAACGGCAAGGAGTACA
AGTGCAAGGTGAGCAACAAGGGCCTGCCCAGCAGCATCGAGAAGACCATCAGCAAGGCCA
AGGGCCAGCCCCGCGAGCCCCAGGTGtgcACCCTGCCCCCCAGCCAGGAGGAGATGACCAAG
AACCAGGTGAGCCTGagcTGCgccGTGAAGGGCTTCTACCCCAGCGACATCGCCGTGGAGTGG
GAGAGCAACGGCCAGCCCGAGAACAACTACAAGACCACCCCCCCCGTGCTGGACAGCGACG
GCAGCTTCTTCCTGgtgAGCagaCTGACCGTGGACAAGAGCCGCTGGCAGGAGGGCAACGTGTT
 
CAGCTGCAGCGTGATGCACGAGGCCCTGCACAACCACTACACCCAGAAGAGCCTGAGCCTG
AGCCTGGGCAAG
SEQ ID NO. 43 hFc PAA Ks Amino acid sequence 
ESKYGPPCPPCPAPEAAGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAKGQPR
EPQVYTLPPCQEEMTKNQVSLWCLVKGFYPSDIAVEWESNGQPENNYKTTPPVLDSDGSFFLYS
RLTVDKSRWQEGNVFSCSVMHEALHNHYTQKSLSLSLGK
SEQ ID NO. 44 hFc PAA Ks Nucleotide sequence 
GAGAGCAAGTACGGCCCCCCCTGCCCCCCCTGCCCCGCCCCCGAGGCCGCCGGCGGCCC
CAGCGTGTTCCTGTTCCCCCCCAAGCCCAAGGACACCCTGATGATCAGCCGCACCCCCGAGG
TGACCTGCGTGGTGGTGGACGTGAGCCAGGAGGACCCCGAGGTGCAGTTCAACTGGTACGT
GGACGGCGTGGAGGTGCACAACGCCAAGACCAAGCCCCGCGAGGAGCAGTTCAACAGCACC
TACCGCGTGGTGAGCGTGCTGACCGTGCTGCACCAGGACTGGCTGAACGGCAAGGAGTACA
AGTGCAAGGTGAGCAACAAGGGCCTGCCCAGCAGCATCGAGAAGACCATCAGCAAGGCCA
AGGGCCAGCCCCGCGAGCCCCAGGTGTACACCCTGCCCCCCtgcCAGGAGGAGATGACCAAG
AACCAGGTGAGCCTGtggTGCCTGGTGAAGGGCTTCTACCCCAGCGACATCGCCGTGGAGTGG
GAGAGCAACGGCCAGCCCGAGAACAACTACAAGACCACCCCCCCCGTGCTGGACAGCGACG
GCAGCTTCTTCCTGTACAGCAGACTGACCGTGGACAAGAGCCGCTGGCAGGAGGGCAACGT
GTTCAGCTGCAGCGTGATGCACGAGGCCCTGCACAACCACTACACCCAGAAGAGCCTGAGC
CTGAGCCTGGGCAAG
SEQ ID NO. 45 OKT3 VH Amino acid sequence 
QVQLVQSGGGVVQPGRSLRLSCKASGYTFTRYTMHWVRQAPGKGLEWIGYINPSRGYTNY
NQKFKDRFTISRDNSKNTAFLQMDSLRPEDTGVYFCARYYDDHYCLDYWGQGTPVTVSS
SEQ ID NO. 46 OKT3 VH Nucleotide sequence 
CAGGTGCAGCTGGTGCAGAGCGGCGGCGTGGTGCAGCCCGGCCGCAGCCTGCGCCT
GAGCTGCAAGGCCAGCGGCTACACCTTCACCCGCTACACCATGCACTGGGTGCGCCAGGCCC
CCGGCAAGGGCCTGGAGTGGATCGGCTACATCAACCCCAGCCGCGGCTACACCAACTACAA
CCAGAAGTTCAAGGACCGCTTCACCATCAGCCGCGACAACAGCAAGAACACCGCCTTCCTG
CAGATGGACAGCCTGCGCCCCGAGGACACCGGCGTGTACTTCTGCGCCCGCTACTACGACGA
CCACTACTGCCTGGACTACTGGGGCCAGGGCACCCCCGTGACCGTGAGCAGC
SEQ ID NO. 47 OKT3 VK Amino acid sequence 
DIQMTQSPSSLSASVGDRVTITCSASSSVSYMNWYQQTPGKAPKRWIYDTSKLASGVPSRFS
GSGSGTDYTFTISSLQPEDIATYYCQQWSSNPFTFGQGTKLQITR
SEQ ID NO. 48 OKT3 VK Nucleotide sequence 
GACATCCAGATGACCCAGAGCCCCAGCAGCCTGAGCGCCAGCGTGGGCGACCGCGTGAC
CATCACCTGCAGCGCCAGCAGCAGCGTGAGCTACATGAACTGGTACCAGCAGACCCCCGGC
AAGGCCCCCAAGCGCTGGATCTACGACACCAGCAAGCTGGCCAGCGGCGTGCCCAGCCGCT
TCAGCGGCAGCGGCAGCGGCACCGACTACACCTTCACCATCAGCAGCCTGCAGCCCGAGGA
 
CATCGCCACCTACTACTGCCAGCAGTGGAGCAGCAACCCCTTCACCTTCGGCCAGGGCACCA
AGCTGCAGATCACCCGC
SEQ ID NO. 49 OKT3 scFab Amino acid sequence 
DIQMTQSPSSLSASVGDRVTITCSASSSVSYMNWYQQTPGKAPKRWIYDTSKLASGVPSRFS
GSGSGTDYTFTISSLQPEDIATYYCQQWSSNPFTFGQGTKLQITRRTVAAPSVFIFPPSDEQLKSGT
ASVVCLLNNFYPREAKVQWKVDNALQSGNSQESVTEQDSKDSTYSLSSTLTLSKADYEKHKVY
ACEVTHQGLSSPVTKSFNRGECGGGGSGGGGSGGGGSGGGGSGGGGSGGGGSQVQLVQSGGG
VVQPGRSLRLSCKASGYTFTRYTMHWVRQAPGKGLEWIGYINPSRGYTNYNQKFKDRFTISRDN
SKNTAFLQMDSLRPEDTGVYFCARYYDDHYCLDYWGQGTPVTVSSASTKGPSVFPLAPCSRSTS
ESTAALGCLVKDYFPEPVTVSWNSGALTSGVHTFPAVLQSSGLYSLSSVVTVPSSSLGTKTYTCN
VDHKPSNTKVDKRV
SEQ ID NO. 50 OKT3 scFab Nucleotide sequence 
GACATCCAGATGACCCAGAGCCCCAGCAGCCTGAGCGCCAGCGTGGGCGACCGCGTGAC
CATCACCTGCAGCGCCAGCAGCAGCGTGAGCTACATGAACTGGTACCAGCAGACCCCCGGC
AAGGCCCCCAAGCGCTGGATCTACGACACCAGCAAGCTGGCCAGCGGCGTGCCCAGCCGCT
TCAGCGGCAGCGGCAGCGGCACCGACTACACCTTCACCATCAGCAGCCTGCAGCCCGAGGA
CATCGCCACCTACTACTGCCAGCAGTGGAGCAGCAACCCCTTCACCTTCGGCCAGGGCACCA
AGCTGCAGATCACCCGCCGTACGGTGGCCGCACCAAGCGTCTTCATCTTCCCGCCATCTGAT
GAGCAGTTGAAATCTGGAACTGCCTCTGTTGTGTGCCTGCTGAATAACTTCTATCCCAGAGA
GGCCAAAGTACAGTGGAAGGTGGATAACGCCCTCCAATCGGGTAACTCCCAGGAGAGTGTC
ACAGAGCAGGACAGCAAGGACAGCACCTACAGCCTCAGCAGCACCCTGACGCTGAGCAAAG
CAGACTACGAGAAACACAAAGTCTACGCCTGCGAAGTCACCCATCAGGGCCTGAGCTCGCC
CGTCACAAAGAGCTTTAACAGAGGCGAGTGCGGCGGCGGCGGATCCGGCGGCGGCGGAAGC
GGCGGCGGCGGATCCGGCGGCGGCGGAAGCGGCGGCGGCGGATCCGGCGGCGGAGGAAGC
CAGGTGCAGCTGGTGCAGAGCGGCGGCGGCGTGGTGCAGCCCGGCCGCAGCCTGCGCCTGA
GCTGCAAGGCCAGCGGCTACACCTTCACCCGCTACACCATGCACTGGGTGCGCCAGGCCCCC
GGCAAGGGCCTGGAGTGGATCGGCTACATCAACCCCAGCCGCGGCTACACCAACTACAACC
AGAAGTTCAAGGACCGCTTCACCATCAGCCGCGACAACAGCAAGAACACCGCCTTCCTGCA
GATGGACAGCCTGCGCCCCGAGGACACCGGCGTGTACTTCTGCGCCCGCTACTACGACGACC
ACTACTGCCTGGACTACTGGGGCCAGGGCACCCCCGTGACCGTGAGCAGCGCTAGCACCAA
GGGCCCCAGCGTGTTCCCCCTGGCCCCCTGCAGCCGCAGCACCAGCGAGAGCACCGCCGCCC
TGGGCTGCCTGGTGAAGGACTACTTCCCCGAGCCCGTGACCGTGAGCTGGAACAGCGGCGC
CCTGACCAGCGGCGTGCACACCTTCCCCGCCGTGCTGCAGAGCAGCGGCCTGTACAGCCTGA
GCAGCGTGGTGACCGTGCCCAGCAGCAGCCTGGGCACCAAGACCTACACCTGCAACGTGGA
CCACAAGCCCAGCAACACCAAGGTGGACAAGCGCGTG
SEQ ID NO. 51 05VH-hFc PAA Hs Amino acid sequence 
EVQLVESGGGVVQPGRSLRLTCAASGFTFSTSGMYWIRQAPGKGLEWVSYISSSSSTIYYADS
VKGRFTISRDNSKNTLYLQMNSLRAEDTAVYYCVTQSCGAGKCYPGVWGQGTTVTVSSESKYG
 
PPCPPCPAPEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPREPQVCTLP
PSQEEMTKNQVSLSCAVKGFYPSDIAVEWESNGQPENNYKTTPPVLDSDGSFFLVSRLTVDKSR
WQEGNVFSCSVMHEALHNHYTQKSLSLSLGK
SEQ ID NO. 52 05VH-hFc PAA Hs Nucleotide sequence 
GAGGTGCAGCTGGTGGAGTCTGGGGGAGGCGTGGTCCAGCCTGGGAGGTCCCTGAGACT
CACCTGTGCAGCCTCTGGATTCACGTTCAGTACCTCTGGCATGTACTGGATTCGCCAGGCTCC
AGGCAAGGGGCTGGAGTGGGTTTCATACATTAGTAGTAGTAGTAGTACCATATACTACGCAG
ACTCCGTGAAGGGCCGGTTCACCATCTCCAGAGACAATTCCAAGAACACGCTGTATCTGCAA
ATGAACAGCCTGAGAGCTGAGGACACGGCTGTGTATTATTGTGTGACTCAGTCGTGTGGTGC
TGGTAAATGCTACCCGGGGGTCTGGGGCCAAGGGACCACGGTCACTGTCTCCTCAGAGAGC
AAGTACGGCCCCCCCTGCCCCCCCTGCCCCGCCCCCGAGGCCGCCGGCGGCCCCAGCGTGTT
CCTGTTCCCCCCCAAGCCCAAGGACACCCTGATGATCAGCCGCACCCCCGAGGTGACCTGCG
TGGTGGTGGACGTGAGCCAGGAGGACCCCGAGGTGCAGTTCAACTGGTACGTGGACGGCGT
GGAGGTGCACAACGCCAAGACCAAGCCCCGCGAGGAGCAGTTCAACAGCACCTACCGCGTG
GTGAGCGTGCTGACCGTGCTGCACCAGGACTGGCTGAACGGCAAGGAGTACAAGTGCAAGG
TGAGCAACAAGGGCCTGCCCAGCAGCATCGAGAAGACCATCAGCAAGGCCAAGGGCCAGCC
CCGCGAGCCCCAGGTGtgcACCCTGCCCCCCAGCCAGGAGGAGATGACCAAGAACCAGGTGA
GCCTGagcTGCgccGTGAAGGGCTTCTACCCCAGCGACATCGCCGTGGAGTGGGAGAGCAACG
GCCAGCCCGAGAACAACTACAAGACCACCCCCCCCGTGCTGGACAGCGACGGCAGCTTCTT
CCTGgtgAGCagaCTGACCGTGGACAAGAGCCGCTGGCAGGAGGGCAACGTGTTCAGCTGCAG
CGTGATGCACGAGGCCCTGCACAACCACTACACCCAGAAGAGCCTGAGCCTGAGCCTGGGC
AAG
SEQ ID NO. 53 OKT3 scFab-H PAA Ks Amino acid sequence 
DIQMTQSPSSLSASVGDRVTITCSASSSVSYMNWYQQTPGKAPKRWIYDTSKLASGVPSRFS
GSGSGTDYTFTISSLQPEDIATYYCQQWSSNPFTFGQGTKLQITRRTVAAPSVFIFPPSDEQLKSGT
ASVVCLLNNFYPREAKVQWKVDNALQSGNSQESVTEQDSKDSTYSLSSTLTLSKADYEKHKVY
ACEVTHQGLSSPVTKSFNRGECGGGGSGGGGSGGGGSGGGGSGGGGSGGGGSQVQLVQSGGG
VVQPGRSLRLSCKASGYTFTRYTMHWVRQAPGKGLEWIGYINPSRGYTNYNQKFKDRFTISRDN
SKNTAFLQMDSLRPEDTGVYFCARYYDDHYCLDYWGQGTPVTVSSASTKGPSVFPLAPCSRSTS
ESTAALGCLVKDYFPEPVTVSWNSGALTSGVHTFPAVLQSSGLYSLSSVVTVPSSSLGTKTYTCN
VDHKPSNTKVDKRVESKYGPPCPPCPAPEAAGGPSVFLFPPKPKDTLMISRTPEVTCVVVDVSQE
DPEVQFNWYVDGVEVHNAKTKPREEQFNSTYRVVSVLTVLHQDWLNGKEYKCKVSNKGLPSSI
EKTISKAKGQPREPQVYTLPPCQEEMTKNQVSLWCLVKGFYPSDIAVEWESNGQPENNYKTTPP
VLDSDGSFFLYSRLTVDKSRWQEGNVFSCSVMHEALHNHYTQKSLSLSLGK
SEQ ID NO. 54 OKT3 scFab-H PAA Ks Nucleotide sequence 
GACATCCAGATGACCCAGAGCCCCAGCAGCCTGAGCGCCAGCGTGGGCGACCGCGTGAC
CATCACCTGCAGCGCCAGCAGCAGCGTGAGCTACATGAACTGGTACCAGCAGACCCCCGGC
 
AAGGCCCCCAAGCGCTGGATCTACGACACCAGCAAGCTGGCCAGCGGCGTGCCCAGCCGCT
TCAGCGGCAGCGGCAGCGGCACCGACTACACCTTCACCATCAGCAGCCTGCAGCCCGAGGA
CATCGCCACCTACTACTGCCAGCAGTGGAGCAGCAACCCCTTCACCTTCGGCCAGGGCACCA
AGCTGCAGATCACCCGCCGTACGGTGGCCGCACCAAGCGTCTTCATCTTCCCGCCATCTGAT
GAGCAGTTGAAATCTGGAACTGCCTCTGTTGTGTGCCTGCTGAATAACTTCTATCCCAGAGA
GGCCAAAGTACAGTGGAAGGTGGATAACGCCCTCCAATCGGGTAACTCCCAGGAGAGTGTC
ACAGAGCAGGACAGCAAGGACAGCACCTACAGCCTCAGCAGCACCCTGACGCTGAGCAAAG
CAGACTACGAGAAACACAAAGTCTACGCCTGCGAAGTCACCCATCAGGGCCTGAGCTCGCC
CGTCACAAAGAGCTTTAACAGAGGCGAGTGCGGCGGCGGCGGATCCGGCGGCGGCGGAAGC
GGCGGCGGCGGATCCGGCGGCGGCGGAAGCGGCGGCGGATCCGGCGGCGGAGGAAGC
CAGGTGCAGCTGGTGCAGAGCGGCGGCGGCGTGGTGCAGCCCGGCCGCAGCCTGCGCCTGA
GCTGCAAGGCCAGCGGCTACACCTTCACCCGCTACACCATGCACTGGGTGCGCCAGGCCCCC
GGCAAGGGCCTGGAGTGGATCGGCTACATCAACCCCAGCCGCGGCTACACCAACTACAACC
AGAAGTTCAAGGACCGCTTCACCATCAGCCGCGACAACAGCAAGAACACCGCCTTCCTGCA
GATGGACAGCCTGCGCCCCGAGGACACCGGCGTGTACTTCTGCGCCCGCTACTACGACGACC
ACTACTGCCTGGACTACTGGGGCCAGGGCACCCCCGTGACCGTGAGCAGCGCTAGCACCAA
GGGCCCCAGCGTGTTCCCCCTGGCCCCCTGCAGCCGCAGCACCAGCGAGAGCACCGCCGCCC
TGGGCTGCCTGGTGAAGGACTACTTCCCCGAGCCCGTGACCGTGAGCTGGAACAGCGGCGC
CCTGACCAGCGGCGTGCACACCTTCCCCGCCGTGCTGCAGAGCAGCGGCCTGTACAGCCTGA
GCAGCGTGGTGACCGTGCCCAGCAGCAGCCTGGGCACCAAGACCTACACCTGCAACGTGGA
CCACAAGCCCAGCAACACCAAGGTGGACAAGCGCGTGGAGAGCAAGTACGGCCCCCCCTGC
CCCCCCTGCCCCGCCCCCGAGGCCGCCGGCGGCCCCAGCGTGTTCCTGTTCCCCCCCAAGCC
CAAGGACACCCTGATGATCAGCCGCACCCCCGAGGTGACCTGCGTGGTGGTGGACGTGAGC
CAGGAGGACCCCGAGGTGCAGTTCAACTGGTACGTGGACGGCGTGGAGGTGCACAACGCCA
AGACCAAGCCCCGCGAGGAGCAGTTCAACAGCACCTACCGCGTGGTGAGCGTGCTGACCGT
GCTGCACCAGGACTGGCTGAACGGCAAGGAGTACAAGTGCAAGGTGAGCAACAAGGGCCTG
CCCAGCAGCATCGAGAAGACCATCAGCAAGGCCAAGGGCCAGCCCCGCGAGCCCCAGGTGT
ACACCCTGCCCCCCtgcCAGGAGGAGATGACCAAGAACCAGGTGAGCCTGtggTGCCTGGTGA
AGGGCTTCTACCCCAGCGACATCGCCGTGGAGTGGGAGAGCAACGGCCAGCCCGAGAACAA
CTACAAGACCACCCCCCCCGTGCTGGACAGCGACGGCAGCTTCTTCCTGTACAGCAGACTGA
CCGTGGACAAGAGCCGCTGGCAGGAGGGCAACGTGTTCAGCTGCAGCGTGATGCACGAGGC
CCTGCACAACCACTACACCCAGAAGAGCCTGAGCCTGAGCCTGGGCAAG
SEQ ID NO. 55 hFc PE Amino acid sequence 
ESKYGPPCPPCPAPEFEGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSDGSFFLYSRL
TVDKSRWQEGNVFSCSVMHEALHNHYTQKSLSLSLGK
SEQ ID NO. 56 hFc PE Nucleotide sequence 
GAGAGCAAGTACGGCCCTCCCTGCCCACCCTGCCCCGCTCCCGAGTTTGAAGGCGGCCC
CAGCGTGTTCCTGTTCCCCCCCAAGCCCAAGGACACCCTGATGATCAGCCGCACCCCCGAGG
TGACCTGCGTGGTGGTGGACGTGAGCCAGGAGGACCCCGAGGTGCAGTTCAACTGGTACGT
GGACGGCGTGGAGGTGCACAACGCCAAGACCAAGCCCCGCGAGGAGCAGTTCAACAGCACC
TACCGCGTGGTGAGCGTGCTGACCGTGCTGCACCAGGACTGGCTGAACGGCAAGGAGTACA
AGTGCAAGGTGAGCAACAAGGGCCTGCCCAGCAGCATCGAGAAGACCATCAGCAAGGCCA
AGGGCCAGCCCCGCGAGCCCCAGGTGTACACCCTGCCCCCCAGCCAGGAGGAGATGACCAA
GAACCAGGTGAGCCTGACCTGCCTGGTGAAGGGCTTCTACCCCAGCGACATCGCCGTGGAGT
GGGAGAGCAACGGCCAGCCCGAGAACAACTACAAGACCACCCCCCCCGTGCTGGACAGCGA
CGGCAGCTTCTTCCTGTACAGCAGACTGACCGTGGACAAGAGCCGCTGGCAGGAGGGCAAC
GTGTTCAGCTGCAGCGTGATGCACGAGGCCCTGCACAACCACTACACCCAGAAGAGCCTGA
GCCTGAGCCTGGGCAAG
SEQ ID NO. 57 05VH-hFc PE Amino acid sequence 
EVQLVESGGGVVQPGRSLRLTCAASGFTFSTSGMYWIRQAPGKGLEWVSYISSSSSTIYYADS
VKGRFTISRDNSKNTLYLQMNSLRAEDTAVYYCVTQSCGAGKCYPGVWGQGTTVTVSSESKYG
PPCPPCPAPEFEGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTVDKSRW
QEGNVFSCSVMHEALHNHYTQKSLSLSLGK
SEQ ID NO. 58 05VH-hFc PE Nucleotide sequence 
GAGGTGCAGCTGGTGGAGTCTGGGGGAGGCGTGGTCCAGCCTGGGAGGTCCCTGAGACT
CACCTGTGCAGCCTCTGGATTCACGTTCAGTACCTCTGGCATGTACTGGATTCGCCAGGCTCC
AGGCAAGGGGCTGGAGTGGGTTTCATACATTAGTAGTAGTAGTAGTACCATATACTACGCAG
ACTCCGTGAAGGGCCGGTTCACCATCTCCAGAGACAATTCCAAGAACACGCTGTATCTGCAA
ATGAACAGCCTGAGAGCTGAGGACACGGCTGTGTATTATTGTGTGACTCAGTCGTGTGGTGC
TGGTAAATGCTACCCGGGGGTCTGGGGCCAAGGGACCACGGTCACTGTCTCCTCAGAGAGC
AAGTACGGCCCTCCCTGCCCACCCTGCCCCGCTCCCGAGTTTGAAGGCGGCCCCAGCGTGTT
CCTGTTCCCCCCCAAGCCCAAGGACACCCTGATGATCAGCCGCACCCCCGAGGTGACCTGCG
TGGTGGTGGACGTGAGCCAGGAGGACCCCGAGGTGCAGTTCAACTGGTACGTGGACGGCGT
GGAGGTGCACAACGCCAAGACCAAGCCCCGCGAGGAGCAGTTCAACAGCACCTACCGCGTG
GTGAGCGTGCTGACCGTGCTGCACCAGGACTGGCTGAACGGCAAGGAGTACAAGTGCAAGG
TGAGCAACAAGGGCCTGCCCAGCAGCATCGAGAAGACCATCAGCAAGGCCAAGGGCCAGCC
CCGCGAGCCCCAGGTGTACACCCTGCCCCCCAGCCAGGAGGAGATGACCAAGAACCAGGTG
AGCCTGACCTGCCTGGTGAAGGGCTTCTACCCCAGCGACATCGCCGTGGAGTGGGAGAGCA
ACGGCCAGCCCGAGAACAACTACAAGACCACCCCCCCCGTGCTGGACAGCGACGGCAGCTT
CTTCCTGTACAGCAGACTGACCGTGGACAAGAGCCGCTGGCAGGAGGGCAACGTGTTCAGC
TGCAGCGTGATGCACGAGGCCCTGCACAACCACTACACCCAGAAGAGCCTGAGCCTGAGCC
TGGGCAAG
SEQ ID NO. 59 human BAFF-R Amino acid sequence 
MRRGPRSLRGRDAPAPTPCVPAECFDLLVRHCVACGLLRTPRPKPAGASSPAPRTALQPQES
VGAGAGEAALPLPGLLFGAPALLGLALVLALVLVGLVSWRRRQRRLRGASSAEAPDGDKDAPE
PLDKVIILSPGISDATAPAWPPPGEDPGTTPPGHSVPVPATELGSTELVTTKTAGPEQQ
SEQ ID NO. 60 cynomolgus BAFF-R Amino acid sequence 
MKRGPRSLRGRDAPAPTPCVPAECFDLLVRHCVACGLLRTPRPKPAPASSPAPRTALQPQES
VGAGAGEAALSLPGLLFGAPALLGLALVLALVLVGLVSWRRRQRRLRGASSAEAPDGDKDKDE
PLDKVIILSPGISDAAAPAWPPPGEDPGTTPPGHSVPVPATELGSTELVTTKTAGPEQQ
SEQ ID NO. 61 mouse BAFF-R Amino acid sequence 
MGARRLRVRSQRSRDSSVPTQCNQTECFDPLVRNCVSCELFHTPDTGHTSSLEPGTALQPQE
GSALRPDVALLVGAPALLGLILALTLVGLVSLVSWRWRQQLRTASPDTSEGVQQESLENVFVPSS
ETPHASAPTWPPLKEDADSALPRHSVPVPATELGSTELVTTKTAGPEQ
SEQ ID NO. 62 human BAFF-R Extracellular domain amino acid sequence 
MRRGPRSLRGRDAPAPTPCVPAECFDLLVRHCVACGLLRTPRPKPAGASSPAPRTALQPQES
VGAGAGEAALPLPGLL
SEQ ID NO. 63 cynomolgus BAFF-R Extracellular domain amino acid sequence 
MKRGPRSLRGRDAPAPTPCVPAECFDLLVRHCVACGLLRTPRPKPAPASSPAPRTALQPQES
VGAGAGEAALSLPGLL
SEQ ID NO. 64 mouse BAFF-R Extracellular domain amino acid sequence 
MGARRLRVRSQRSRDSSVPTQCNQTECFDPLVRNCVSCELFHTPDTGHTSSLEPGTALQPQE
GSALRPDVALL
SEQ ID NO. 65 SP34 VH Amino acid sequence 
EVQLVESGGGLVQPKGSLKLSCAASGFTFNTYAMNWVRQAPGKGLEWVARIRSKYNNYAT
YYADSVKDRFTISRDDSQSILYLQMNNLKTEDTAMYYCVRHGNFGNSYVSWFAYWGQGTLVT
VSS
SEQ ID NO. 66 SP34 VH Nucleotide sequence 
GAGGTGCAGCTGGTGGAGAGCGGCGGCGGCCTGGTGCAGCCCAAGGGCAGCCTGAAGC
TGAGCTGCGCCGCCAGCGGCTTCACCTTCAACACCTACGCCATGAACTGGGTGCGCCAGGCC
CCCGGCAAGGGCCTGGAGTGGGTGGCCCGCATCCGCAGCAAGTACAACAACTACGCCACCT
ACTACGCCGACAGCGTGAAGGACCGCTTCACCATCAGCCGCGACGACAGCCAGAGCATCCT
GTACCTGCAGATGAACAACCTGAAGACCGAGGACACCGCCATGTACTACTGCGTGCGCCAC
GGCAACTTCGGCAACAGCTACGTGAGCTGGTTCGCCTACTGGGGCCAGGGCACCCTGGTGAC
CGTGAGCAGC
SEQ ID NO. 67 SP34 VL Amino acid sequence 
QAVVTQESALTTSPGETVTLTCRSSTGAVTTSNYANWVQEKPDHLFTGLIGGTNKRAPGVPA
RFSGSLIGDKAALTITGAQTEDEAIYFCALWYSNLWVFGGGTKLTVL
SEQ ID NO. 68 SP34 VL Nucleotide sequence 
CAGGCCGTGGTGACCCAGGAGAGCGCCCTGACCACCAGCCCCGGCGAGACCGTGACCCT
GACCTGCCGCAGCAGCACCGGCGCCGTGACCACCAGCAACTACGCCAACTGGGTGCAGGAG
AAGCCCGACCACCTGTTCACCGGCCTGATCGGCGGCACCAACAAGCGCGCCCCCGGCGTGC
CCGCCCGCTTCAGCGGCAGCCTGATCGGCGACAAGGCCGCCCTGACCATCACCGGCGCCCA
GACCGAGGACGAGGCCATCTACTTCTGCGCCCTGTGGTACAGCAACCTGTGGGTGTTCGGCG
GCGGCACCAAGCTGACCGTGCTG
SEQ ID NO. 69 SP34-H PE Amino acid sequence 
EVQLVESGGGLVQPKGSLKLSCAASGFTFNTYAMNWVRQAPGKGLEWVARIRSKYNNYAT
YYADSVKDRFTISRDDSQSILYLQMNNLKTEDTAMYYCVRHGNFGNSYVSWFAYWGQGTLVT
VSSASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEFEGGPSVFLFPPKPKD
TLMISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFYPSDI
AVEWESNGQPENNYKTTPPVLDSDGSFFLYSRLTVDKSRWQEGNVFSCSVMHEALHNHYTQKS
LSLSLGK
SEQ ID NO. 70 SP34-H PE Nucleotide sequence 
GAGGTGCAGCTGGTGGAGAGCGGCGGCCTGGTGCAGCCCAAGGGCAGCCTGAAGC
TGAGCTGCGCCGCCAGCGGCTTCACCTTCAACACCTACGCCATGAACTGGGTGCGCCAGGCC
CCCGGCAAGGGCCTGGAGTGGGTGGCCCGCATCCGCAGCAAGTACAACAACTACGCCACCT
ACTACGCCGACAGCGTGAAGGACCGCTTCACCATCAGCCGCGACGACAGCCAGAGCATCCT
GTACCTGCAGATGAACAACCTGAAGACCGAGGACACCGCCATGTACTACTGCGTGCGCCAC
GGCAACTTCGGCAACAGCTACGTGAGCTGGTTCGCCTACTGGGGCCAGGGCACCCTGGTGAC
CGTGAGCAGCGCTAGCACCAAGGGCCCCAGCGTGTTCCCCCTGGCCCCCTGCAGCCGCAGCA
CCAGCGAGAGCACCGCCGCCCTGGGCTGCCTGGTGAAGGACTACTTCCCCGAGCCCGTGACC
GTGAGCTGGAACAGCGGCGCCCTGACCAGCGGCGTGCACACCTTCCCCGCCGTGCTGCAGA
GCAGCGGCCTGTACAGCCTGAGCAGCGTGGTGACCGTGCCCAGCAGCAGCCTGGGCACCAA
GACCTACACCTGCAACGTGGACCACAAGCCCAGCAACACCAAGGTGGACAAGCGCGTGGAG
AGCAAGTACGGCCCTCCCTGCCCACCCTGCCCCGCTCCCGAGTTTGAAGGCGGCCCCAGCGT
GTTCCTGTTCCCCCCCAAGCCCAAGGACACCCTGATGATCAGCCGCACCCCCGAGGTGACCT
GCGTGGTGGTGGACGTGAGCCAGGAGGACCCCGAGGTGCAGTTCAACTGGTACGTGGACGG
CGTGGAGGTGCACAACGCCAAGACCAAGCCCCGCGAGGAGCAGTTCAACAGCACCTACCGC
GTGGTGAGCGTGCTGACCGTGCTGCACCAGGACTGGCTGAACGGCAAGGAGTACAAGTGCA
AGGTGAGCAACAAGGGCCTGCCCAGCAGCATCGAGAAGACCATCAGCAAGGCCAAGGGCC
AGCCCCGCGAGCCCCAGGTGTACACCCTGCCCCCCAGCCAGGAGGAGATGACCAAGAACCA
GGTGAGCCTGACCTGCCTGGTGAAGGGCTTCTACCCCAGCGACATCGCCGTGGAGTGGGAG
AGCAACGGCCAGCCCGAGAACAACTACAAGACCACCCCCCCCGTGCTGGACAGCGACGGCA
GCTTCTTCCTGTACAGCAGACTGACCGTGGACAAGAGCCGCTGGCAGGAGGGCAACGTGTTC
 
AGCTGCAGCGTGATGCACGAGGCCCTGCACAACCACTACACCCAGAAGAGCCTGAGCCTGA
GCCTGGGCAAG
SEQ ID NO. 71 SP34-L Amino acid sequence 
QAVVTQESALTTSPGETVTLTCRSSTGAVTTSNYANWVQEKPDHLFTGLIGGTNKRAPGVPA
RFSGSLIGDKAALTITGAQTEDEAIYFCALWYSNLWVFGGGTKLTVLGQPKAAPSVTLFPPSSEEL
QANKATLVCLISDFYPGAVTVAWKADSSPVKAGVETTTPSKQSNNKYAASSYLSLTPEQWKSH
KSYSCQVTHEGSTVEKTVAPTECS
SEQ ID NO. 72 SP34-L Nucleotide sequence 
CAGGCCGTGGTGACCCAGGAGAGCGCCCTGACCACCAGCCCCGGCGAGACCGTGACCCT
GACCTGCCGCAGCAGCACCGGCGCCGTGACCACCAGCAACTACGCCAACTGGGTGCAGGAG
AAGCCCGACCACCTGTTCACCGGCCTGATCGGCGGCACCAACAAGCGCGCCCCCGGCGTGC
CCGCCCGCTTCAGCGGCAGCCTGATCGGCGACAAGGCCGCCCTGACCATCACCGGCGCCCA
GACCGAGGACGAGGCCATCTACTTCTGCGCCCTGTGGTACAGCAACCTGTGGGTGTTCGGCG
GCGGCACCAAGCTGACCGTGCTGGGTCAGCCCAAGGCTGCCCCTAGCGTGACACTGTTCCCA
CCTTCTAGCGAGGAACTGCAAGCGAATAAGGCTACACTGGTGTGCCTGATTAGCGACTTCTA
CCCTGGCGCCGTGACCGTAGCATGGAAAGCGGACTCTAGCCCCGTGAAGGCCGGCGTGGAG
ACCACAACCCCATCTAAGCAGTCTAACAACAAGTACGCCGCCTCTTCCTACCTGTCCCTCAC
CCCTGAGCAGTGGAAGTCTCACAAGTCTTACTCTTGCCAGGTGACACACGAGGGCTCTACAG
TGGAAAAGACAGTGGCCCCTACAGAGTGCAGC
SEQ ID NO. 73 SP34-H PAA Ks Amino acid sequence 
EVQLVESGGGLVQPKGSLKLSCAASGFTFNTYAMNWVRQAPGKGLEWVARIRSKYNNYAT
YYADSVKDRFTISRDDSQSILYLQMNNLKTEDTAMYYCVRHGNFGNSYVSWFAYWGQGTLVT
VSSASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEAAGGPSVFLFPPKPKD
TLMISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPCQEEMTKNQVSLWCLVKGFYPSD
IAVEWESNGQPENNYKTTPPVLDSDGSFFLYSRLTVDKSRWQEGNVFSCSVMHEALHNHYTQKS
LSLSLGK
SEQ ID NO. 74 SP34-H PAA Ks Nucleotide sequence 
GAGGTGCAGCTGGTGGAGAGCGGCGGCGGCCTGGTGCAGCCCAAGGGCAGCCTGAAGC
TGAGCTGCGCCGCCAGCGGCTTCACCTTCAACACCTACGCCATGAACTGGGTGCGCCAGGCC
CCCGGCAAGGGCCTGGAGTGGGTGGCCCGCATCCGCAGCAAGTACAACAACTACGCCACCT
ACTACGCCGACAGCGTGAAGGACCGCTTCACCATCAGCCGCGACGACAGCCAGAGCATCCT
GTACCTGCAGATGAACAACCTGAAGACCGAGGACACCGCCATGTACTACTGCGTGCGCCAC
GGCAACTTCGGCAACAGCTACGTGAGCTGGTTCGCCTACTGGGGCCAGGGCACCCTGGTGAC
CGTGAGCAGCGCTAGCACCAAGGGCCCCAGCGTGTTCCCCCTGGCCCCCTGCAGCCGCAGCA
CCAGCGAGAGCACCGCCGCCCTGGGCTGCCTGGTGAAGGACTACTTCCCCGAGCCCGTGACC
GTGAGCTGGAACAGCGGCGCCCTGACCAGCGGCGTGCACACCTTCCCCGCCGTGCTGCAGA
 
GCAGCGGCCTGTACAGCCTGAGCAGCGTGGTGACCGTGCCCAGCAGCAGCCTGGGCACCAA
GACCTACACCTGCAACGTGGACCACAAGCCCAGCAACACCAAGGTGGACAAGCGCGTGGAG
AGCAAGTACGGCCCCCCCTGCCCCCCCTGCCCCGCCCCCGAGGCCGCCGGCGGCCCCAGCGT
GTTCCTGTTCCCCCCCAAGCCCAAGGACACCCTGATGATCAGCCGCACCCCCGAGGTGACCT
GCGTGGTGGTGGACGTGAGCCAGGAGGACCCCGAGGTGCAGTTCAACTGGTACGTGGACGG
CGTGGAGGTGCACAACGCCAAGACCAAGCCCCGCGAGGAGCAGTTCAACAGCACCTACCGC
GTGGTGAGCGTGCTGACCGTGCTGCACCAGGACTGGCTGAACGGCAAGGAGTACAAGTGCA
AGGTGAGCAACAAGGGCCTGCCCAGCAGCATCGAGAAGACCATCAGCAAGGCCAAGGGCC
AGCCCCGCGAGCCCCAGGTGTACACCCTGCCCCCCtgcCAGGAGGAGATGACCAAGAACCAG
GTGAGCCTGtggTGCCTGGTGAAGGGCTTCTACCCCAGCGACATCGCCGTGGAGTGGGAGAGC
AACGGCCAGCCCGAGAACAACTACAAGACCACCCCCCCCGTGCTGGACAGCGACGGCAGCT
TCTTCCTGTACAGCAGACTGACCGTGGACAAGAGCCGCTGGCAGGAGGGCAACGTGTTCAG
CTGCAGCGTGATGCACGAGGCCCTGCACAACCACTACACCCAGAAGAGCCTGAGCCTGAGC
CTGGGCAAG
SEQ ID NO. 75 05VH-SP34-H PAA Amino acid sequence 
EVQLVESGGGVVQPGRSLRLTCAASGFTFSTSGMYWIRQAPGKGLEWVSYISSSSSTIYYADS
VKGRFTISRDNSKNTLYLQMNSLRAEDTAVYYCVTQSCGAGKCYPGVWGQGTTVTVSSGGGGS
GGGGSEVQLVESGGGLVQPKGSLKLSCAASGFTFNTYAMNWVRQAPGKGLEWVARIRSKYNN
YATYYADSVKDRFTISRDDSQSILYLQMNNLKTEDTAMYYCVRHGNFGNSYVSWFAYWGQGT
LVTVSSASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEAAGGPSVFLFPP
KPKDTLMISRTPEVTCVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCSVMHEALHNHY
TQKSLSLSLGK
SEQ ID NO. 76 05VH-SP34-H PAA Nucleotide sequence 
GAGGTGCAGCTGGTGGAGTCTGGGGGAGGCGTGGTCCAGCCTGGGAGGTCCCTGAGACT
CACCTGTGCAGCCTCTGGATTCACGTTCAGTACCTCTGGCATGTACTGGATTCGCCAGGCTCC
AGGCAAGGGGCTGGAGTGGGTTTCATACATTAGTAGTAGTAGTAGTACCATATACTACGCAG
ACTCCGTGAAGGGCCGGTTCACCATCTCCAGAGACAATTCCAAGAACACGCTGTATCTGCAA
ATGAACAGCCTGAGAGCTGAGGACACGGCTGTGTATTATTGTGTGACTCAGTCGTGTGGTGC
TGGTAAATGCTACCCGGGGGTCTGGGGCCAAGGGACCACGGTCACTGTCTCCTCAGGCGGA
GGCGGTAGCGGAGGTGGAGGGTCTGAGGTGCAGCTGGTGGAGAGCGGCGGCCTGGTGC
AGCCCAAGGGCAGCCTGAAGCTGAGCTGCGCCGCCAGCGGCTTCACCTTCAACACCTACGCC
ATGAACTGGGTGCGCCAGGCCCCCGGCAAGGGCCTGGAGTGGGTGGCCCGCATCCGCAGCA
AGTACAACAACTACGCCACCTACTACGCCGACAGCGTGAAGGACCGCTTCACCATCAGCCG
CGACGACAGCCAGAGCATCCTGTACCTGCAGATGAACAACCTGAAGACCGAGGACACCGCC
ATGTACTACTGCGTGCGCCACGGCAACTTCGGCAACAGCTACGTGAGCTGGTTCGCCTACTG
 
GGGCCAGGGCACCCTGGTGACCGTGAGCAGCGCTAGCACCAAGGGCCCCAGCGTGTTCCCC
CTGGCCCCCTGCAGCCGCAGCACCAGCGAGAGCACCGCCGCCCTGGGCTGCCTGGTGAAGG
ACTACTTCCCCGAGCCCGTGACCGTGAGCTGGAACAGCGGCGCCCTGACCAGCGGCGTGCA
CACCTTCCCCGCCGTGCTGCAGAGCAGCGGCCTGTACAGCCTGAGCAGCGTGGTGACCGTGC
CCAGCAGCAGCCTGGGCACCAAGACCTACACCTGCAACGTGGACCACAAGCCCAGCAACAC
CAAGGTGGACAAGCGCGTGGAGAGCAAGTACGGCCCCCCCTGCCCCCCCTGCCCCGCCCCC
GAGGCCGCCGGCGGCCCCAGCGTGTTCCTGTTCCCCCCCAAGCCCAAGGACACCCTGATGAT
CAGCCGCACCCCCGAGGTGACCTGCGTGGTGGTGGACGTGAGCCAGGAGGACCCCGAGGTG
CAGTTCAACTGGTACGTGGACGGCGTGGAGGTGCACAACGCCAAGACCAAGCCCCGCGAGG
AGCAGTTCAACAGCACCTACCGCGTGGTGAGCGTGCTGACCGTGCTGCACCAGGACTGGCTG
AACGGCAAGGAGTACAAGTGCAAGGTGAGCAACAAGGGCCTGCCCAGCAGCATCGAGAAG
ACCATCAGCAAGGCCAAGGGCCAGCCCCGCGAGCCCCAGGTGTACACCCTGCCCCCCAGCC
AGGAGGAGATGACCAAGAACCAGGTGAGCCTGACCTGCCTGGTGAAGGGCTTCTACCCCAG
CGACATCGCCGTGGAGTGGGAGAGCAACGGCCAGCCCGAGAACAACTACAAGACCACCCCC
CCCGTGCTGGACAGCGACGGCAGCTTCTTCCTGTACAGCAGACTGACCGTGGACAAGAGCC
GCTGGCAGGAGGGCAACGTGTTCAGCTGCAGCGTGATGCACGAGGCCCTGCACAACCACTA
CACCCAGAAGAGCCTGAGCCTGAGCCTGGGCAAG
SEQ ID NO. 77 05VH-SP34-H PAA Ks Amino acid sequence 
EVQLVESGGGVVQPGRSLRLTCAASGFTFSTSGMYWIRQAPGKGLEWVSYISSSSSTIYYADS
VKGRFTISRDNSKNTLYLQMNSLRAEDTAVYYCVTQSCGAGKCYPGVWGQGTTVTVSSGGGGS
GGGGSEVQLVESGGGLVQPKGSLKLSCAASGFTFNTYAMNWVRQAPGKGLEWVARIRSKYNN
YATYYADSVKDRFTISRDDSQSILYLQMNNLKTEDTAMYYCVRHGNFGNSYVSWFAYWGQGT
LVTVSSASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEAAGGPSVFLFPP
KPKDTLMISRTPEVTCVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPCQEEMTKNQVSLWCLVKGF
YPSDIAVEWESNGQPENNYKTTPPVLDSDGSFFLYSRLTVDKSRWQEGNVFSCSVMHEALHNHY
TQKSLSLSLGK
SEQ ID NO. 78 05VH-SP34-H PAA Ks Nucleotide sequence 
GAGGTGCAGCTGGTGGAGTCTGGGGGAGGCGTGGTCCAGCCTGGGAGGTCCCTGAGACT
CACCTGTGCAGCCTCTGGATTCACGTTCAGTACCTCTGGCATGTACTGGATTCGCCAGGCTCC
AGGCAAGGGGCTGGAGTGGGTTTCATACATTAGTAGTAGTAGTAGTACCATATACTACGCAG
ACTCCGTGAAGGGCCGGTTCACCATCTCCAGAGACAATTCCAAGAACACGCTGTATCTGCAA
ATGAACAGCCTGAGAGCTGAGGACACGGCTGTGTATTATTGTGTGACTCAGTCGTGTGGTGC
TGGTAAATGCTACCCGGGGGTCTGGGGCCAAGGGACCACGGTCACTGTCTCCTCAGGCGGA
GGCGGTAGCGGAGGTGGAGGGTCTGAGGTGCAGCTGGTGGAGAGCGGCGGCGGCCTGGTGC
AGCCCAAGGGCAGCCTGAAGCTGAGCTGCGCCGCCAGCGGCTTCACCTTCAACACCTACGCC
ATGAACTGGGTGCGCCAGGCCCCCGGCAAGGGCCTGGAGTGGGTGGCCCGCATCCGCAGCA
 
AGTACAACAACTACGCCACCTACTACGCCGACAGCGTGAAGGACCGCTTCACCATCAGCCG
CGACGACAGCCAGAGCATCCTGTACCTGCAGATGAACAACCTGAAGACCGAGGACACCGCC
ATGTACTACTGCGTGCGCCACGGCAACTTCGGCAACAGCTACGTGAGCTGGTTCGCCTACTG
GGGCCAGGGCACCCTGGTGACCGTGAGCAGCGCTAGCACCAAGGGCCCCAGCGTGTTCCCC
CTGGCCCCCTGCAGCCGCAGCACCAGCGAGAGCACCGCCGCCCTGGGCTGCCTGGTGAAGG
ACTACTTCCCCGAGCCCGTGACCGTGAGCTGGAACAGCGGCGCCCTGACCAGCGGCGTGCA
CACCTTCCCCGCCGTGCTGCAGAGCAGCGGCCTGTACAGCCTGAGCAGCGTGGTGACCGTGC
CCAGCAGCAGCCTGGGCACCAAGACCTACACCTGCAACGTGGACCACAAGCCCAGCAACAC
CAAGGTGGACAAGCGCGTGGAGAGCAAGTACGGCCCCCCCTGCCCCCCCTGCCCCGCCCCC
GAGGCCGCCGGCGGCCCCAGCGTGTTCCTGTTCCCCCCCAAGCCCAAGGACACCCTGATGAT
CAGCCGCACCCCCGAGGTGACCTGCGTGGTGGTGGACGTGAGCCAGGAGGACCCCGAGGTG
CAGTTCAACTGGTACGTGGACGGCGTGGAGGTGCACAACGCCAAGACCAAGCCCCGCGAGG
AGCAGTTCAACAGCACCTACCGCGTGGTGAGCGTGCTGACCGTGCTGCACCAGGACTGGCTG
AACGGCAAGGAGTACAAGTGCAAGGTGAGCAACAAGGGCCTGCCCAGCAGCATCGAGAAG
ACCATCAGCAAGGCCAAGGGCCAGCCCCGCGAGCCCCAGGTGTACACCCTGCCCCCCtgcCA
GGAGGAGATGACCAAGAACCAGGTGAGCCTGtggTGCCTGGTGAAGGGCTTCTACCCCAGCG
ACATCGCCGTGGAGTGGGAGAGCAACGGCCAGCCCGAGAACAACTACAAGACCACCCCCCC
CGTGCTGGACAGCGACGGCAGCTTCTTCCTGTACAGCAGACTGACCGTGGACAAGAGCCGCT
GGCAGGAGGGCAACGTGTTCAGCTGCAGCGTGATGCACGAGGCCCTGCACAACCACTACAC
CCAGAAGAGCCTGAGCCTGAGCCTGGGCAAG
SEQ ID NO. 79 SP34 H-CDR1
   GFTFNTYA
SEQ ID NO. 80 SP34 H-CDR2
   IRSKYNNYAT
SEQ ID NO. 81 SP34 H-CDR3
   VRHGNFGNSYVSWFAY
SEQ ID NO. 82 SP34 L-CDR1
   TGAVTTSNY
SP34 L-CDR2
   GTN
SEQ ID NO. 83 SP34 L-CDR3
   ALWYSNLWV

All documents mentioned in the present invention are incorporated herein by reference in their entirety, as if each document were individually incorporated by reference. Furthermore, it should be understood that after reading the above teachings of the present invention, those skilled in the art can make various alterations or modifications to the present invention, and such equivalent forms shall also fall within the scope defined by the appended claims of the present application.

## Claims

1. A single-domain antibody targeting B-cell activating factor receptor (BAFF-R), wherein the VHH chain of the single-domain antibody comprises the following complementarity-determining regions (CDRs): CDR1 set forth in SEQ ID NO. 27, CDR2 set forth in SEQ ID NO. 28, and CDR3 set forth in SEQ ID NO. 29;
any one of the aforementioned amino acid sequences also includes derivative sequences optionally obtained by addition, deletion, modification and/or substitution of at least one amino acid while being capable of retaining the binding affinity to BAFF-R.

2. An antibody targeting B-cell activating factor receptor (BAFF-R), wherein the heavy chain variable region of the antibody comprises the following complementarity-determining regions (CDRs): CDR1 set forth in SEQ ID NO. 27, CDR2 set forth in SEQ ID NO. 28, and CDR3 set forth in SEQ ID NO. 29; or
the light chain variable region of the antibody comprises the following complementarity-determining regions (CDRs): CDR1 set forth in SEQ ID NO. 36, CDR2 set forth in AAS, and CDR3 set forth in SEQ ID NO. 37, and the heavy chain variable region of the antibody comprises the following complementarity-determining regions (CDRs): CDR1 set forth in SEQ ID NO. 38, CDR2 set forth in SEQ ID NO. 39, and CDR3 set forth in SEQ ID NO. 40;
any one of the aforementioned amino acid sequences also includes derivative sequences optionally obtained by addition, deletion, modification and/or substitution of at least one amino acid while being capable of retaining the binding affinity to BAFF-R.

3. The antibody according to claim 2, wherein the heavy chain variable region has an amino acid sequence set forth in SEQ ID NO. 25, or an amino acid sequence having at least 70%, 75%, 80%, 85%, 90%, 91%, 92%, 93%, 94%, 95%, 96%, 97%, 98%, 99%, 100% identity thereto.

4. The antibody according to claim 2, wherein the light chain variable region has an amino acid sequence set forth in SEQ ID NO. 30, or an amino acid sequence having at least 70%, 75%, 80%, 85%, 90%, 91%, 92%, 93%, 94%, 95%, 96%, 97%, 98%, 99%, 100% identity thereto, and the heavy chain variable region has an amino acid sequence set forth in SEQ ID NO. 32, or an amino acid sequence having at least 70%, 75%, 80%, 85%, 90%, 91%, 92%, 93%, 94%, 95%, 96%, 97%, 98%, 99%, 100% identity thereto.

5. A chimeric antigen receptor, wherein the antigen-binding domain of the chimeric antigen receptor comprises an antigen-binding fragment targeting BAFF-R, and the heavy chain variable region of the antigen-binding fragment comprises the following complementarity-determining regions (CDRs): CDR1 set forth in SEQ ID NO. 27, CDR2 set forth in SEQ ID NO. 28, and CDR3 set forth in SEQ ID NO. 29;
or the light chain variable region of the antigen-binding fragment comprises the following complementarity-determining regions (CDRs): CDR1 set forth in SEQ ID NO. 36, CDR2 set forth in AAS, and CDR3 set forth in SEQ ID NO. 37, and the heavy chain variable region of the antibody comprises the following complementarity-determining regions (CDRs): CDR1 set forth in SEQ ID NO. 38, CDR2 set forth in SEQ ID NO. 39, and CDR3 set forth in SEQ ID NO. 40.

6. A multispecific antibody, wherein the multispecific antibody comprises the single-domain antibody targeting BAFF-R according to claim 1 or the antibody targeting BAFF-R according to claim 2.

7. A bispecific antibody, wherein the bispecific antibody comprises:
a first antigen-binding region targeting BAFF-R; and
a second antigen-binding region targeting CD3; wherein,
the first antigen-binding region targeting BAFF-R comprises the following complementarity-determining regions CDRs: CDR1 set forth in SEQ ID NO. 27, CDR2 set forth in SEQ ID NO. 28, and CDR3 set forth in SEQ ID NO. 29.

8. The bispecific antibody according to claim 7, wherein the bispecific antibody has the following structure:
(a) two heavy chains, the first heavy chain comprising, from N-terminus to C-terminus: anti-BAFF-R VH, anti-CD3 VH, antibody heavy chain constant region (CH1), and human IgG Fc region; the second heavy chain comprising, from N-terminus to C-terminus: anti-BAFF-R VH and human IgG Fc region;
(b) one light chain, comprising, from N-terminus to C-terminus: anti-CD3 VL and antibody light chain constant region (CL),
wherein a disulfide bond is present between the antibody heavy chain constant region (CH1) and the antibody light chain constant region (CL);
or, the bispecific antibody has the following structure:
(a) two identical heavy chains, each comprising, from N-terminus to C-terminus: anti-BAFF-R VH, anti-CD3 VH, antibody heavy chain constant region (CH1), and human IgG Fc region;
(b) two identical light chains, each comprising, from N-terminus to C-terminus: anti-CD3 VL and antibody light chain constant region (CL),
wherein a disulfide bond is present between the antibody heavy chain constant region (CH1) and the antibody light chain constant region (CL).

9. A recombinant protein, wherein the recombinant protein has:
(i) the single-domain antibody targeting BAFF-R according to claim 1, the antibody targeting BAFF-R according to any one of claims 2-4, the chimeric antigen receptor according to claim 5, the multispecific antibody according to claim 6, or the bispecific antibody according to claim 7; and
(ii) optionally, a polypeptide molecule or fragment having therapeutic function; and/or
(iii) optionally, a functional domain that improves the physicochemical properties or druggability of the protein.

10. A nucleic acid molecule, wherein the nucleic acid molecule encodes a polypeptide selected from the group consisting of:
(1) the single-domain antibody targeting BAFF-R according to claim 1, the antibody targeting BAFF-R according to any one of claims 2-4, the chimeric antigen receptor according to claim 5, the multispecific antibody according to claim 6, or the bispecific antibody according to claim 7; or
(2) the recombinant protein according to claim 9.

11. A vector, wherein the vector comprises the nucleic acid molecule according to claim 10.

12. A host cell, wherein the host cell comprises the vector according to claim 11 or has integrated into its chromosome the exogenous nucleic acid molecule according to claim 10.

13. An engineered immune cell, wherein the immune cell comprises the vector according to claim 11, or has integrated into its chromosome the exogenous nucleic acid molecule according to claim 10, or expresses the antibody according to claim 1 or the chimeric antigen receptor according to claim 5.

14. An immunoconjugate, wherein the immunoconjugate comprises:
(a) an antibody moiety, the antibody moiety being the single-domain antibody targeting BAFF-R according to claim 1, the antibody targeting BAFF-R according to any one of claims 2-4, the multispecific antibody according to claim 6, or the bispecific antibody according to claim 7; and
(b) a conjugate moiety conjugated to the antibody moiety, the conjugate moiety being selected from the group consisting of: a detectable label, a drug, a toxin, a cytokine, an enzyme, or a combination thereof.

15. A pharmaceutical composition or formulation, comprising:
(i) the single-domain antibody targeting BAFF-R according to claim 1, the antibody targeting BAFF-R according to any one of claims 2-4, the multispecific antibody according to claim 6, or the bispecific antibody according to claim 7, the recombinant protein according to claim 9, the engineered immune cell according to claim 13, or the immunoconjugate according to claim 14; and
(ii) a pharmaceutically acceptable carrier.

16. Use of an active ingredient in (a) preparing a detection reagent, a detection plate or a kit; and/or (b) preparing a drug for the prevention and/or treatment of a BAFF-R-associated disease, the active ingredient being selected from the group consisting of: the single-domain antibody targeting BAFF-R according to claim 1, the antibody targeting BAFF-R according to any one of claims 2-4, the multispecific antibody according to claim 6, or the bispecific antibody according to claim 7, the recombinant protein according to claim 9, the engineered immune cell according to claim 13, or the immunoconjugate according to claim 14, or a combination thereof.
